# EUROPEAN PATENT APPLICATION

(11) **EP 4 400 500 A1**
(43) Date of publication of application: **17.07.2024**
(21) Application number: 22866773.9
(22) Date of filing: 09.09.2022
(51) Int. Cl.: C07D 403/04, C07D 487/04, A61K 31/519, A61K 31/501, A61P 13/12

(54) **HALOGEN-SUBSTITUTED PYRIDAZINONE COMPOUND AND APPLICATION THEREOF**

(30) Priority: 10.09.2021 CN 202111064358; 08.02.2022 CN 202210119219
(71) Applicant: Medshine Discovery Inc., Nanjing, Jiangsu 210032 (CN)
(72) Inventor: ZHENG, Xing, Shanghai 200131 (CN); MAO, Qinghua, Shanghai 200131 (CN); YU, Tao, Shanghai 200131 (CN); WU, Chengde, Shanghai 200131 (CN); CHEN, Shuhui, Shanghai 200131 (CN)
(74) Representative: Dehns
(86) International application number: PCT/CN2022/118212
(87) International publication number: WO 2023/036312

(57) **Abstract**

A halogen-substituted pyridazinone compound and an application thereof. Specifically disclosed is a compound represented by formula (I) or a pharmaceutically acceptable salt thereof.

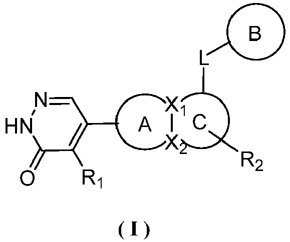

## Description

The present disclosure claims the right of priority of:
CN 202111064358.7 (filing date: September 10, 2021);
CN 202210119219.8 (filing date: February 08, 2022).

### TECHNICAL FIELD

The present disclosure relates to a halogen-substituted pyridazinone compound and an application thereof, in particular to a compound represented by formula (I) or a pharmaceutically acceptable salt thereof.

### BACKGROUND

In 2017, there were nearly 700 million patients with chronic kidney disease (CKD), accounting for 9.1% of the global population. Among them, about 132 million patients were from China. In 2017, a total of 2.6 million people died from CKD and subsequently induced cardiovascular diseases, accounting for 4.6% of the total global deaths. In the past nearly 30 years, there has been a significant decline in the mortality of other non-communicable diseases, for example, the mortality of cancer drops by 14.9%, chronic obstructive pulmonary disease by 41.3%, and cardiovascular diseases by 30.4%. However, such a reduction has not been observed in CKD, which has a mortality change of +2.8%. It is estimated that by 2040, CKD will cause 2.2 to 4 million deaths.

Proteinuria is one of the common manifestations of kidney disease. Due to kidney tissue injury, most patients with chronic kidney disease develop symptoms of proteinuria in the early stages. The development of proteinuria is closely related to the glomerular filtration barrier function. Due to the filtration of the glomerular capillary wall and the reabsorption of the renal tubules, the content of protein (mostly proteins with smaller molecular weights) in the urine of healthy people is very small (the daily excretion is less than 150 mg). When the filtration function of the glomerular capillary wall is impaired, and the reabsorption function of the renal tubules is blocked, a large amount of protein is excreted in the urine. If the urine protein content is ≥ 3.5 g/24 h, it is referred to as massive proteinuria. Prolonged protein leakage can lead to kidney failure. Therefore, more effective methods are required to treat or reduce proteinuria and reduce the risk of developing kidney disease.

The TRP superfamily of ion channels is of great importance in renal physiology. TRP channels are generally non-selective cation channels with high Ca2⁺ permeability. Based on sequence homology, the TRP superfamily can be further divided into six subfamilies with different functions: TRPC, TRPV, TRPA1, TRPM, TRPML, and TRPP. Several TRP proteins (TRPC1, TRPC3, TRPC4, TRPC5, TRPC6, TRPV1, TRPV4, TRPV5, TRPV6, TRPM2, TRPM3, TRPM4, TRPM6, and PKD2) have been reported to be expressed in various cells of the kidney. Podocytes are epithelial cells in the visceral layer of the renal capsule. They are attached to the outside of the glomerular basement membrane (GBM), and together with the vascular endothelial cells and glomerular basement membrane, form the glomerular blood filtration barrier. TRPC5, expressed on the podocytes, mainly mediates Ca²⁺ influx. Activation of Ras-related protein 1 (Rac1) signaling causes TRPC5 to migrate to the surface of the podocyte plasma membrane, enabling the ion channel to be activated by the angiotensin II type 1 receptor (AT1R). The activated ion channel leads to a transient influx of Ca²⁺ into the podocytes, which further activates Rac1 in the podocytes and facilitates the induction of remodeling of the actin cytoskeleton. Hence, the injured podocytes are allowed to be dissociated from the glomerular basement membrane, and the shed podocytes affect the glomerular filtration rate, causing proteinuria. TRPC5 knockout or inhibition by inhibitors such as ML204 or AC1903 significantly reduces LPS-induced proteinuria, inhibits protamine sulfate-induced podocyte injury, and protects podocyte cytoskeletal remodeling. Therefore, TRPC5 is a potential therapeutic target for kidney disease.

### CONTENT OF THE PRESENT INVENTION

The present disclosure provides a compound represented by formula (I) or a pharmaceutically acceptable salt thereof, 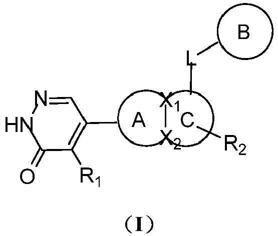 wherein
X₁ and X₂ are each independently selected from C and N;
L is selected from -O-, -S-, -S(=O)-, -C(=O)-, -CF₂-, -CH(OCH₃)-, -N(OCH₃)-, 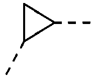 and C₂₋₃ alkenyl, and the -CH(OCH₃)-, 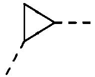 and C₂₋₃ alkenyl are optionally substituted with 1, 2 or 3 Rₐ;
R₁ is selected from F, Cl, Br and I;
R₂ is selected from H, F, Cl, Br, I, OH, NH₂, CN, COOH, CONH₂, C₁₋₃ alkyl, C₁₋₃ alkoxy and C₁₋₃ alkyl-C₁₋₃ alkoxy, and the C₁₋₃ alkyl, C₁₋₃ alkoxy and C₁₋₃ alkyl-C₁₋₃ alkoxy are each independently and optionally substituted with 1, 2 or 3 R_{b};
the structural moiety 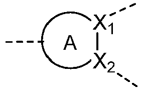 is selected from 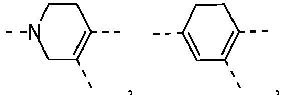 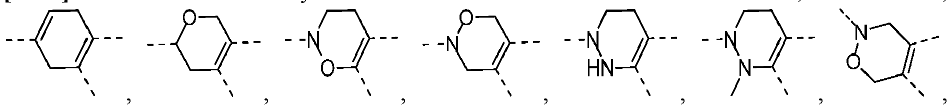 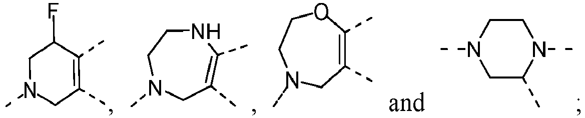
ring B is selected from phenyl and 5- to 6-membered heteroaryl, and the phenyl and 5- to 6-membered heteroaryl are each independently substituted with 1, 2 or 3 R_{c};
ring C is selected from 6-membered heteroaryl;
Rₐ is selected from F, Cl, Br, I and C₁₋₃ alkyl;
R_{b} is selected from F, Cl, Br, I, OH, NH₂, CN, COOH and CONH₂;
R_{c} is selected from F, Cl, Br, I, CONH₂, C₁₋₃ alkyl, C₁₋₃ alkoxy and C₂₋₃ alkenyl, and the C₁₋₃ alkyl, C₁₋₃ alkoxy and C₂₋₃ alkenyl are each independently and optionally substituted with 1, 2 or 3 halogen,
provided that:
   1) when R₁ is Cl, ring A is 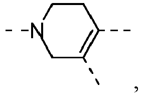 L is O, and ring B is phenyl, then at least one substituent on ring B is Br, CONH₂, CF₂CH₃, C₁₋₃ alkoxy or C₂₋₃ alkenyl, or, ring B is substituted with 3 R_{c};
   2) when R₁ is Cl, ring A is 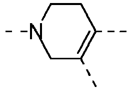 , L is O, and ring B is 2-trifluoromethyl-4-fluorophenyl, then R₂ is CN, COOH, CONH₂, CH₃, CH₂CN, CH₂COOH, CH₂CONH₂ or CH₂OCH₃.

In some embodiments of the present disclosure, the term "hetero" in each occurrence in the above-mentioned 5- to 6-membered heteroaryl or 6-membered heteroaryl independently represents 1, 2 or 3 heteroatoms or heteroatom groups independently selected from N, NH, O and S.

In some embodiments of the present disclosure, the above-mentioned R_{c} is selected from F, Cl, Br, I, CONH₂, CH₃, CH₂CH₃, OCH₃ and -CH=CH₂, the CH₃, CH₂CH₃, OCH₃ and -CH=CH₂ are each independently and optionally substituted with 1, 2 or 3 halogen, and other variables are as defined in the present disclosure.

In some embodiments of the present disclosure, the above-mentioned R_{c} is selected from F, Cl, Br, I, CONH₂, CH₃, CH₂F, CHF₂, CF₃, CH₂CH₃, CF₂CH₃, OCH₃, OCF₃ and - CF=CH₂, and other variables are as defined in the present disclosure.

In some embodiments of the present disclosure, the above-mentioned L is selected from -O-, -S-, -S(=O)-, -C(=O)-, -CF₂-, -CH(OCH₃)-, -N(OCH₃)-, 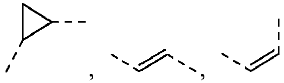 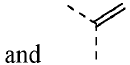 , the -CH(OCH₃)-, 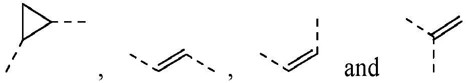 are each independently and optionally substituted with 1, 2 or 3 Rₐ, and other variables are as defined in the present disclosure.

In some embodiments of the present disclosure, the above-mentioned L is selected from -O-, -S-, -S(=O)-, -C(=O)-, -CF₂-, -CH(OCH₃)-, -N(OCH₃)-, 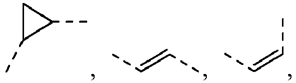 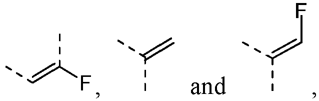 and other variables are as defined in the present disclosure.

In some embodiments of the present disclosure, the above-mentioned L is selected from -O-, -S-, -S(=O)- and -C(=O)-, and other variables are as defined in the present disclosure.

In some embodiments of the present disclosure, the above-mentioned L is selected from -O- and -S-, and other variables are as defined in the present disclosure.

In some embodiments of the present disclosure, the above-mentioned L is selected from 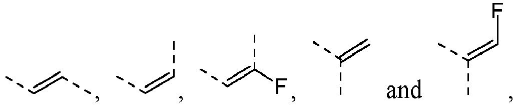 and other variables are as defined in the present disclosure.

In some embodiments of the present disclosure, the above-mentioned L is selected from -CF₂-, -CH(OCH₃)-, -N(OCH₃)- and 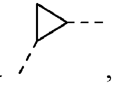 and other variables are as defined in the present disclosure.

In some embodiments of the present disclosure, the above-mentioned R₂ is selected from H, F, Cl, Br, I, OH, NH₂, CN, COOH, CONH₂, CH₃, CH₂CH₃, OCH₃ and CH₂OCH₃, the CH₃, CH₂CH₃, OCH₃ and CH₂OCH₃ are each independently and optionally substituted with 1, 2 or 3 R_{b}, and other variables are as defined in the present disclosure.

In some embodiments of the present disclosure, the above-mentioned R₂ is selected from H, F, Cl, Br, I, OH, NH₂, CN, COOH, CONH₂, CH₃, CH₂CN, CH₂COOH, CH₂CONH₂, CH₂OH, CH₂CH₃, CH(OH)CH₃, CH(OH)CH₂OH, OCH₃ and CH₂OCH₃, and other variables are as defined in the present disclosure.

In some embodiments of the present disclosure, the above-mentioned R₂ is selected from CN, COOH, CONH₂, CH₃, CH₂CN, CH₂COOH, CH₂CONH₂ and CH₂OCH₃, and other variables are as defined in the present disclosure.

In some embodiments of the present disclosure, the above-mentioned R₂ is selected from CH₃, CH₂CN, CH₂COOH, CH₂CONH₂ and CH₂OCH₃, and other variables are as defined in the present disclosure.

In some embodiments of the present disclosure, the above-mentioned R₂ is selected from CN, COOH and CONH₂, and other variables are as defined in the present disclosure.

In some embodiments of the present disclosure, the above-mentioned R₂ is selected from H, and other variables are as defined in the present disclosure.

In some embodiments of the present disclosure, the above-mentioned R₂ is selected from CH₂OCH₃, and other variables are as defined in the present disclosure.

In some embodiments of the present disclosure, the above-mentioned ring A is selected from 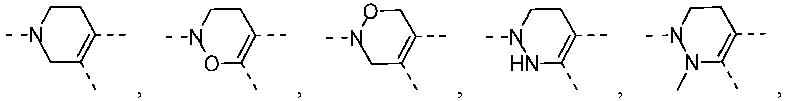 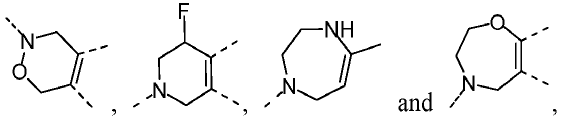 and other variables are as defined in the present disclosure.

In some embodiments of the present disclosure, the above-mentioned ring A is selected from 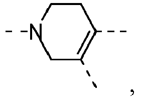 and other variables are as defined in the present disclosure.

In some embodiments of the present disclosure, the above-mentioned ring B is selected from phenyl, thienyl and pyridyl, the phenyl, thienyl and pyridyl are each independently and optionally substituted with 1, 2 or 3 R_{c}, and other variables are as defined in the present disclosure.

In some embodiments of the present disclosure, the above-mentioned ring B is selected from 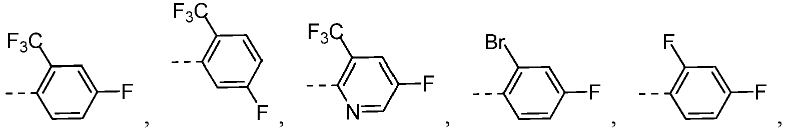 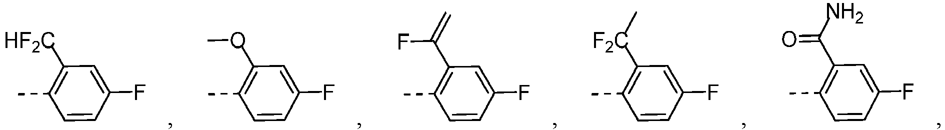 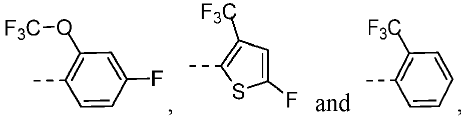 and other variables are as defined in the present disclosure.

In some embodiments of the present disclosure, the above-mentioned ring B is selected from 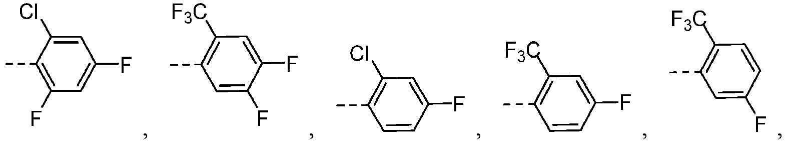 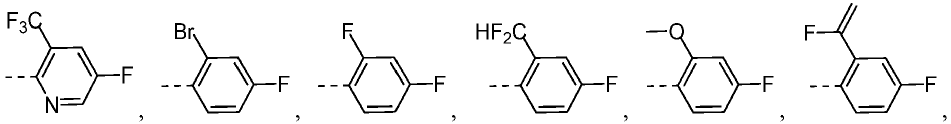 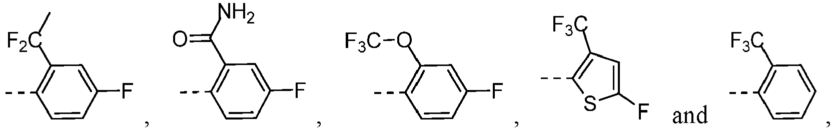 and other variables are as defined in the present disclosure.

In some embodiments of the present disclosure, the above-mentioned ring B is selected from 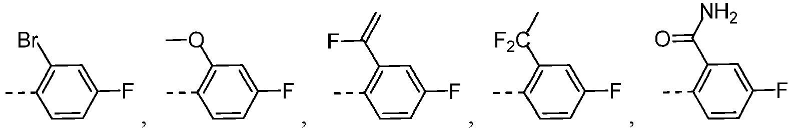 and 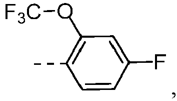 and other variables are as defined in the present disclosure.

In some embodiments of the present disclosure, the above-mentioned ring B is selected from 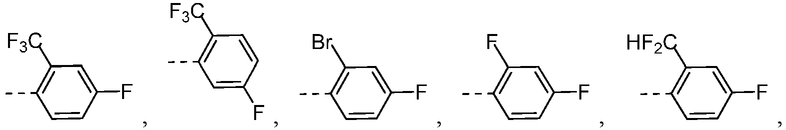 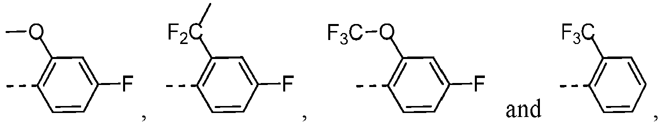 and other variables are as defined in the present disclosure.

In some embodiments of the present disclosure, the above-mentioned ring B is selected from 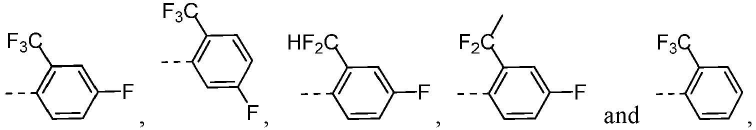 and other variables are as defined in the present disclosure.

In some embodiments of the present disclosure, the above-mentioned ring B is selected from 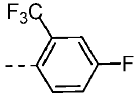 , and other variables are as defined in the present disclosure.

In some embodiments of the present disclosure, the above-mentioned ring B is selected from 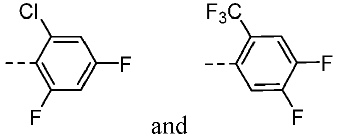 , and other variables are as defined in the present disclosure.

In some embodiments of the present disclosure, the above-mentioned ring C is selected from pyridyl and pyrimidyl, and other variables are as defined in the present disclosure.

In some embodiments of the present disclosure, the above-mentioned ring C is selected from pyrimidyl, and other variables are as defined in the present disclosure.

In some embodiments of the present disclosure, the above-mentioned compound or the pharmaceutically acceptable salt thereof is selected from 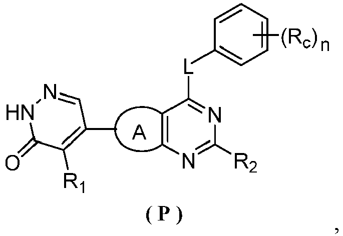 wherein
n is selected from 0, 1, 2 and 3;
R₁, R₃, R_{c}, L and ring A are as defined in the present disclosure.

In some embodiments of the present disclosure, the above-mentioned compound or the pharmaceutically acceptable salt thereof is selected from 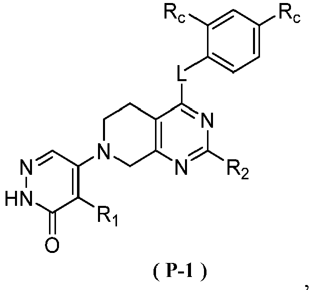 wherein R₁, R₃ and R_{c} are as defined in the present disclosure.

The present disclosure also provides a compound represented by formula (I) or a pharmaceutically acceptable salt thereof, 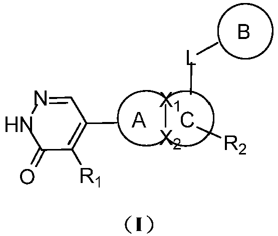 wherein
X₁ and X₂ are each independently selected from CH and N;
L is selected from -O-, -S-, -S(=O)-, -C(=O)-, -CF₂-, -CH(OCH₃)-, -N(OCH₃)-, 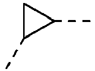 and C₂₋₃ alkenyl, and the -CH(OCH₃)-, 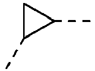 and C₂₋₃ alkenyl are optionally substituted with 1, 2 or 3 Rₐ;
R₁ is selected from F, Cl, Br and I;
R₂ is selected from H, F, Cl, Br, I, OH, NH₂, CN, COOH, CONH₂, C₁₋₃ alkyl, C₁₋₃ alkoxy and C₁₋₃ alkyl-C₁₋₃ alkoxy, and the C₁₋₃ alkyl, C₁₋₃ alkoxy and C₁₋₃ alkyl-C₁₋₃ alkoxy are each independently and optionally substituted with 1, 2 or 3 R_{b};
ring A is selected from 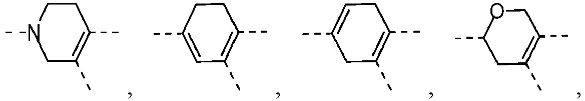 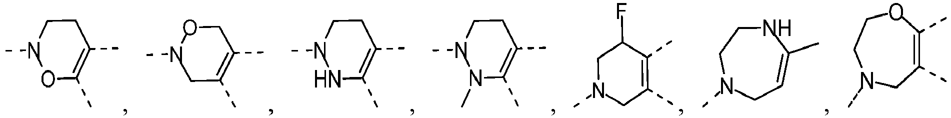 and 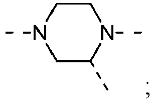
ring B is selected from phenyl and 5- to 6-membered heteroaryl, and the phenyl and 5- to 6-membered heteroaryl are each independently substituted with 1, 2 or 3 R_{c};
ring C is selected from 6-membered heteroaryl;
Rₐ is selected from F, Cl, Br, I and C₁₋₃ alkyl;
R_{b} is selected from F, Cl, Br, I, OH, NH₂, CN, COOH and CONH₂;
R_{c} is selected from F, Cl, Br, I, CONH₂, C₁₋₃ alkyl, C₁₋₃ alkoxy and C₂₋₃ alkenyl, and the C₁₋₃ alkyl, C₁₋₃ alkoxy and C₂₋₃ alkenyl are each independently and optionally substituted with 1, 2 or 3 halogen,
provided that:
   1) when R₁ is Cl, ring A is 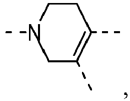 L is O, and ring B is phenyl, then at least one substituent on ring B is Br, CONH₂, CF₂CH₃, C₁₋₃ alkoxy or C₂₋₃ alkenyl;
when R₁ is Cl, ring A is 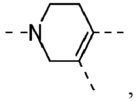 L is O, and ring B is 2-trifluoromethyl-4-fluorophenyl, then R₂ is CN, COOH, CONH₂, CH₃, CH₂CN, CH₂COOH, CH₂CONH₂ or CH₂OCH₃.

Other embodiments of the present disclosure are generated by any combination of the above-mentioned variables.

The present disclosure provides a compound represented by the following formula or a pharmaceutically acceptable salt thereof,

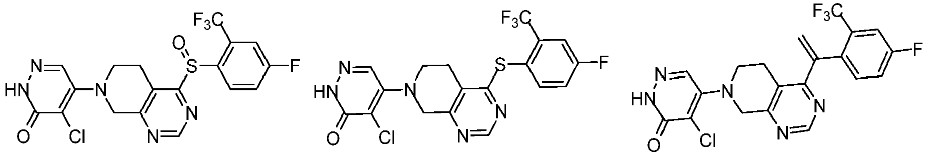

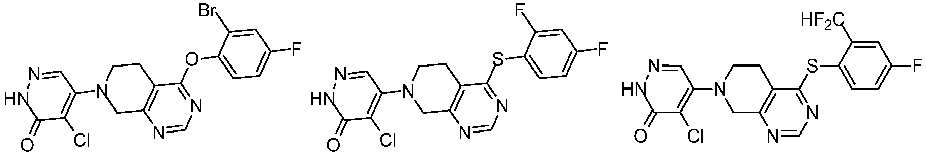

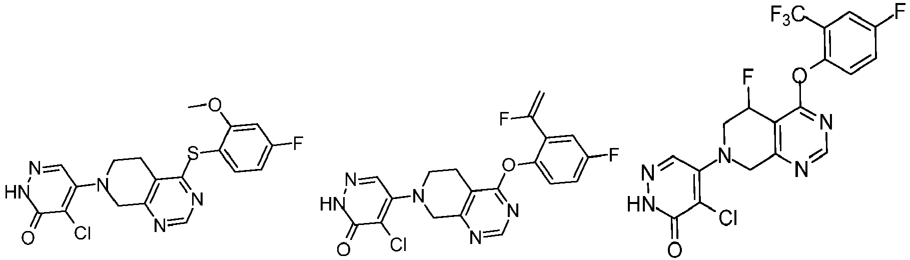

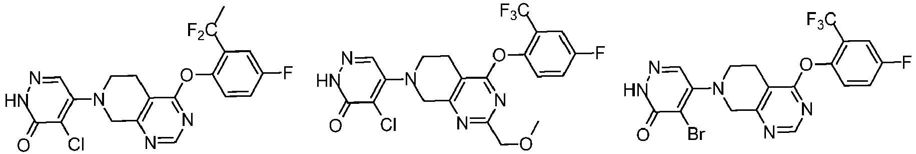

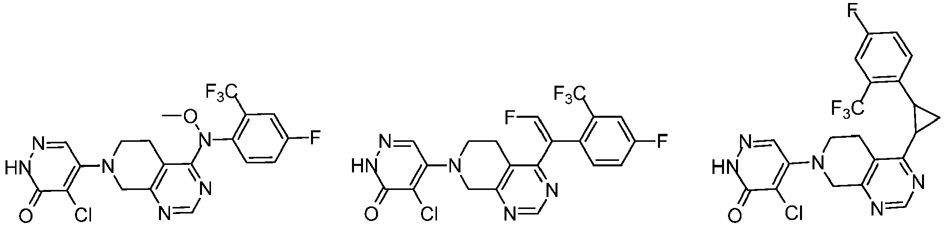

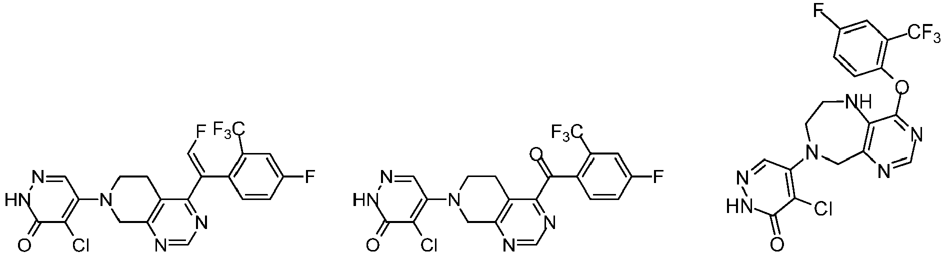

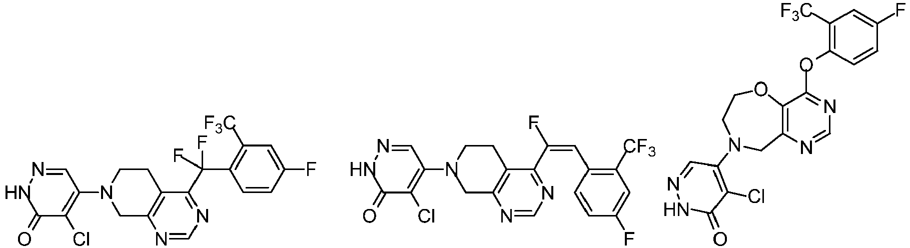

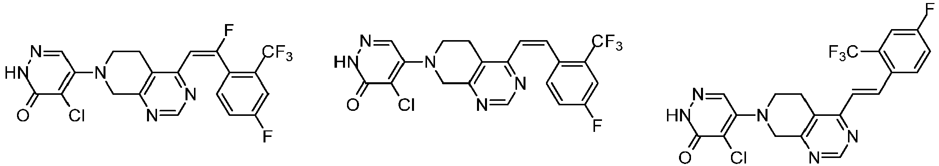

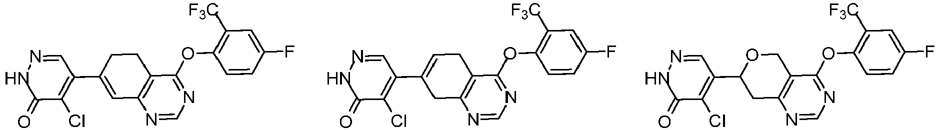

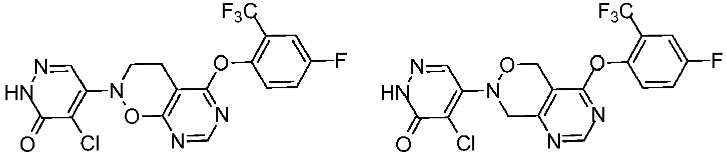

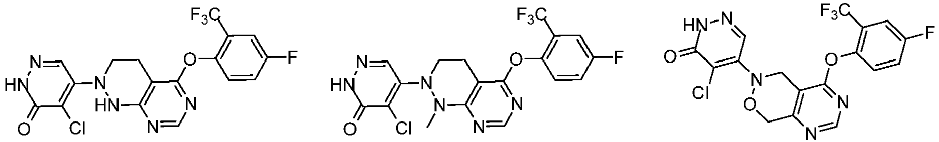

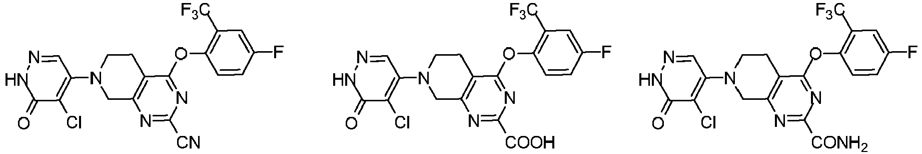

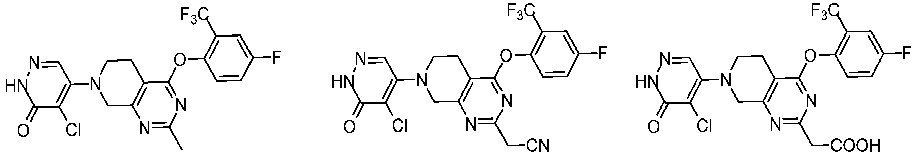

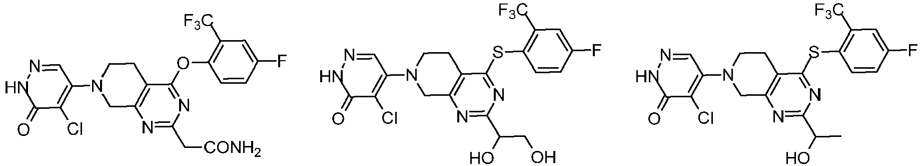

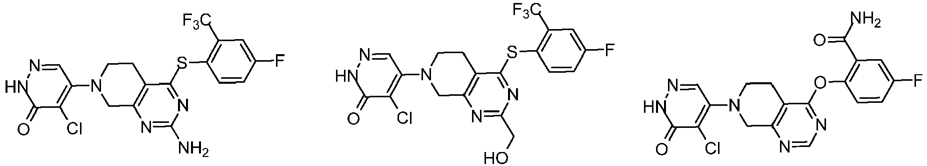

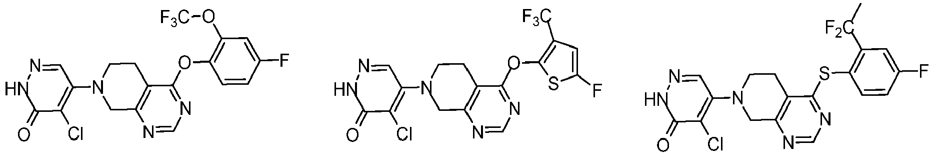

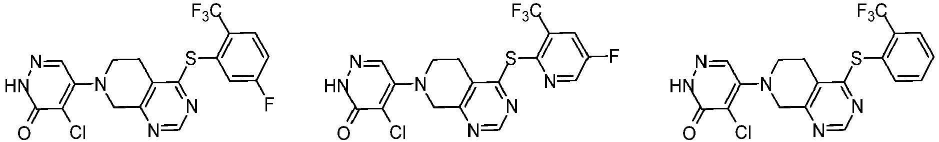

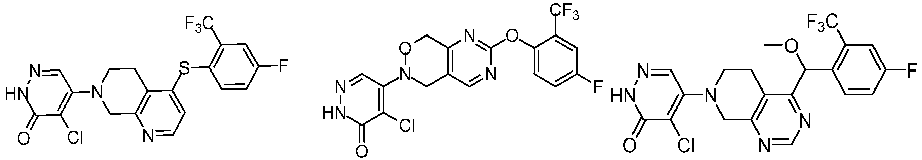

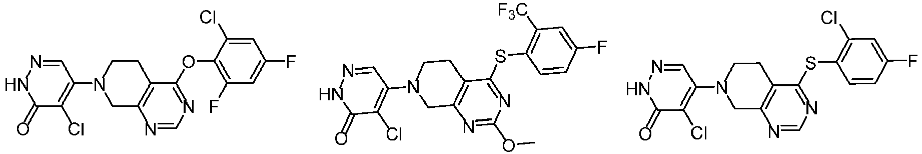

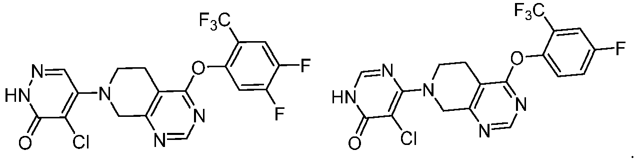

In some embodiments of the present disclosure, the above-mentioned compound or the pharmaceutically acceptable salt thereof is selected from

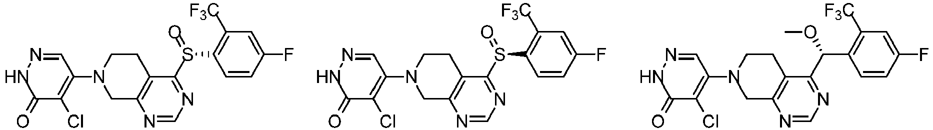

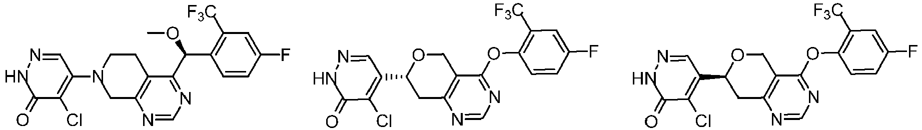

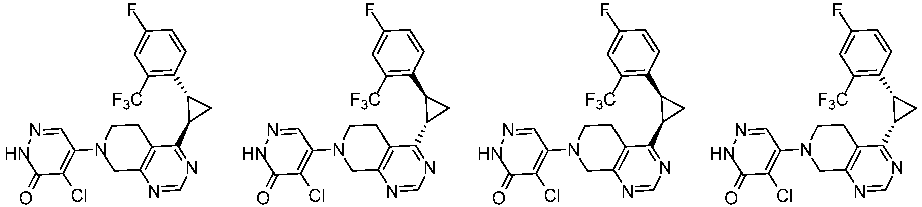

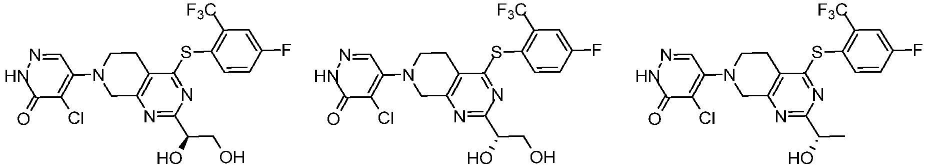

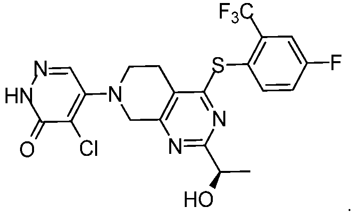

The present disclosure also provides the use of the above-mentioned compound or pharmaceutically acceptable salt thereof in a medicament related to TRPC5 inhibitors.

### Technical Effects

The compound of the present disclosure has a significant inhibitory effect on TRPC5, exhibits good pharmacokinetic properties in rats, and shows a high kidney and liver distribution and a low brain distribution. The compound can also significantly reduce the urinary albumin level in rat models of hypertensive renal disease, and dose-dependently reduces urinary Rac1 expression in rat models of hypertensive renal disease.

### Definition and Description

Unless otherwise stated, the following terms and phrases used herein are intended to have the following meanings. A specific term or phrase should not be considered uncertain or unclear unless specifically defined, but should be understood in its ordinary meaning. When a trade name appears herein, it is intended to refer to the corresponding commodity or an active ingredient thereof.

The term "pharmaceutically acceptable" as used herein refers to those compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The term "pharmaceutically acceptable salt" refers to a salt of the compound of the present disclosure, which is prepared from the compound having specific substituents found in the present disclosure with relatively non-toxic acids or bases. When the compounds of the present disclosure contain relatively acidic functional groups, base addition salts can be obtained by contacting such compounds with a sufficient amount of base, either in pure solution or a suitable inert solvent. When the compounds of the present disclosure contain relatively basic functional groups, acid addition salts can be obtained by contacting such compounds with a sufficient amount of acid, either in pure solution or a suitable inert solvent. Certain specific compounds of the present disclosure contain basic and acidic functional groups and thus can be converted to any base or acid addition salt.

The pharmaceutically acceptable salts of the present disclosure can be synthesized from a parent compound containing acid radicals or base radicals by conventional chemical methods. In general, the method for preparing such salts comprises: in water or an organic solvent or a mixture of both, reacting these compounds in free acid or base forms with a stoichiometric amount of a suitable base or acid to prepare the salts.

Unless otherwise stated, the term "isomer" is intended to include geometric isomers, cis-trans isomers, stereoisomers, enantiomers, optical isomers, diastereomers and tautomers.

The compounds of the present disclosure may exist in specific geometric or stereoisomeric forms. The present disclosure contemplates all such compounds, including cis and trans isomers, (-)- and (+)-enantiomers, (*R*)- and (*S*)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in a substituent such as an alkyl group. All these isomers and mixtures thereof are included in the scope of the present disclosure.

Unless otherwise stated, the term "enantiomer" or "optical isomer" refers to stereoisomers that are mirror images of each other.

Unless otherwise stated, the term "cis-trans isomer" or "geometric isomer" is caused by the fact that double bonds or single bonds of ring-forming carbon atoms cannot rotate freely.

Unless otherwise stated, the term "diastereomer" refers to stereoisomers in which molecules have two or more chiral centers and are not mirror images of each other.

Unless otherwise stated, "(+)" represents right-handed, "(-)" represents left-handed, and "(±)" means racemic.

Unless otherwise stated, the wedge-shaped solid bond ( 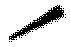 ) and the wedge-shaped dotted bond ( 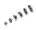 ) represent the absolute configuration of a stereoscopic center; the straight solid bond ( 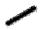 ) and the straight dotted bond ( 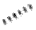 ) represent the relative configuration of a stereoscopic center; the wavy line ( 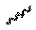 ) represents the wedge-shaped solid bond ( 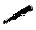 ) or the wedge-shaped dotted bond ( 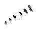 ); or the wavy line ( 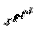 ) represents the straight solid bond ( 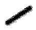 ) or the straight dotted bond ( 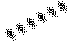 ).

Unless otherwise stated, the term "rich in one isomer", "isomer enriched", "rich in one enantiomer" or "enantiomerically enriched" refers to that the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomeric excess (ee value) is 80%.

Optically active (*R*)- and (*S*)-isomers and *D* and *L* isomers can be prepared using chiral synthesis or chiral reagents or other conventional techniques. If a particular enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as an amino group) or an acidic functional group (such as a carboxyl group), diastereomeric salts can be formed with an appropriate optically active acid or base, followed by resolution of the diastereomers using conventional methods well known in the art, and subsequent recovery of the pure enantiomers. In addition, separation of enantiomers and diastereomers is frequently accomplished using chromatography, which uses chiral stationary phases, optionally in combination with chemical derivatization methods (e.g., formation of carbamates from amines).

The compounds of the present disclosure may contain unnatural proportions of atomic isotopes at one or more of the atoms constituting the compound. For example, the compounds may be radiolabeled with radioactive isotopes, such as tritium (³H), iodine-125 (¹²⁵ I) or C-14 (¹⁴C). For another example, the hydrogen can be substituted by heavy hydrogen to form deuterated drugs. The bond formed by deuterium and carbon is stronger than the bond formed by ordinary hydrogen and carbon. Compared with undeuterated drugs, deuterated drugs have reduced toxic and side effects, increased drug stability, enhanced efficacy, prolonged biological half-life of drugs and other advantages. All isotopic variations of the compounds of the present disclosure, whether radioactive or not, are intended to be encompassed within the scope of the present disclosure.

The term "optional" or "optionally" means that the subsequently described event or circumstance may, but not necessarily occur, and that the description includes instances where said event or circumstance occurs and instances where said event or circumstance does not occur.

The term "substituted" means that any one or more hydrogen atoms on the designated atom are substituted by a substituent, which may include heavy hydrogen and hydrogen variants, provided that the valence state of the designated atom is normal, and the substituted compound is stable. Where the substituent is oxygen (i.e., =O), it means that two hydrogen atoms are substituted. Oxygen substitution does not occur on aromatic groups. The term "optionally substituted" means that it may or may not be substituted. Unless otherwise specified, the type and number of substituents may be arbitrary on the basis that they can be achieved in chemistry.

Where any variable (such as R) appears more than once in the composition or structure of a compound, its definition in each case is independent. Thus, for example, if a group is substituted with 0-2 R, the group can optionally be substituted with up to two R, and R in each case has independent options. In addition, combinations of substituents and/or variants thereof are permissible only if such combinations result in stable compounds.

When the number of a linking group is 0, such as -(CRR)₀-, it means that the linking group is a single bond.

When the number of a substituent is 0, it means that the substituent does not exist. For example, -A-(R)₀ means that the structure is actually -A.

When a substituent is vacant, it means that the substituent does not exist. For example, when X is vacant in A-X, it means that the structure is actually A.

When one of the variables is selected from a single bond, it means that the two groups to which it is connected are directly connected. For example, when L represents a single bond in A-L-Z, it means that the structure is actually A-Z.

When the bond of a substituent can be cross-connected to more than two atoms on a ring, the substituent can be bonded to any atom on the ring, for example, the structural moiety 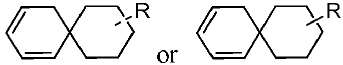 indicates that the substituent R can be substituted at any position on the cyclohexyl or cyclohexadiene. When the substituents listed do not indicate through which atom they are connected to the substituted group, such substituents can be bonded through any of the atoms thereof, for example, pyridyl as a substituent can be attached to the substituted group via any carbon atom on the pyridine ring.

When the linking group listed does not indicate the linking direction thereof, the linking direction is arbitrary, for example, the linking group L is -M-W- in 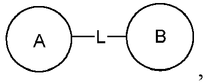 at this situation, -M-W- can connect ring A and ring B in the same direction as the reading order from left to right to form 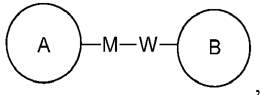 and can also connect ring A and ring B in the opposite direction as the reading order from left to right to form 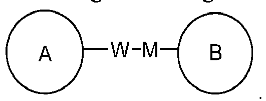 Combinations of the linking groups, substituents, and/or variants thereof are permissible only if such combinations result in stable compounds.

Unless otherwise specified, when a group has one or more connectable sites, any one or more sites of the group can be connected to other groups through chemical bonds. When the connection mode of the chemical bond is not positioned, and there is an H atom at the connectable site, the number of H atoms at the site will decrease correspondingly with the number of chemical bonds connected to become a group with the corresponding valence when the chemical bond is connected. The chemical bonds between the sites and other groups can be represented by a straight solid bond ( 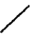 ), a straight dotted bond ( 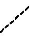 ), or a wavy line ( 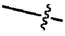 ). For example, the straight solid bond in -OCH₃ means that the group is connected to other groups through the oxygen atom in the group; the straight dashed bond in 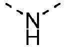 means that the group is connected to other groups through the two ends of the nitrogen atom in the group; the wavy line in 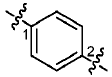 means that the group is connected to other groups through the 1 and 2 carbon atoms in the phenyl group; 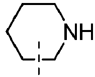 means that any connectable site on the piperidinyl can be connected to other groups through one chemical bond, including at least four connection modes: 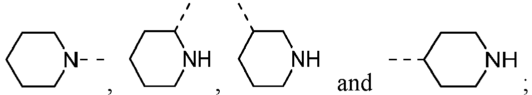 even if the H atom is drawn on -N-, 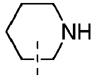 still includes the group of the connection mode 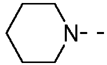 ; but the H at the site will decrease correspondingly by one and become the corresponding monovalent piperidinyl when one chemical bond is connected.

Unless otherwise specified, the number of atoms in a ring is usually defined as the member number of the ring. For example, "5- to 7-membered ring" means a "ring" with 5-7 atoms arranging in a circle.

Unless otherwise specified, the term "halo" or "halogen" by itself or as part of another substituent means a fluorine, chlorine, bromine or iodine atom.

Unless otherwise specified, the term "C₁₋₃ alkyl" is used to represent a linear or branched saturated hydrocarbon group consisting of 1 to 3 carbon atoms. The C₁₋₃ alkyl includes C₁₋₂ alkyl, C₂₋₃ alkyl, *etc.*; and it can be monovalent (such as methyl), divalent (such as methylene) or multivalent (such as methine). Examples of C₁₋₃ alkyl include, but are not limited to, methyl (Me), ethyl (Et), propyl (including *n*-propyl and isopropyl), etc.

Unless otherwise specified, the term "C₁₋₃ alkoxy" means those alkyl groups comprising 1 to 3 carbon atoms that are connected to the rest of the molecule through one oxygen atom. The C₁₋₃ alkoxy includes C₁₋₂ alkoxy, C₂₋₃ alkoxy, C₃ alkoxy, C₂ alkoxy, etc. Examples of C₁₋₃ alkoxy include but are not limited to methoxy, ethoxy, propoxy (including n-propoxy and isopropoxy), etc.

Unless otherwise specified, "C₂₋₃ alkenyl" is used to represent a linear or branched hydrocarbon group consisting of 2 to 3 carbon atoms and comprising at least one carbon-carbon double bond, wherein the carbon-carbon double bond may be located at any position of the group. The C₂₋₃ alkenyl includes C₃ alkenyl and C₂ alkenyl; and the C₂₋₃ alkenyl can be monovalent, bivalent or multivalent. Examples of C₂₋₃ alkenyl include, but are not limited to ethenyl, propenyl, etc.

Unless otherwise specified, the terms "5- to 6-membered heteroaryl ring" and "5- to 6-membered heteroaryl" of the present disclosure can be used interchangeably, and the term "5- to 6-membered heteroaryl" represents a monocyclic group having a conjugated *π*-electron system and consisting of 5 to 6 ring atoms, of which 1, 2, 3 or 4 ring atoms are heteroatoms independently selected from O, S and N, and the rest of which are carbon atoms, wherein the nitrogen atom is optionally quaternized, and the nitrogen and sulfur heteroatoms can be optionally oxidized (i.e., NO and S(O)ₚ, wherein p is 1 or 2). The 5- to 6-membered heteroaryl can be connected to the rest of the molecule through a heteroatom or a carbon atom. The 5-to 6-membered heteroaryl includes 5-membered and 6-membered heteroaryl. Examples of the 5- to 6-membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (1*H*-1,2,3-triazolyl, 2*H*-1,2,3-triazolyl, 1*H-*1,2,4-triazolyl, 4*H*-1,2,4-triazolyl, etc.), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furyl (including 2-furyl, 3-furyl, etc.), thienyl (including 2-thienyl, 3-thienyl, etc.), pyridyl (including 2-pyridyl, 3-pyridyl, 4-pyridyl, etc.), pyrazinyl or pyrimidyl (including 2-pyrimidyl, 4-pyrimidyl, etc.).

The term "protecting group" includes, but is not limited to, "amino protecting group", "hydroxyl protecting group" or "mercapto protecting group". The term "amino protecting group" refers to a protecting group suitable for preventing side reactions occurring at the nitrogen atom of an amino group. Representative amino protecting groups include, but are not limited to: formyl; acyl, such as alkanoyl (e.g., acetyl, trichloroacetyl or trifluoroacetyl); alkoxycarbonyl, such as tert-butoxycarbonyl (Boc); aryl methoxycarbonyl, such as benzyloxycarbonyl (Cbz) and 9-fluorenylmethoxycarbonyl (Fmoc); aryl methyl, such as benzyl (Bn), triphenylmethyl (Tr), 1,1-bis-(4'-methoxyphenyl)methyl; silyl such as trimethylsilyl (TMS) and tert-butyl dimethylsilyl (TBS). The term "hydroxyl protecting group" refers to a protecting group suitable for preventing side reactions of a hydroxyl group. Representative hydroxyl protecting groups include, but are not limited to: alkyl, such as methyl, ethyl and tert-butyl; acyl, such as alkanoyl (e.g., acetyl); arylmethyl such as benzyl (Bn), p-methoxybenzyl (PMB), 9-fluorenylmethyl (Fm) and diphenylmethyl (benzhydryl, DPM); silyl such as trimethylsilyl (TMS) and tert-butyl dimethylsilyl (TBS).

The structure of the compound of the present disclosure can be confirmed by conventional methods well known to a person skilled in the art. If the present disclosure relates to the absolute configuration of the compound, the absolute configuration can be confirmed by conventional technical means in the art. For example, single-crystal X-ray diffraction (SXRD) uses a Bruker D8 venture diffractometer to collect the diffraction intensity data of the cultivated single crystal, with a light source of CuKα radiation, and a scanning mode of ϕ/ω scanning. After the related data is collected, a direct method (Shelxs97) is further used to resolve the crystal structure, so that the absolute configuration can be confirmed.

The compounds of the present disclosure can be prepared by various synthetic methods well known to a person skilled in the art, including the specific embodiments listed below, the embodiments formed by the combination with other chemical synthesis methods, and equivalent alternative embodiments well known to a person skilled in the art, wherein the preferred embodiments include but are not limited to the examples of the present disclosure.

The present disclosure uses the following abbreviations: aq represents water; *eq* represents equivalent; DCM represents dichloromethane; PE represents petroleum ether; DMSO represents dimethyl sulfoxide; EA or EtOAc represents ethyl acetate; EtOH represents ethanol; MeOH represents methanol; DMF represents N,N-dimethylformamide; Cbz represents benzyloxycarbonyl, which is an amine protecting group; Boc represents tert-butoxycarbonyl, which is an amine protecting group; r.t. represents room temperature; O/N represents overnight; THF represents tetrahydrofuran; Boc₂O represents di-tert-butyl dicarbonate; TFA represents trifluoroacetic acid; HCl represents hydrochloric acid; iPrOH represents 2-propanol; mp represents melting point; Pd(PPh₃)₄ represents tetrakis(triphenylphosphine)palladium; Pd(dppf)Cl₂ represents [1,1'-bis(diphenylphosphino)ferrocene]palladium dichloride; DIBAL-H represents diisobutylaluminum hydride; FA represents formic acid; ACN represents acetonitrile; DEA represents diethylamine; THP represents 2-tetrahydropyran ether group; prep-TLC represents preparative thin layer chromatographic separation; DIEA represents N,N-diisopropylethylamine; DMA represents N,N-dimethylacetamide; DBU represents 1.8-diazabicyclo[5.4.0]undec-7-ene.

The solvents used in the present disclosure are commercially available. Compounds are named according to conventional naming principles in the field or using ChemDraw^{®} software, and commercially available compounds are named using supplier catalog names.

### BRIEF DESCRIPTION OF THE DRAWINGS

FIG. 1 shows the urinary albumin endpoint results of male rat models of hypertensive renal disease induced with deoxycorticosterone acetate-sodium chloride (DOCA-NaCl).
FIG. 2 shows the urinary Rac1/creatinine endpoint results of male rat models of hypertensive renal disease induced with deoxycorticosterone acetate-sodium chloride (DOCA-NaCl).

### DETAILED DESCRIPTION OF THE PREFERRED EMBODIMENT

The present disclosure will be described in detail with the following examples, but not imply any adverse limitation to the present disclosure. The present disclosure has been described in detail herein, and the specific embodiments thereof are also disclosed therein. For a person skilled in the art, without departing from the spirit and scope of the present disclosure, all the variations and improvements made to the specific embodiments of the present disclosure would have been obvious.

### Reference example 1: Fragment A-1

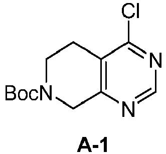

### Synthetic route:

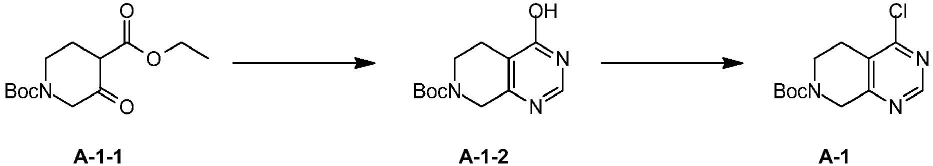

### Step 1: Synthesis of compound A-1-2

Sodium ethoxide (3.2 g, 47.02 mmol, 2.55 *eq*) was dissolved in ethanol (100 mL). Under nitrogen protection, compound **A-1-1** (5 g, 18.43 mmol, 1 *eq*) and formamidine acetate (2.9 g, 27.86 mmol, 1.51 *eq*) were added. The reaction was stirred at 100 °C for 5 hours. After cooling, 250 mL of water was added, and the mixture was extracted with dichloromethane (3 × 250 mL). The organic phases were combined, washed with saturated brine, dried over anhydrous sodium sulfate, and concentrated under reduced pressure to obtain a crude. Ethyl acetate (75 mL) was added to the crude, and the resulting mixture was stirred for 2 hours and then filtered. The filter cake was washed with 5 mL of ethyl acetate and dried to obtain compound **A-1-2**. ¹H NMR (400 MHz, CDCl₃) δ ppm 1.42 - 1.56 (m, 9 H), 2.64 (br s, 2 H), 3.65 (t, *J*=5.77 Hz, 2 H), 4.43 (br s, 2 H), 8.07 (s, 1 H), 12.62 (br s, 1 H).

### Step 2: Synthesis of compound A-1

Compound **A-1-2** (18 g, 71.63 mmol, 1 *eq*) and trichloroacetonitrile (15.55 g, 107.71 mmol, 1.5 *eq*) were suspended in toluene (250 mL). Triphenylphosphine (56.00 g, 213.51 mmol, 2.98 *eq*) was added. Under nitrogen protection, the mixture was stirred at 120 °C for 1.5 hours. The reaction liquid was concentrated under reduced pressure and to the residue was added 250 mL of water. The resulting mixture was extracted with dichloromethane (3 × 150 mL). The organic phases were combined, washed with saturated brine, dried over anhydrous sodium sulfate, and concentrated under reduced pressure to obtain a crude. The crude was purified by column chromatography (petroleum ether : ethyl acetate = 10 : 1) to obtain compound **A-1-3**. ¹H NMR (400 MHz, CDCl₃) δ ppm 1.50 (s, 9 H), 2.88 (t, *J*=5.52 Hz, 2 H), 3.75 (t, J=5.77 Hz, 2 H), 4.65 (s, 2 H), 8.80 (s, 1 H).

### Reference example 2: Fragment A-2

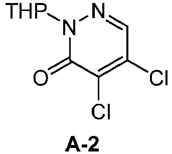

### Synthetic route:

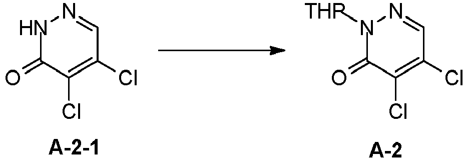

**A-2-1** (186 g, 1.13 mol, 1 *eq*) was added to a reaction flask containing tetrahydrofuran (1116 mL). Pyridinium p-toluenesulfonate (56.66 g, 225.49 mmol, 0.2 *eq)* and 3,4-dihydro-2H-pyran (142.25 g, 1.69 mol, 154.62 mL, 1.5 *eq*) were added and the mixture was reacted at 100 °C for 5 hours. Then 3,4-dihydro-2H-pyran (94.83 g, 1.13 mol, 103.08 mL, 1 *eq*) was added and the mixture was reacted at 100 °C for 12 hours. The reaction liquid was concentrated and dissolved in ethyl acetate. The organic phase was sequentially washed with 2 M sodium hydroxide solution and saturated brine, dried over anhydrous sodium sulfate, filtered and concentrated to obtain a crude. The crude was preliminarily purified by column chromatography (petroleum ether : ethyl acetate = 1 : 0-15 : 1-10 : 1-5 : 1-2.5 : 1) and then slurried with petroleum ether to obtain compound **A-2**. LCMS: m/z [M+23]⁺=271.

### Reference example 3: Fragment A-3

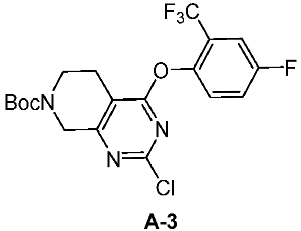

### Synthetic route:

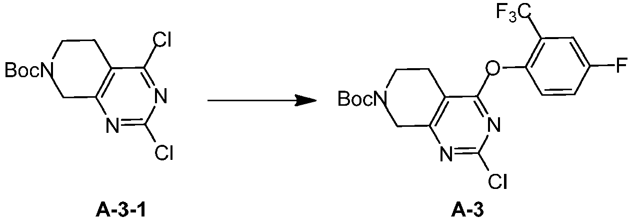

**A-3-1** (35 g, 115.07 mmol, 1 *eq*) was added to a reaction flask containing acetonitrile (300 mL). Potassium carbonate (31.81 g, 230.13 mmol, 2 *eq*), and a solution of 4-fluoro-2-(trifluoromethyl)phenol (24.87 g, 138.08 mmol, 1.2 *eq*) in acetonitrile (240 mL) were added and the mixture was reacted at 50 °C for 16 hours. To the reaction liquid was added 300 mL of water and the mixture was extracted with ethyl acetate (3 × 300 mL). The organic phases were combined, washed with saturated brine, dried over anhydrous sodium sulfate, subjected to suction filtration, and concentrated to obtain a crude. To the crude was added a mixed solvent (petroleum ether : ethyl acetate = 9 : 1) and the mixture was stirred and then subjected to suction filtration. The filter cake was washed with a mixed solvent (petroleum ether : ethyl acetate = 9 : 1) and concentrated to obtain a filter cake. The filter cake was purified by column chromatography (petroleum ether : ethyl acetate = 1 : 0-9 : 1-4 : 1-0 : 1) to obtain compound **A-3**. ¹H NMR (400 MHz, CDCl₃) δ ppm 1.51 (s, 9 H), 2.86 (br t, J=5.02 Hz, 2 H), 3.76 (t, J=5.77 Hz, 2 H), 4.63 (s, 2 H), 7.31 - 7.38 (m, 2 H), 7.40 - 7.48 (m, 1 H); LCMS: m/z [M-55]⁺=391.9.

### Example 1

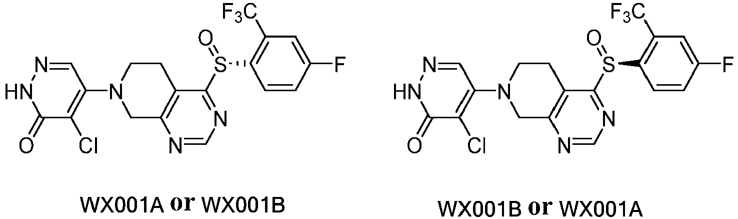

### Synthetic route:

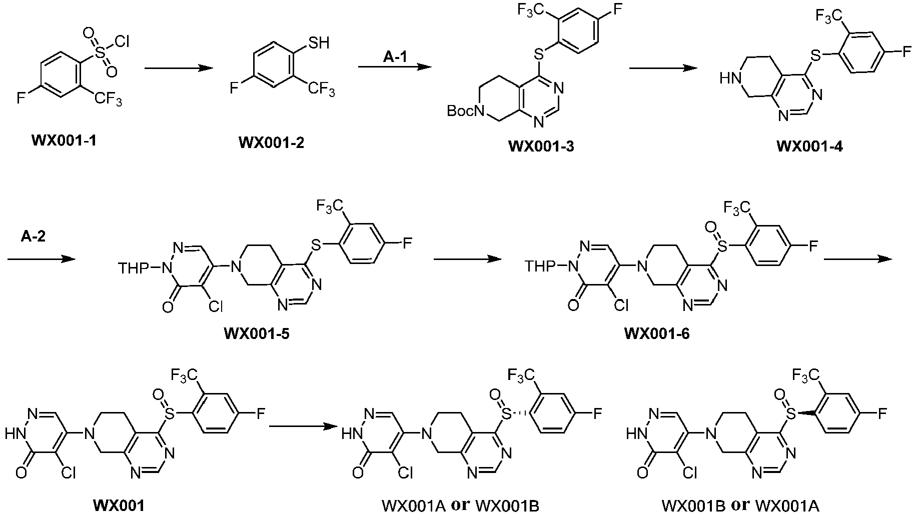

### Step 1: Synthesis of compound WX001-2

Under nitrogen protection, to a reaction flask containing 1,4-dioxane (30 mL) was added **WX001-1** (5 g, 19.04 mmol, 1 *eq*), followed by water (7.5 mL) and tris(2-carboxyethyl)phosphine hydrochloride (21.83 g, 76.14 mmol, 4 *eq)*, and the resulting mixture was reacted at 120 °C for 2 hours. The reaction system was cooled to room temperature. Water (75 mL) was added and the mixture was extracted with dichloromethane (3 × 100 mL). The organic phases were combined, washed with saturated brine, dried over anhydrous sodium sulfate, filtered and concentrated to obtain a crude. The crude was purified by low-pressure column chromatography (petroleum ether : ethyl acetate = 10 : 1) to obtain compound **WX001-2**. ¹H NMR (400 MHz, DMSO-*d*₆) δ ppm 6.13 (br s, 1 H), 7.33 - 7.97 (m, 3 H).

### Step 2: Synthesis of compound WX001-3

To a reaction flask containing N,N-dimethylformamide (27 mL) were added **A-1** (2.47 g, 9.16 mmol, 1 *eq*) and **WX001-2** (1.89 g, 9.62 mmol, 1.05 *eq*), followed by 1,8-diazabicyclo[5.4.0]undec-7-ene (2.79 g, 18.31 mmol, 2.76 mL, 2 *eq*), and the resulting mixture was reacted at 100 °C for 0.5 hours. The reaction system was cooled to room temperature and 300 mL of ethyl acetate was added. The organic phase was sequentially washed with half-saturated brine (5 × 30 mL) and brine, dried over anhydrous sodium sulfate, filtered and concentrated to obtain a crude. The crude was purified by column chromatography (petroleum ether : ethyl acetate = 1 : 0-4 : 1) to obtain compound **WX001-3**. LCMS: m/z [M+1]⁺=430.

### Step 3: Synthesis of compound WX001-4

To a reaction flask containing dichloromethane (76 mL) was added **WX001-3** (1.64 g, 3.82 mmol, 1 eq), followed by trifluoroacetic acid (13.06 g, 114.57 mmol, 8.48 mL, 30 eq), and the resulting mixture was reacted at 20 °C for 0.5 hours. To the reaction liquid was added 70 mL of water and the mixture was adjusted to pH = 8 with sodium carbonate. The resulting mixture was extracted with dichloromethane (3 × 70 mL). The organic phases were combined, washed with saturated brine, dried over anhydrous sodium sulfate, filtered and concentrated to obtain **WX001-4**. LCMS: m/z [M+1]⁺ = 330.

### Step 4: Synthesis of compound WX001-5

To a reaction flask containing N,N-diisopropylethylamine (64.75 g, 500.96 mmol, 87.26 mL, 57.48 *eq*) was added **WX001-4** (2.87 g, 8.72 mmol, 1 *eq*), followed by **A-2** (2.39 g, 9.59 mmol, 1.1 *eq*), and the resulting mixture was reacted at 100 °C for 16 hours. The reaction liquid was directly concentrated to obtain a crude. The crude was purified by column chromatography (petroleum ether : ethyl acetate = 1 : 0-1 : 1) to obtain compound **WX001-5**. LCMS: m/z [M+1]⁺ = 542.

### Step 5: Synthesis of compound WX001-6

**WX001-5** (100 mg, 184.52 µmοl, 1 *eq*) was added to a reaction flask containing dichloromethane (4.4 mL). At 0 °C, a solution of 3-chloroperoxybenzoic acid (44.95 mg, 221.42 µmοl, purity: 85%, 1.2 *eq*) in dichloromethane (2.2 mL) was added and the resulting mixture was reacted at 0 °C for 3 hours. To the reaction liquid was added saturated sodium thiosulfate solution to quench the reaction (a potassium iodide-starch test paper, when wetted with the reaction solution, not turning blue). Subsequently, sodium carbonate was added to adjust the system to pH = 8. The reaction system was extracted with dichloromethane (3 × 4 mL). The organic phases were combined, washed with saturated brine, dried over anhydrous sodium sulfate, filtered and concentrated to obtain a crude. The crude was purified by column chromatography (petroleum ether : ethyl acetate = 1 : 0-2 : 3) to obtain compound **WX001-6**. ¹H NMR (400 MHz, CDCl₃) δ ppm 1.60 - 1.82 (m, 4 H), 2.04 - 2.21 (m, 2 H), 3.33 - 3.51 (m, 1 H), 3.54 - 3.68 (m, 1 H), 3.77 (br t, J=11.54 Hz, 1 H), 3.81 - 3.95 (m, 2 H), 4.12 - 4.17 (m, 1 H), 4.71 (d, J=4.52 Hz, 2 H), 6.01 - 6.15 (m, 1 H), 7.48 (dd, J=8.03, 2.51 Hz, 1 H), 7.53 - 7.63 (m, 1 H), 7.79 (s, 1 H), 8.38 - 8.45 (m, 1 H), 8.94 (s, 1 H); LCMS: m/z [M+1]⁺ = 558.

### Step 6: Synthesis of compound WX001

To a reaction flask containing dichloromethane (22 mL) was added **WX001-6** (1.23 g, 2.20 mmol, 1 *eq*), followed by trifluoroacetic acid (7.54 g, 66.14 mmol, 4.90 mL, 30 *eq*), and the resulting mixture was reacted at 20 °C for 0.5 hours. To the reaction liquid was added 22 mL of water and the mixture was adjusted to pH = 8 with sodium carbonate. The resulting mixture was extracted with dichloromethane (3 × 22 mL). The organic phases were combined, washed with saturated brine, dried over anhydrous sodium sulfate, filtered and concentrated to obtain a crude. To the crude was added 5 mL of dichloromethane, and the mixture was ultrasonically treated for 2 minutes; the mixture was subjected to suction filtration, to the filter cake was added 3 mL of methanol, and the resulting mixture was ultrasonically treated for 2 minutes; the mixture was subjected to suction filtration, and the filter cake was dried to obtain compound **WX001**. LCMS: m/z [M+1]⁺ = 474.

### Step 7: Synthesis of compounds WX001A and WX001B

**WX001-7** was subjected to resolution by SFC (chromatographic column: DAICEL CHIRALPAK IG (250 mm * 30 mm, 10 µm); mobile phase: A: CO₂, B: [0.1% NH₃H₂O-EtOH]; gradient B%: 60%-60%) to obtain **WX001A** and **WX001B.**

**WX001A:** (retention time: 3.558 minutes, ee = 100%). ¹H NMR (400 MHz, CD₃CN) δ ppm 3.24 - 3.36 (m, 1 H), 3.40 - 3.51 (m, 1 H), 3.80 (t, J=5.77 Hz, 2 H), 4.70 (s, 2H), 7.59 - 7.71 (m, 2 H), 7.80 (s, 1 H), 8.29 (dd, J=8.78, 5.27 Hz, 1 H), 8.88 (s, 1 H), 10.92 (br s, 1 H); LCMS: m/z [M+1]⁺= 474.

**WX001B:** (retention time: 1.738 minutes, ee = 100%). ¹H NMR (400 MHz, CD₃CN) δ ppm 3.19 - 3.38 (m, 1 H), 3.39 - 3.53 (m, 1 H), 3.80 (t, J=5.77 Hz, 2 H), 4.70 (s, 2 H), 7.54 - 7.73 (m, 2 H), 7.80 (s, 1 H), 8.29 (dd, J=8.78, 5.27 Hz, 1 H), 8.88 (s, 1 H), 10.93 (br s, 1 H); LCMS: m/z [M+1]⁺= 474.

SFC analysis and detection conditions: chromatographic column: Chiralpak IG-3 50 × 4.6 mm I.D., 3 µm; mobile phase: phase A: CO₂; phase B: ethanol (0.05% DEA); gradient B%: 40%.

### Example 2

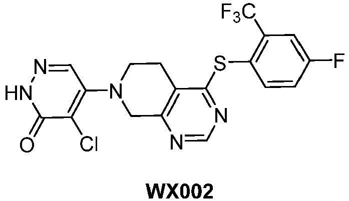

### Synthetic route:

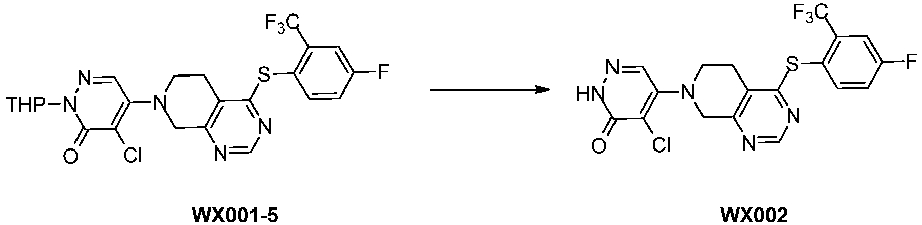

To a reaction flask containing dichloromethane (160 mL) was added **WX001-5** (16 g, 29.52 mmol, 1 *eq*), followed by trifluoroacetic acid (100 mL), and the resulting mixture was stirred at 25 °C for 0.5 hours. The reaction system was cooled to 0 °C and the reaction liquid was adjusted to pH = 8 by dropwise adding saturated sodium carbonate solution. The resulting mixture was subj ected to suction filtration to obtain a filter cake. The filter cake was washed with dichloromethane and then dried to obtain compound **WX002**. ¹H NMR (400 MHz, DMSO-*d*₆) δ ppm 2.90 (br t, *J*=5.27 Hz, 2 H), 3.80 (t, *J*=5.52 Hz, 2 H), 4.63 (s, 2 H), 7.71 (td, *J*=8.47, 2.89 Hz, 1 H), 7.84 - 7.93 (m, 2 H), 8.00 (s, 1 H), 8.61 (s, 1 H), 13.00 (br s, 1 H); LCMS: m/z [M+1]⁺ = 458.

### Example 3

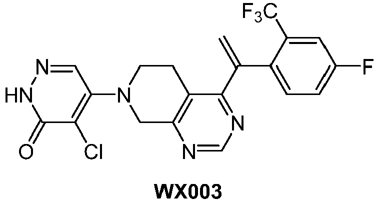

### Synthetic route:

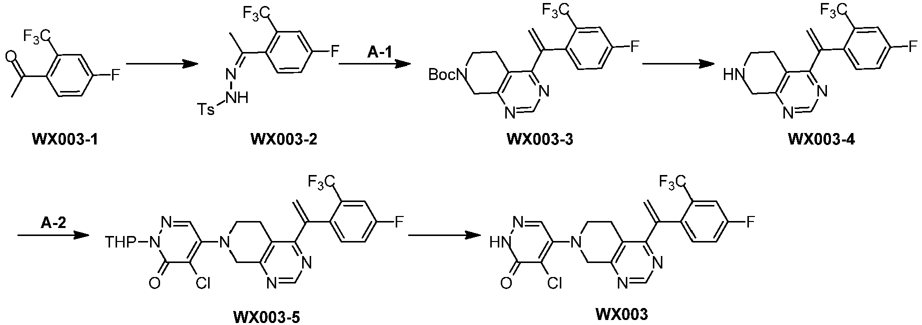

### Step 1: Synthesis of compound WX003-2

**WX003-1** (8 g, 38.81 mmol, 1 *eq*) was added to a reaction flask containing ethanol (160 mL). The reaction system was subjected to nitrogen replacement. Then p-methylbenzenesulfonohydrazide (7.23 g, 38.81 mmol, 1 *eq*) was added in portions and the resulting mixture was reacted at 90 °C for 12 hours. The mixture was concentrated to obtain a crude. To the crude was added 10 mL of methanol. The mixture was stirred for 5 minutes and subjected to suction filtration. The filter cake was dried to obtain compound **WX003-2**. LCMS: m/z [M+1]⁺ = 375.

### Step 2: Synthesis of compound WX003-3

Under nitrogen protection, to a reaction flask containing 1,4-dioxane (60 mL) were added **WX003-2** (3.74 g, 10.00 mmol, 1 *eq*), tris(dibenzylideneacetone)dipalladium (915.72 mg, 1.00 mmol, 0.1 *eq*), and 2-dicyclohexylphosphino-2',4',6'-triisopropylbiphenyl (953.43 mg, 2.00 mmol, 0.2 *eq*), followed by lithium tert-butoxide (1.76 g, 22.00 mmol, 1.98 mL, 2.2 *eq*), and the resulting mixture was stirred for 1 minute; **A-1** (2.70 g, 10 mmol, 1 *eq*) was added and the resulting mixture was reacted at 100 °C for 12 hours. The reaction system was cooled to room temperature, and ethyl acetate was added. The resulting mixture was filtered through diatomaceous earth and concentrated to obtain a crude. The crude was purified by column chromatography (petroleum ether : ethyl acetate = 1 : 0-4 : 1) to obtain compound **WX003-3**. LCMS: m/z [M+1]⁺= 424.

### Step 3: Synthesis of compound WX003-4

To a reaction flask containing dichloromethane (6.5 mL) was added **WX003-3** (1.82 g, 4.30 mmol, 1 *eq*), followed by trifluoroacetic acid (2.60 g, 22.78 mmol, 1.69 mL, 5.3 *eq*), and the resulting mixture was reacted at 20 °C for 12 hours. Water (10 mL) was added and the mixture was adjusted to pH = 8 with sodium carbonate, and then extracted with dichloromethane (3 × 10 mL). The organic phases were combined, washed with saturated brine, dried over anhydrous sodium sulfate, filtered and concentrated to obtain a crude. The crude was purified by column chromatography (dichloromethane : methanol = 1 : 0-9 : 1) to obtain compound **WX003-4**. LCMS: m/z [M+1]⁺ = 324.

### Step 4: Synthesis of compound WX003-5

To a reaction flask containing N,N-diisopropylethylamine (25.25 g, 195.34 mmol, 34.02 mL, 69.56 *eq*) was added **WX003-4** (986.1 mg, 2.81 mmol, purity: 92.07%, 1 *eq*), followed by **A-2** (839.45 mg, 3.37 mmol, 1.2 *eq*), and the resulting mixture was reacted at 100 °C for 16 hours. The mixture was concentrated to obtain a crude. The crude was purified by column chromatography (petroleum ether : ethyl acetate = 1 : 0-0 : 1) to obtain compound **WX003-5**. LCMS: m/z [M+1]⁺ = 536.

### Step 5: Synthesis of compound WX003

To a reaction flask containing dichloromethane (10 mL) was added **WX003-5** (0.3 g, 559.78 µmοl, purity: 100%, 1 *eq*), followed by trifluoroacetic acid (1.91 g, 16.79 mmol, 1.24 mL, 30 *eq)*, and the resulting mixture was reacted at 20 °C for 0.5 hours. Water (10 mL) was added and the mixture was adjusted to pH = 8 with sodium carbonate. At this moment, a solid was precipitated out from the organic phase. The aqueous phase was extracted with a mixed solvent (DCM : MeOH = 20 : 1). The organic phases were combined, washed with saturated brine and concentrated to obtain a crude. To the crude were added 5 mL of dichloromethane and 5 mL of methanol, and the resulting mixture was stirred for 10 minutes, and subjected to suction filtration. The filter cake was dried to obtain compound **WX003**. ¹H NMR (400 MHz, DMSO-*d*₆) δ ppm 3.07 (br s, 2 H), 3.70 (br s, 2 H), 4.73 (s, 2 H), 5.94 (s, 1 H), 6.12 (s, 1 H), 7.52 - 7.73 (m, 3 H), 8.00 (s, 1 H), 8.87 (s, 1 H), 12.97 (br s, 1 H); LCMS: m/z [M+1]⁺ = 452.

### Example 4

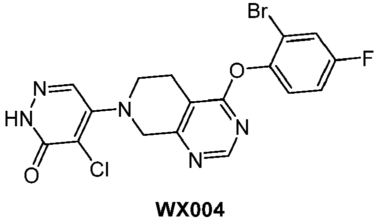

### Synthetic route:

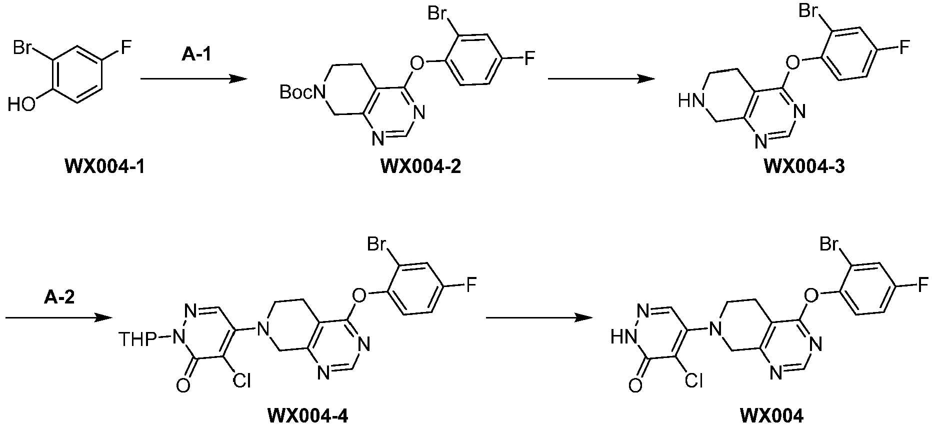

### Step 1: Synthesis of compound WX004-2

**A-1** (34 g, 126.05 mmol, 1 *eq*) was added to a reaction flask containing N,N-dimethylformamide (328 mL). After complete dissolution, **WX004-1** (25.28 g, 132.36 mmol, 1.05 *eq*) and 1,8-diazabicyclo[5.4.0]undec-7-ene (38.38 g, 252.11 mmol, 38.00 mL, 2 *eq*) were added, and the resulting mixture was reacted at 100 °C for 1.5 hours. Under stirring, the reaction liquid was added slowly to 3500 mL of water. At this moment, a solid was precipitated out. Suction filtration was performed using diatomaceous earth. The filter cake was washed with water, dissolved with dichloromethane, and then subjected to suction filtration. The filtrate was concentrated to obtain a crude. The crude was purified with chromatographic column (petroleum ether : ethyl acetate = 1 : 1) to obtain compound **WX004-2**. LCMS: m/z [M+1]⁺ = 424, 426.

### Step 2: Synthesis of compound WX004-3

To a reaction flask was added **WX004-2** (51 g, 120.21 mmol, 1 *eq*), followed by dichloromethane (500 mL) and trifluoroacetic acid (255.64 g, 2.24 mol, 166.00 mL, 18.65 *eq),* and the resulting mixture was reacted at 20 °C for 0.5 hours. To the reaction liquid was added 500 mL of water and then the mixture was adjusted to pH = 7 with sodium carbonate and extracted with dichloromethane (3 × 500 mL). The organic phases were combined, washed with saturated brine (500 mL), dried over anhydrous sodium sulfate, filtered and concentrated to obtain compound **WX004-3**. LCMS: m/z [M+1]⁺ = 324, 326.

### Step 3: Synthesis of compound WX004-4

Under nitrogen protection, to a reaction flask containing N,N-diisopropylethylamine (371.00 g, 2.87 mol, 500 mL, 46.52 *eq*) was added **WX004-3** (20 g, 61.70 mmol, 1 *eq*), followed by **A-2** (16.91 g, 67.87 mmol, 1.1 *eq*), and the resulting mixture was reacted at 100 °C for 16 hours. The mixture was directly concentrated to obtain a crude. The crude was purified by column chromatography (petroleum ether : ethyl acetate = 1 : 4) to obtain compound **WX004-4**. LCMS: m/z [M+1]⁺ = 536, 538.

### Step 4: Synthesis of compound WX004

To a reaction flask containing dichloromethane (238 mL) was added **WX004-4** (24 g, 44.71 mmol, 1 *eq*), followed by trifluoroacetic acid (244.86 g, 2.15 mol, 159 mL, 48.03 *eq*), and the resulting mixture was reacted at 20 °C for 0.5 hours. To the reaction liquid was added 500 mL of water and the mixture was adjusted to pH = 7 with sodium carbonate, extracted with a mixed solvent (dichloromethane : methanol = 20 : 1) (2 * 500 mL), and concentrated under reduced pressure to about 500 mL of the solvent. Suction filtration was performed and the filter cake was dried to obtain compound **WX004**. ¹H NMR (400 MHz, DMSO-*d*₆) δ ppm 3.01 (br s, 2 H), 3.73 - 3.82 (m, 2 H), 4.66 (s, 2 H), 7.37 (td, J=8.53, 2.51 Hz, 1 H), 7.43 - 7.53 (m, 1 H), 7.74 (br dd, J=8.03, 2.51 Hz, 1 H), 8.02 (s, 1 H), 8.53 (s, 1 H), 11.21 -13.86(m, 1 H); LCMS: m/z [M+1]⁺ = 452, 454.

### Example 5

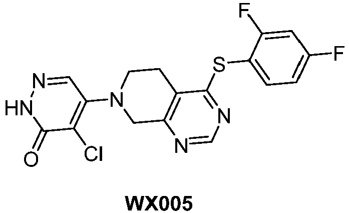

### Synthetic route:

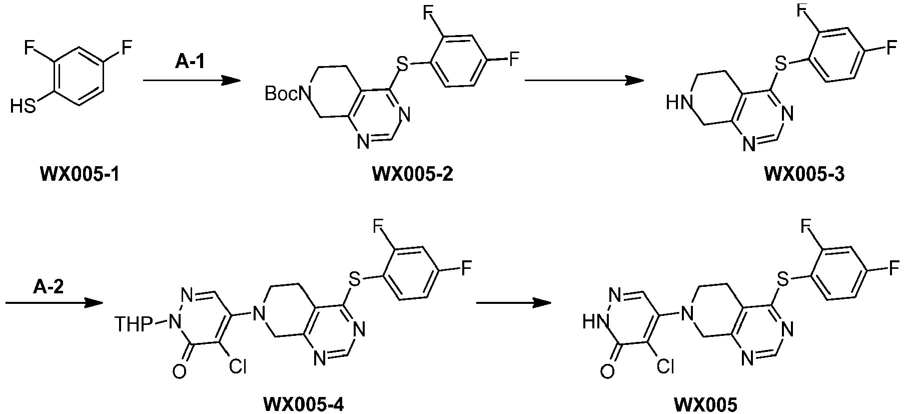

### Step 1: Synthesis of compound WX005-2

**A-1** (1.5 g, 5.56 mmol, 1 *eq*) and **WX005-1** (812.81 mg, 5.56 mmol, 630.08 µL, 1 *eq*) were added to N,N-dimethylformamide (15 mL). 1,8-diazabicyclo[5.4.0]undec-7-ene (1.69 g, 11.12 mmol, 1.68 mL, 2 *eq*) was added and the resulting mixture was reacted at 100 °C for 1.5 hours. To the reaction liquid was added 150 mL of ethyl acetate. The mixture was sequentially washed with half-saturated brine (6 × 50 mL) and saturated brine, dried over anhydrous sodium sulfate, filtered and concentrated to obtain a crude. The crude was separated and purified by column chromatography (petroleum ether : ethyl acetate = 1 : 0-3 : 1) to obtain compound **WX005-2**. LCMS: m/z [M+1]⁺ = 380.

### Step 2: Synthesis of compound WX005-3

To a reaction flask containing dichloromethane (50 mL) was added **WX005-2** (1 g, 2.64 mmol, 1 *eq*), followed by trifluoroacetic acid (11.59 g, 101.63mmol, 7.52 mL, 38.56 *eq*), and the resulting mixture was reacted at 20 °C for 0.5 hours. To the reaction liquid was added 50 mL of water and the mixture was adjusted to pH = 9 with sodium carbonate, and extracted with dichloromethane (3 × 50 mL). The organic phases were combined, washed with saturated brine, dried over anhydrous sodium sulfate, filtered and concentrated to obtain compound **WX005-3**. LCMS: m/z [M+1]⁺ = 280.

### Step 3: Synthesis of compound WX005-4

To a reaction flask containing N,N-diisopropylethylamine (18.35 g, 141.96 mmol, 24.73 mL, 69.56 *eq*) was added **WX005-3** (570 mg, 2.04 mmol, 1 *eq*), followed by **A-2** (508.34 mg, 2.04 mmol, 1 *eq*), and the resulting mixture was reacted at 100 °C for 16 hours. The reaction liquid was concentrated and 30 mL of water was added. The mixture was extracted with dichloromethane (3 × 30 mL). The organic phases were combined, washed with saturated brine, dried over anhydrous sodium sulfate, filtered and concentrated to obtain a crude. The crude was separated and purified by column chromatography (petroleum ether : ethyl acetate = 1 : 0-3 : 1) to obtain compound **WX005-4**. LCMS: m/z [M+1]⁺ = 492.

### Step 4: Synthesis of compound WX005

To a reaction flask containing dichloromethane (10 mL) was added **WX005-4** (0.43 g, 874.09 µmοl, 1 *eq*), followed by trifluoroacetic acid (2.99 g, 26.22 mmol, 1.94 mL, 30 *eq*), and the resulting mixture was reacted at 20 °C for 0.5 hours. To the reaction liquid was added 20 mL of water and the mixture was adjusted to pH = 9 with sodium carbonate, and extracted with dichloromethane (3 × 20 mL). After concentration, the residue was dissolved with 7 mL of dimethyl sulfoxide, and then 7 mL of water was added to precipitate out a solid. Suction filtration was performed to obtain a filter cake. To the filter cake were added 1 mL of methanol and 1 mL of ethyl acetate. The mixture was stirred and subjected to suction filtration. The filter cake was dried to obtain compound **WX005**. ¹H NMR (400 MHz, DMSO-*d*₆) δ ppm 2.91 (br t, *J*=5.40 Hz, 2 H), 3.81 (br t, *J*=5.65 Hz, 2 H), 4.63 (s, 2 H), 7.26 (td, *J*=8.34, 2.38 Hz, 1 H), 7.52 (td, *J*=9.22, 2.64 Hz, 1 H), 7.66 - 7.80 (m, 1 H), 8.00 (s, 1 H), 8.64 (s, 1 H), 12.98 (s, 1 H); LCMS: m/z [M+1]⁺ = 408.

### Example 6

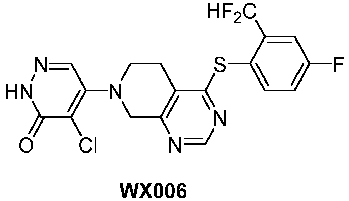

### Synthetic route:

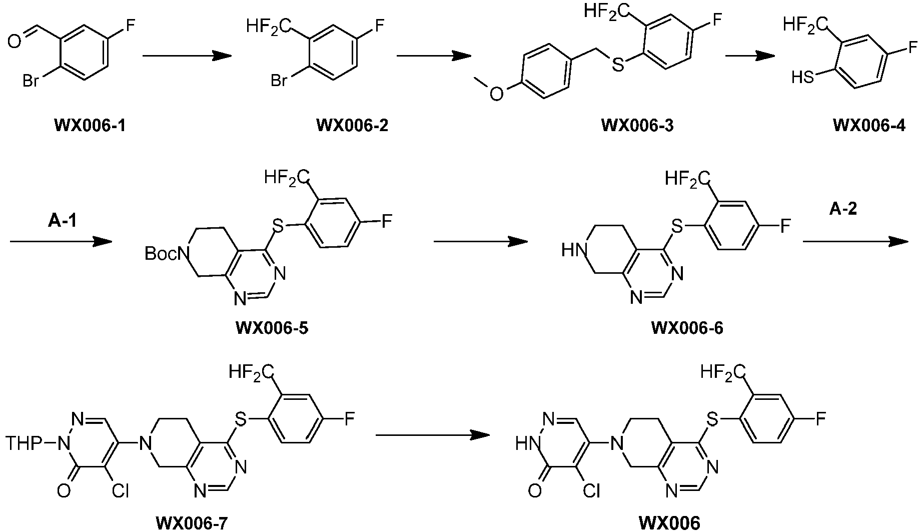

### Step 1: Synthesis of compound WX006-2

At 0 °C, a solution of diethylaminosulfur trifluoride (1.19 g, 7.39 mmol, 976.23 µL, 1.5 *eq*) in dichloromethane (5 mL) was added dropwise to a solution of **WX006-1** (1 g, 4.93 mmol, 1 *eq*) in dichloromethane (10 mL). The reaction liquid was warmed naturally to 25 °C and stirred for 18 hours. The reaction liquid was added dropwise to saturated sodium bicarbonate solution (40 mL). The mixture was stirred for 10 minutes and dichloromethane (20 mL) was added. The organic phase was washed with water (2 × 20 mL) and saturated brine, dried over anhydrous sodium sulfate and filtered and the filtrate was concentrated to obtain compound **WX006-2**. ¹H NMR (400 MHz, CDCl₃) δ ppm 6.72 - 7.04 (m, 1 H), 7.11 (br dd, *J*=2.01, 1.00 Hz, 1 H), 7.41 (dd, *J*=8.66, 2.89 Hz, 1 H), 7.60 (dd, *J*=8.78, 5.02 Hz, 1 H).

### Step 2: Synthesis of compound WX006-3

To **WX006-2** (2 g, 8.89 mmol, 1 *eq*) and a solution of N,N-diisopropylethylamine (3.45 g, 26.67 mmol, 4.64 mL, 3 *eq*) in 1,4-dioxane (20 mL) was added 4-methoxybenzyl mercaptan (1.51 g, 9.78 mmol, 1.36 mL, 1.1 *eq*)*.* After nitrogen replacement, tris(dibenzylideneacetone)dipalladium (244.19 mg, 266.66 µmοl, 0.03 *eq*) and 4,5-bisdiphenylphosphino-9,9-dimethylxanthene (360.02 mg, 622.21 µmοl, 0.07 *eq*) were added. After additional nitrogen replacement, the mixture was reacted at 90 °C for 2 hours. The reaction liquid was concentrated, then dissolved in ethyl acetate (20 mL), washed with water (3 × 10 mL) and saturated brine, dried over anhydrous sodium sulfate and filtered and the filtrate was concentrated to obtain a crude. The crude was purified by column chromatography (petroleum ether : ethyl acetate = 1 : 0-19 : 1) to obtain compound **WX006-3**. ¹H NMR (400 MHz, DMSO-*d*₆) δ ppm 3.71 (s, 3 H), 4.14 (s, 2 H), 6.82 - 6.88 (m, 3 H), 7.14 - 7.19 (m, 2 H), 7.25 (d, *J*=8.53 Hz, 1 H), 7.41 (br d, *J*=9.29 Hz, 1 H), 7.63 (br dd, *J*=8.16, 5.40 Hz, 1 H).

### Step 3: Synthesis of compound WX006-4

**WX006-3** (2.5 g, 8.38 mmol, 1 *eq*) was dissolved in trifluoroacetic acid (10 mL) and anisole (5 mL) and the mixture was stirred at 80 °C for 1 hour. The reaction liquid was cooled to 25 °C and ice water was added. The mixture was extracted with ethyl acetate (50 mL) and the organic phase was extracted with 5 M aqueous sodium hydroxide solution (3 × 30 mL). The aqueous phases were combined and adjusted to pH 2 with 2 M hydrochloric acid and extracted with dichloromethane (2 × 50 mL). The organic phases were combined, washed with water and saturated brine, dried over anhydrous sodium sulfate and filtered. The filtrate was concentrated to obtain compound **WX006-4**. ¹H NMR (400 MHz, DMSO-*d*₆) δ ppm 3.75 (s, 1 H), 6.79 - 6.98 (m, 1 H), 7.26 - 7.37 (m, 1 H), 7.39 - 7.45 (m, 1 H), 7.55 - 7.67 (m, 1 H).

### Step 4: Synthesis of compound WX006-5

**WX006-4** (450.84 mg, 2.53 mmol, 1.05 *eq*), 1,8-diazabicyclo[5.4.0]undec-7-ene (733.74 mg, 4.82 mmol, 726.47 µL, 2 *eq*) and **A-1** (650 mg, 2.41 mmol, 1 *eq*) were added to N,N-dimethylformamide (6.5 mL) and the resulting mixture was stirred at 100 °C for 0.5 hours. The reaction liquid was cooled to 25 °C and 10 mL of ethyl acetate was added. The organic phase was washed with water (3 × 5 mL) and saturated brine, dried over anhydrous sodium sulfate, filtered and concentrated to obtain a crude. The crude was purified by column chromatography (petroleum ether : ethyl acetate = 1 : 0-5 : 1) to obtain compound **WX006-5**. LCMS: m/z [M+1]⁺ = 412.

### Step 5: Synthesis of compound WX006-6

**WX006-5** (550 mg, 1.34 mmol, 1 *eq*) was added to a reaction flask containing trifluoroacetic acid (2 mL) and dichloromethane (5 mL) and the resulting mixture was reacted at 25 °C for 1 hour. The mixture was adjusted to pH = 8 with saturated sodium carbonate solution and dichloromethane (5 mL) was added. The mixture was washed with water (3 × 3 mL) and saturated brine, dried over anhydrous sodium sulfate and filtered. The filtrate was concentrated to obtain compound **WX006-6**. LCMS: m/z [M+1]⁺ = 312.

### Step 6: Synthesis of compound WX006-7

**WX006-6** (330 mg, 739.02 µmοl, purity: 69.72%, 1 *eq*) and **A-2** (202.49 mg, 812.92 µmοl, 1.1 *eq*) were added to a reaction flask containing N,N-diisopropylethylamine (6 mL) and the resulting mixture was reacted at 100 °C for 16 hours. The reaction liquid was concentrated to obtain a crude. The crude was purified by column chromatography (petroleum ether : ethyl acetate = 1 : 1-1 : 3) to obtain compound **WX006-7**. LCMS: m/z [M+1]⁺ = 524.

### Step 7: Synthesis of compound WX006

To a reaction flask containing dichloromethane (3.5 mL) was added **WX006-7** (357 mg, 681.35 µmοl, 1 *eq*), followed by trifluoroacetic acid (2 mL), and the resulting mixture was stirred at 25 °C for 0.5 hours. The reaction system was cooled to 0 °C and the reaction liquid was adjusted to pH = 8 by dropwise adding saturated sodium carbonate solution. Suction filtration was performed and the filter cake was washed with water and dichloromethane and dried to obtain compound **WX006**. ¹H NMR (400 MHz, DMSO-*d*₆) δ ppm 2.92 (br t, *J*=5.40 Hz, 2 H), 3.77 (t, *J*=5.65 Hz, 1 H), 3.83 - 3.88 (m, 1 H), 4.59 (s, 2 H), 7.12 (s, 1 H), 7.53 - 7.62 (m, 1 H), 7.64 - 7.71 (m, 1 H), 7.73 - 7.84 (m, 1 H), 7.99 (s, 1 H), 8.61 (s, 1 H), 12.95 (br s, 1 H); LCMS: m/z [M+1]⁺ = 440.

### Example 7

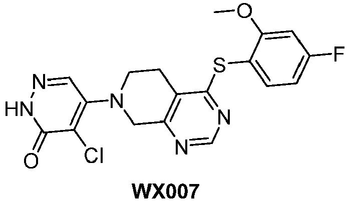

### Synthetic route:

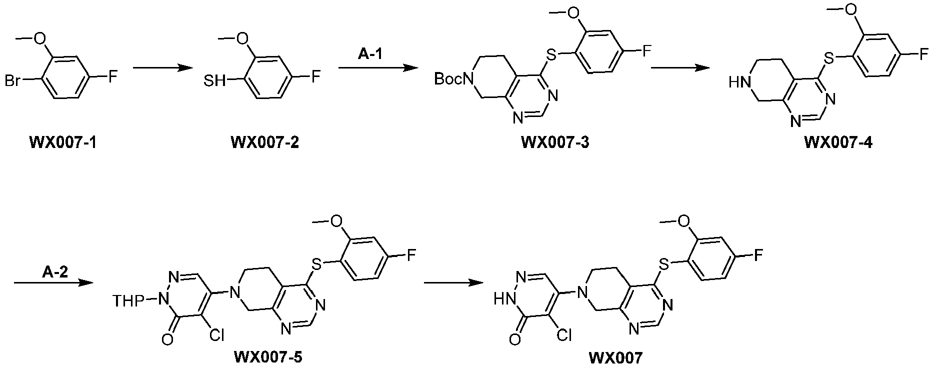

### Step 1: Synthesis of compound WX007-2

Under nitrogen protection, **WX007-1** (2 g, 9.75 mmol, 1 *eq*) and powdered sulfur (469.19 mg, 14.63 mmol, 1.5 *eq*) were added to a reaction flask containing tetrahydrofuran (50 mL). The reaction flask was placed in a dry ice-acetone bath and the mixture was stirred for 10 minutes; then tert-butyl lithium (1.3 M, 16.51 mL, 2.2 *eq*) was added slowly dropwise and the mixture was reacted in a dry ice-acetone bath for 1 hour. Upon completion of the reaction, the reaction liquid was added dropwise to 80 mL of saturated ammonium chloride solution. The mixture was extracted with ethyl acetate (2 × 80 mL). The organic phases were combined, washed with 2 M hydrochloric acid (40 mL), dried over anhydrous sodium sulfate and filtered. The filtrate was concentrated to obtain a crude. The crude was purified by column chromatography (petroleum ether : ethyl acetate = 1 : 0-10 : 1) to obtain compound **WX007-2**. ¹H NMR (400 MHz, DMSO-*d*₆) δ ppm 3.83 (s, 3 H), 4.83 (s, 1 H), 6.71 (td, *J*=8.41, 2.76 Hz, 1 H), 6.90 - 6.97 (m, 1 H), 7.32 (dd, *J*=8.53, 6.53 Hz, 1 H).

### Step 2: Synthesis of compound WX007-3

**A-1** (2.65 g, 9.82 mmol, 1 *eq*) was dissolved in N,N-dimethylformamide (20 mL). 1,8-diazabicyclo[5.4.0]undec-7-ene (2.99 g, 19.65 mmol, 2.96 mL, 2 *eq*) was added dropwise and then **WX007-2** (1.55 g, 9.82 mmol, 1 *eq*) was added. The resulting mixture was reacted at 100 °C for 1.5 hours. Ethyl acetate (200 mL) was added and the organic phase was washed with half-saturated brine (6 × 50 mL) and 1M hydrochloric acid, dried over anhydrous sodium sulfate and filtered. The filtrate was concentrated to obtain a crude. The crude was separated and purified by column chromatography (petroleum ether : ethyl acetate = 1 : 0-4 : 1) to obtain compound **WX007-3**.+ LCMS: m/z [M+1]⁺ = 392.

### Step 3: Synthesis of compound WX007-4

**WX007-3** (1.55 g, 3.96 mmol, 1 *eq*) was dissolved in a reaction flask containing dichloromethane (20 mL). Trifluoroacetic acid (17.41 g, 152.68 mmol, 11.30 mL, 38.56 *eq*) was added dropwise and the resulting mixture was reacted at 20 °C for 0.5 hours. Under an ice-bath, to the reaction liquid was added dropwise saturated sodium carbonate solution to adjust the mixture to pH = 8. The mixture was extracted with dichloromethane (3 × 50 mL). The organic phases were combined, washed with saturated brine, dried over anhydrous sodium sulfate and filtered. The filtrate was concentrated to obtain compound **WX007-4**. LCMS: m/z [M+1]⁺ = 292.

### Step 4: Synthesis of compound WX007-5

To a reaction flask containing N,N-diisopropylethylamine (46 mL) was added **WX007-4** (1.1 g, 3.78 mmol, 1 *eq*), followed by **A-2** (1.13 g, 4.53 mmol, 1.2 *eq*) and the resulting mixture was reacted at 100 °C for 16 hours. The reaction liquid was concentrated and 50 mL of water was added. The mixture was extracted with dichloromethane (3 × 50 mL). The organic phases were combined, washed with saturated brine, dried over anhydrous sodium sulfate and filtered. The filtrate was concentrated to obtain a crude. The crude was purified by column chromatography (petroleum ether : ethyl acetate = 1 : 2) to obtain compound **WX007-5**. LCMS: m/z [M+1]⁺ = 504.

### Step 5: Synthesis of compound WX007

To a reaction flask containing dichloromethane (30 mL) was added **WX007-5** (1 g, 1.98 mmol, 1 *eq*)*.* Trifluoroacetic acid (6.79 g, 59.53 mmol, 4.41 mL, 30 *eq*) was added dropwise and the resulting mixture was reacted at 20 °C for 0.5 hours. Under an ice-bath, the mixture was adjusted to pH = 9 with saturated brine, and extracted with dichloromethane (3 × 40 mL). The organic phases were combined, washed with saturated brine, dried over anhydrous sodium sulfate and filtered. The filtrate was concentrated to obtain a crude. The crude was separated and purified by preparative high-performance liquid chromatography (chromatographic column: Phenomenex C18 80 * 40 mm * 3 µm; mobile phase: [water (NH₃H₂O)-ACN]; gradient (ACN%): 31%-61%) to obtain compound **WX007**. ¹H NMR (400 MHz, DMSO-*d*₆) δ ppm 2.88 (br t, *J*=5.14 Hz, 2 H), 3.76 (s, 3 H), 3.79 (br t, *J*=5.65 Hz, 2 H), 4.60 (s, 2 H), 6.90 (td, *J*=8.41, 2.26 Hz, 1 H), 7.11 (dd, *J*=11.17, 2.38 Hz, 1 H), 7.47 - 7.65 (m, 1 H), 8.00 (s, 1 H), 8.58 (s, 1 H), 12.96 (br s, 1 H); LCMS: m/z [M+1]⁺ = 420.

### Example 8

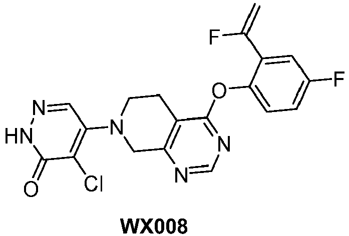

### Synthetic route:

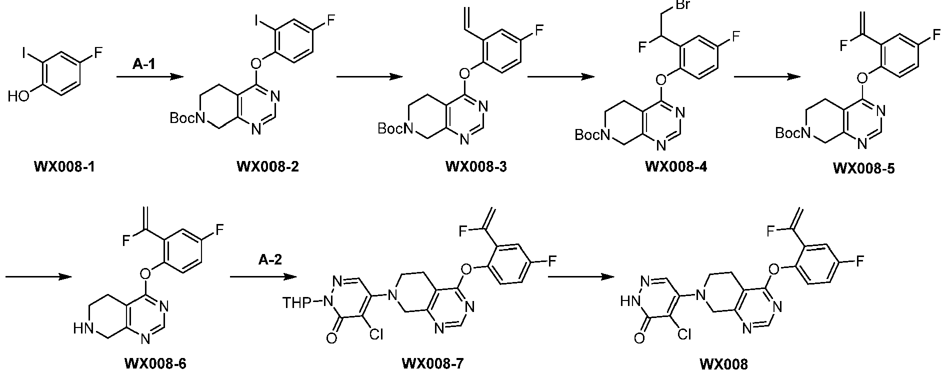

### Step 1: Synthesis of compound WX008-2

**A-1** (5 g, 18.54 mmol, 1 *eq*) was dissolved in N,N-dimethylformamide (500 mL). 1,8-diazabicyclo[5.4.0]undec-7-ene (5.64 g, 37.07 mmol, 5.59 mL, 2 *eq*) was added, followed by **WX008-1** (5.29 g, 22.24 mmol, 1.2 *eq*) and the resulting mixture was reacted at 100 °C for 1.5 hours. To the reaction liquid was added 1000 mL of ethyl acetate. The mixture was washed with half-saturated brine (6 × 100 mL) and saturated brine, dried over anhydrous sodium sulfate, filtered and concentrated to obtain a crude. The crude was purified by column chromatography (petroleum ether : ethyl acetate = 4 : 1) to obtain compound WX008-2. LCMS: m/z [M+1]⁺= 472.

### Step 2: Synthesis of compound WX008-3

Under nitrogen protection, **WX008-2** (5 g, 10.61 mmol, 1 *eq*) was dissolved in a mixed solution of N,N-dimethylformamide (50 mL) and H₂O (10 mL). Vinylboronic acid pinacol ester (5.72 g, 37.13 mmol, 6.30 mL, 3.5 *eq*), tetra(triphenylphosphine)palladium (1.23 g, 1.06 mmol, 0.1 *eq*) and sodium carbonate (6.75 g, 63.66 mmol, 6 *eq*) were added and the resulting mixture was reacted at 80 °C for 1 hour. To the filtrate was added 1000 mL of ethyl acetate. The mixture was washed with half-saturated brine (6 × 100 mL) and saturated brine, dried over anhydrous sodium sulfate, filtered and concentrated to obtain a crude. The crude was purified by column chromatography (petroleum ether : ethyl acetate = 6 : 1) to obtain compound **WX008-3**. LCMS: m/z [M+1]⁺ = 372.

### Step 3: Synthesis of compound WX008-4

At 0 °C, **WX008-3** (3.4 g, 9.15 mmol, 1 *eq*) was dissolved in dichloromethane (60 mL). To the mixture was added dropwise N-bromosuccinimide (1.79 g, 10.07 mmol, 1.1 *eq*) and then triethylamine trihydrofluoride (2.95 g, 18.31 mmol, 2.98 mL, 2 *eq*) was added. The resulting mixture was warmed slowly to 20 °C and reacted for 16 hours. The reaction liquid was poured into a mixed solution of ice water (50 mL) and ammonia water (10 mL). The mixture was extracted with dichloromethane (3 × 60 mL). The organic phases were combined, washed with 1 M hydrochloric acid, dried over anhydrous sodium sulfate, filtered and concentrated to obtain a crude. The crude was purified by column chromatography (petroleum ether : ethyl acetate = 1 : 0-5 : 1) to obtain compound **WX008-4**. LCMS: m/z [M+1]⁺ = 470.

### Step 4: Synthesis of compound WX008-5

**WX008-4** (2.54 g, 5.40 mmol, 1 *eq*) was dissolved in a reaction flask containing dichloromethane (30 mL). 1,8-diazabicyclo[5.4.0]undec-7-ene (1.15 g, 7.56 mmol, 1.14 mL, 1.4 *eq*) was added dropwise and the resulting mixture was reacted at 60 °C for 16 hours. The reaction liquid was directly concentrated to obtain a crude. The crude was purified by column chromatography (petroleum ether : ethyl acetate = 1 : 0-3 : 1) to obtain compound **WX008-5**. LCMS: m/z [M+1]⁺ = 390.

### Step 5: Synthesis of compound WX008-6

**WX008-5** (1.71 g, 4.39 mmol, 1 *eq*) was dissolved in a reaction flask containing dichloromethane (20 mL). Trifluoroacetic acid (19.31 g, 169.33 mmol, 12.54 mL, 38.56 *eq*) was added dropwise and the resulting mixture was reacted at 20 °C for 0.5 hours. Under an ice-bath, to the reaction liquid was added dropwise saturated sodium carbonate solution to adjust the mixture to pH = 8. The mixture was extracted with dichloromethane (3 × 20 mL). The organic phases were combined, washed with saturated brine, dried over anhydrous sodium sulfate, filtered and concentrated to obtain compound **WX008-6**. LCMS: m/z [M+1]⁺ = 290.

### Step 6: Synthesis of compound WX008-7

To a reaction flask containing N,N-diisopropylethylamine (48 mL) was added **WX008-6** (1.14 g, 3.94 mmol, 1 *eq*), followed by **A-2** (1.18 g, 4.73 mmol, 1.2 *eq*) and the resulting mixture was reacted at 100 °C for 16 hours. The reaction liquid was concentrated and 50 mL of water was added. The mixture was extracted with dichloromethane (50 mL × 3). The organic phases were combined, washed with saturated brine, dried over anhydrous sodium sulfate, filtered and concentrated to obtain a crude. The crude was purified by column chromatography (petroleum ether : ethyl acetate = 1 : 2) to obtain compound **WX008-7**. LCMS: m/z [M+1]⁺= 502.

### Step 7: Synthesis of compound WX008

To a reaction flask containing dichloromethane (15 mL) was added **WX008-7** (1 g, 1.99 mmol, 1 *eq*)*.* Trifluoroacetic acid (6.82 g, 59.77 mmol, 4.43 mL, 30 *eq*) was added dropwise and the resulting mixture was reacted at 20 °C for 0.5 hours. Under an ice-bath, the mixture was adjusted to pH = 9 with saturated sodium carbonate solution and extracted with dichloromethane (3 × 15 mL). The organic phases were combined, washed with saturated brine, dried over anhydrous sodium sulfate, filtered and concentrated to obtain a crude. The crude was purified by preparative high-performance liquid chromatography (chromatographic column: Phenomenex C18 80 * 40 mm * 3 µm; mobile phase: [water (NH₃H₂O)-ACN]; gradient (ACN%): 33%-63%) to obtain compound **WX008**. ¹H NMR (400 MHz, DMSO-*d*₆) δ ppm 3.00 (br t, *J*=5.27 Hz, 2 H), 3.79 (br t, *J*=5.52 Hz, 2 H), 4.66 (s, 2 H), 5.06 - 5.11 (m, 1 H), 5.11 - 5.24 (m, 1 H), 7.39 - 7.43 (m, 2 H), 7.50 (dd, *J*=9.16, 2.38 Hz, 1 H), 8.03 (s, 1 H), 8.53 (s, 1 H), 12.56 - 13.07 (m, 1 H); LCMS: m/z [M+1]⁺ = 418.

### Example 9

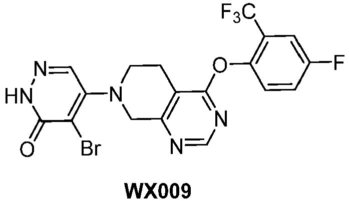

### Synthetic route:

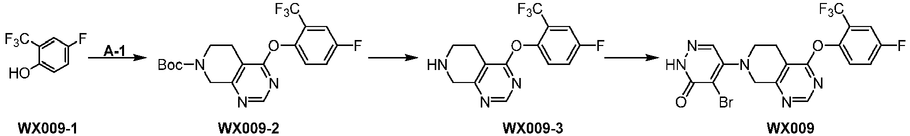

### Step 1: Synthesis of compound WX009-2

To a reaction flask containing N,N-dimethylformamide (30 mL), **A-1** (3 g, 11.12 mmol, 1 *eq*) was added, followed by 1,8-diazabicyclo[5.4.0]undec-7-ene (3.39 g, 22.24 mmol, 3.35 mL, 2 *eq*) and **WX009-1** (2.20 g, 12.23 mmol, 1.1 *eq*)*.* The resulting mixture was reacted at 100 °C for 0.5 hours. Then to the reaction liquid was added 800 mL of ethyl acetate. The organic phase was washed with half-saturated brine (5 × 250 mL) and saturated brine, dried over anhydrous sodium sulfate, filtered and concentrated to obtain compound **WX009-2**. LCMS: m/z [M+1]⁺ = 414.

### Step 2: Synthesis of compound WX009-3

To a reaction flask was added **WX009-2** (2 g, 4.84 mmol, 1 *eq*), followed by dichloromethane (20 mL) and trifluoroacetic acid (10.29 g, 90.23 mmol, 6.68 mL, 18.65 *eq*), and the resulting mixture was reacted at 20 °C for 0.5 hours. To the reaction liquid was added 20 mL of water and then the mixture was adjusted to pH = 7 with sodium carbonate and extracted with dichloromethane (2 × 20 mL). The organic phases were combined, washed with saturated brine, dried over anhydrous sodium sulfate, filtered and concentrated to obtain compound **WX009-3**. LCMS: m/z [M+1]⁺ = 314.

### Step 3: Synthesis of compound WX009

Under nitrogen protection, to a reaction flask containing N,N-diisopropylethylamine (19.19 g, 148.51 mmol, 25.87 mL, 46.52 *eq*) was added **WX009-3** (1 g, 3.19 mmol, 1 *eq*), followed by 4,5-dibromopyridazin-3-one (891.52 mg, 3.51 mmol, 1.1 *eq*), and the resulting mixture was reacted at 100 °C for 24 hours. The reaction liquid was directly concentrated and then 20 mL of a mixed solvent (dichloromethane : methanol = 20 : 1) was added. The organic phase was sequentially washed with water and saturated brine, dried over anhydrous sodium sulfate, filtered and concentrated to obtain a crude. The crude was separated by preparative high-performance liquid chromatography (chromatographic column: Xtimate C18 150 * 40 mm * 10 µm; mobile phase: [water (FA)-ACN]; ACN%: 35%-65%). DCM (2 mL) was added and the mixture was stirred at 20 °C for 16 hours. Then suction filtration was performed and the filter cake was washed with 1 mL of DCM and then dried to obtain compound **WX009**. ¹H NMR (400 MHz, CDCl₃) δ ppm 3.12 (t, J=5.77 Hz, 2 H), 3.88 (t, J=5.77 Hz, 2 H), 4.66 (s, 2 H), 7.32 - 7.38 (m, 2 H), 7.45 (br d, J=7.53 Hz, 1 H), 7.77 (s, 1 H), 8.55 (s, 1 H); LCMS: m/z [M+1]⁺ = 486.

### Example 10

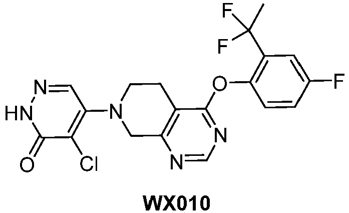

### Synthetic route:

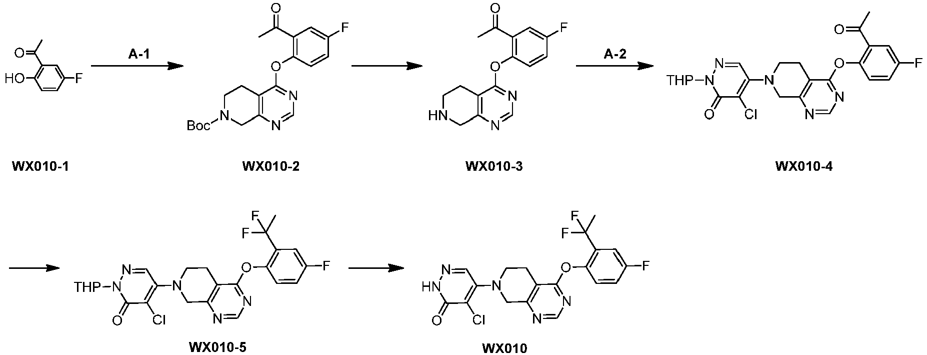

### Step 1: Synthesis of compound WX010-2

**A-1** (5 g, 18.54 mmol, 1 *eq*) and **WX010-1** (4.29 g, 27.81 mmol, 1.5 *eq*) were dissolved in N,N-dimethylformamide (40 mL). Then potassium carbonate (7.69 g, 55.61 mmol, 3 *eq*) was added. After the reaction was subjected to nitrogen replacement 3 times, the mixture was stirred at 60 °C for 20 hours. Ethyl acetate (100 mL) was added, and the organic phase was washed with water (100 mL). The aqueous phase was extracted with dichloromethane (3 × 50 mL). The organic phases were combined, washed with saturated brine (50 mL), dried over anhydrous sodium sulfate, filtered and concentrated to obtain a crude. The crude was purified by column chromatography (petroleum ether : ethyl acetate = 1 : 0-15 : 1) to obtain compound **WX010-2**. ¹H NMR (400 MHz, CDCl₃) δ ppm 1.52 (s, 9 H), 2.47 (s, 3 H), 2.92 (br t, J=5.4 Hz, 2 H), 3.79 (t, J=5.8 Hz, 2 H), 4.65 (s, 2 H), 7.14 (dd, J=8.8, 4.5 Hz, 1 H), 7.28 - 7.35 (m, 1 H), 7.56 (dd, J=8.7, 3.1 Hz, 1 H), 8.49 (s, 1 H); LCMS: m/z [M+1]⁺ = 388.

### Step 2: Synthesis of compound WX010-3

**WX010-2** (2.3 g, 5.94 mmol, 1 *eq*) was dissolved in dichloromethane (100 mL). Then trifluoroacetic acid (25.72 g, 225.60 mmol, 16.70 mL, 38 *eq*) was added and the resulting reaction mixture was stirred at 20 °C for 1 hour. To the reaction liquid was added 100 mL of water and the mixture was adjusted to pH = 9 with sodium carbonate. The aqueous phase was extracted with dichloromethane (3 × 50 mL). The organic phases were combined, washed with saturated brine (100 mL), dried over anhydrous sodium sulfate, filtered and concentrated to obtain compound **WX010-3.** LCMS: m/z [M+1]⁺ = 288.

### Step 3: Synthesis of compound WX010-4

**WX010-3** (2.25 g, 7.83 mmol, 1 *eq*) was dissolved in N,N-diisopropylethylamine (70.85 g, 548.23 mmol, 95.49 mL, 70 *eq*) and **A-1** (2.34 g, 9.40 mmol, 1.2 *eq*) was added. Then the reaction mixture was stirred at 100 °C for 16 hours. The mixture was distilled under reduced pressure to obtain a crude. The crude was purified by column chromatography (petroleum ether : ethyl acetate = 1 : 0-5 : 1) to obtain compound **WX010-4.** LCMS: m/z [M+1]⁺ = 500.

### Step 4: Synthesis of compound WX010-5

**WX010-4** (0.5 g, 1.00 mmol, 1 *eq*) was dissolved in dichloromethane (20 mL) and then bis(2-methoxyethyl)aminosulfur trifluoride (2.21 g, 10.00 mmol, 2.19 mL, 10 *eq*) was added slowly. The reaction mixture was stirred at 40 °C for 20 hours. The reaction liquid was added slowly to 50 mL of saturated sodium bicarbonate solution. The mixture was stirred for 5 minutes. The aqueous phase was extracted with dichloromethane (2 × 30 mL). The organic phases were combined and dried over anhydrous sodium sulfate. The filtrate was concentrated to obtain a crude. The crude was separated by preparative high-performance liquid chromatography (chromatographic column: Phenomenex C18 150 * 40 mm * 5 µm; mobile phase: [water (HCl)-ACN]; gradient (ACN%): 50%-60%) to obtain compound **WX010-5.** ¹H NMR (400 MHz, CDCl₃) δ ppm 1.55 - 1.62 (m, 1 H), 1.70 - 1.82 (m, 3 H), 2.00 - 2.07 (m, 1 H), 2.09 - 2.20 (m, 1 H), 2.49 (s, 3 H), 3.11 (br t, J=5.5 Hz, 2 H), 3.70 - 3.94 (m, 3 H), 4.09 - 4.20 (m, 1 H), 4.62 (s, 2 H), 6.08 (dd, J=10.7, 1.6 Hz, 1 H), 7.17 (dd, J=9.0, 4.5 Hz, 1 H), 7.28 - 7.36 (m, 1 H), 7.57 (dd, J=8.5, 3.0 Hz, 1 H), 7.81 (s, 1 H), 8.50 (s, 1 H); LCMS: m/z [M+23]⁺ = 544.

### Step 5: Synthesis of compound WX010

**WX010-5** (0.088 g, 168.61 µmοl, 1 *eq*) was dissolved in ethyl acetate (1 mL) and then hydrogen chloride-4 M ethyl acetate solution (2 mL) was added. The reaction mixture was stirred at 20 °C for 1 hour. The reaction liquid was concentrated to obtain a crude. The crude was dissolved in 50 mL of water and the mixture was adjusted to pH = 9 by slowly adding saturated sodium carbonate solution. The mixture was filtered and dried. Then to the mixture was added 2 mL of dichloromethane. The resulting mixture was stirred for 0.5 hours and then filtered. The filter cake was washed with 0.5 mL of dichloromethane and dried to obtain compound **WX010**. ¹H NMR (400 MHz, DMSO-*d*₆) δ ppm 1.97 (br t, J=19.1 Hz, 3 H), 3.00 (br s, 2 H), 3.79 (br s, 2 H), 4.66 (br s, 2 H), 7.47 (br s, 3 H), 8.03 (s, 1 H), 8.54 (s, 1 H), 13.00 (br s, 1 H); LCMS: m/z [M+1]⁺ = 438.

### Example 11

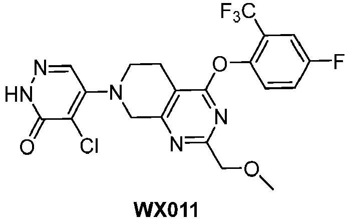

### Synthetic route:

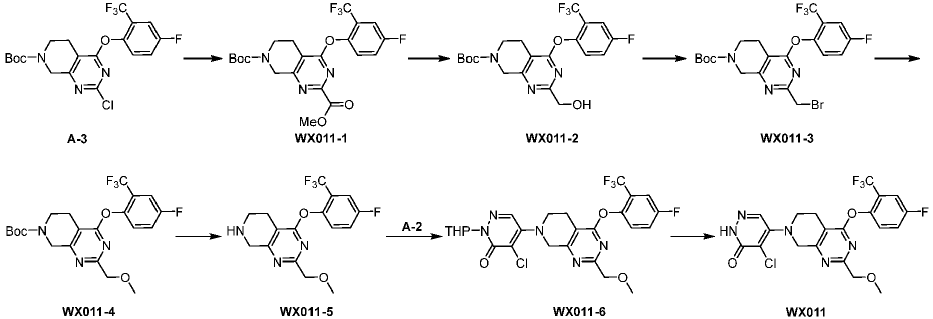

### Step 1: Synthesis of compound WX011-1

**A-3** (10 g, 22.33 mmol, 1 *eq*), [1,1'-bis(diphenylphosphino)ferrocene]palladium dichloride (326.79 mg, 446.62 µmοl, 0.02 *eq*) and triethylamine (44.66 mmol, 6.22 mL, 2 *eq*) were added to an autoclave containing methanol (400 mL). Under nitric oxide (150 Psi) atmosphere, the reaction mixture was reacted at 100 °C for 18 hours. The reaction liquid was concentrated to obtain a crude. The crude was purified by column chromatography (dichloromethane : methanol = 1 : 0-9 : 1) to obtain compound **WX011-1.** LCMS: m/z [M+1]⁺ = 472.

### Step 2: Synthesis of compound WX011-2

**WX011-1** (0.20 g, 424.27 µmοl, 1 *eq*) and tert-butanol (5 mL) were added to a reaction flask and at room temperature, sodium borohydride (50 mg, 1.32 mmol, 3.12 *eq*) was added. The mixture was warmed slowly to 70 °C and stirred at 70 °C for 3 hours. To the reaction liquid was added dropwise saturated ammonium chloride solution (10 mL) to quench the reaction. The mixture was extracted with ethyl acetate (3 × 10 mL). The organic phases were combined, washed with saturated brine, dried over anhydrous sodium sulfate, filtered and concentrated to obtain a crude. The crude was separated and purified by preparative thin layer chromatography (petroleum ether : ethyl acetate = 3 : 1) to obtain compound **WX011-2**. LCMS: m/z [M+1]⁺ = 444.

### Step 3: Synthesis of compound WX011-3

**WX011-2** (60 mg, 135.32 µmοl, 1 *eq*) and anhydrous tetrahydrofuran (1 mL) were added to a reaction flask. At 0 °C, phosphorus tribromide (270.64 µmοl, 25.44 µL, 2 *eq*) was added and the reaction system was stirred at 0 °C for 1 hour. To the reaction liquid was added saturated sodium carbonate solution (5 mL) to quench the reaction. The mixture was extracted with ethyl acetate (3 × 5 mL). The organic phases were combined, washed with saturated brine, dried over anhydrous sodium sulfate, filtered and concentrated to obtain a crude. The crude was separated and purified by preparative thin layer chromatography (petroleum ether : ethyl acetate = 3 : 1) to obtain compound **WX011-3**. ¹H NMR (400 MHz, CDCl₃) δ ppm 1.51 (s, 9 H), 2.89 (br s, 2 H), 3.77 (br t, J=5.77 Hz, 2 H), 4.32 (s, 2 H), 4.64 (s, 2 H), 7.30 - 7.40 (m, 2 H), 7.43 (dd, J=8.16, 2.64 Hz, 1 H); LCMS: m/z [M+1]⁺ = 506.

### Step 4: Synthesis of compound WX011-4

**WX011-3** (40 mg, 79.01 µmοl, 1 *eq*) and anhydrous methanol (0.5 mL) were added to a reaction flask and sodium methoxide (42.68 mg, 790.06 µmοl, 10 *eq*) was added. The resulting mixture was stirred at room temperature for 1 hour. The reaction liquid was adjusted to about pH 7 by dropwise adding citric acid solution (1 M). The mixture was concentrated to removed methanol. Water (5 mL) was added and the mixture was extracted with ethyl acetate (3 × 5 mL). The organic phases were combined, washed with saturated brine, dried over anhydrous sodium sulfate, filtered and concentrated to obtain compound **WX011-4**. LCMS: m/z [M+1]⁺ = 458.

### Step 5: Synthesis of compound WX011-5

To a reaction flask were added **WX011-4** (60 mg, 131.17 µmοl, 1 *eq*) and anhydrous dichloromethane (1 mL), followed by trifluoroacetic acid (4.05 mmol, 0.3 mL, 30.89 *eq*)*.* The resulting mixture was stirred at room temperature for 1 hour. The reaction liquid was adjusted to about pH 7 by dropwise adding saturated sodium bicarbonate solution. The mixture was subjected to liquid separation and the aqueous phase was extracted with dichloromethane (2 × 5 mL). The organic phases were combined, washed with saturated brine, dried over anhydrous sodium sulfate, filtered and concentrated to obtain compound **WX011-5**. LCMS: m/z [M+1]⁺ = 358.

### Step 6: Synthesis of compound WX011-6

**WX011-5** (60 mg, 167.93 µmοl, 1 *eq*), **A-2** (50 mg, 200.73 µmοl, 1.20 *eq*) and N,N-diisopropylethylamine (5.74 mmol, 1 mL, 34.19 *eq*) were added to a reaction flask and the mixture was stirred at 90 °C for 16 hours. To the reaction liquid was added water (5 mL). The mixture was extracted with ethyl acetate (3 × 5 mL). The organic phases were combined, washed with saturated brine, dried over anhydrous sodium sulfate, filtered and concentrated to obtain a crude. The crude was separated and purified by preparative thin layer chromatography (petroleum ether : ethyl acetate = 1 : 1) to obtain compound **WX011-6**. LCMS: m/z [M+1]⁺= 570.

### Step 7: Synthesis of compound WX011

To a reaction flask were added **WX011-6** (30 mg, 52.64 µmοl, 1 *eq*) and anhydrous dichloromethane (0.5 mL), followed by trifluoroacetic acid (2.70 mmol, 0.20 mL, 51.32 *eq*) and the resulting mixture was stirred at room temperature for 1 hour. The reaction liquid was concentrated to obtain a crude. The crude was separated and purified by preparative high-performance liquid chromatography (chromatographic column: Welch Xtimate C18 150 * 25 mm * 5 µm; mobile phase: [water (NH₄HCO₃)-ACN]; gradient (ACN%): 23%-53%) to obtain compound **WX011**. ¹H NMR (400 MHz, CDCl₃) δ ppm 3.09 (s, 2H), 3.45 (s, 3 H), 3.87 (t, J=5.65 Hz, 2 H), 4.43 (s, 2 H), 4.69 (s, 2 H), 7.32 - 7.39 (m, 2 H), 7.42 - 7.46 (m, 1 H), 7.73 (s, 1 H), 10.33 (br s, 1 H); LCMS: m/z [M+1]⁺ = 486.

### Example 12

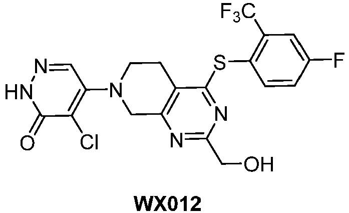

### Synthetic route:

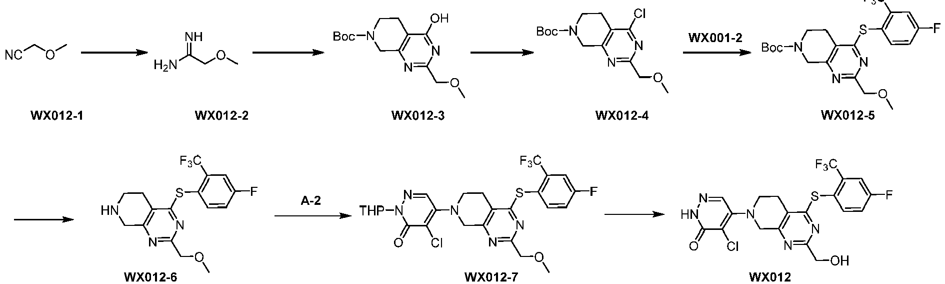

### Step 1: Synthesis of compound WX012-2

**WX012-1** (478.35 mmol, 35.56 mL, 1 *eq*)*,* MeOH (170 mL) and sodium methoxide (3.40 g, 62.94 mmol, 0.13 *eq*) were added to a reaction flask and the reaction system was stirred at room temperature (20 °C) for 1 hour. Then ammonium chloride (25.84 g, 483.07 mmol, 1.01 *eq)* was added and the reaction system was stirred at 40 °C for 3 hours. The reaction liquid was filtered and the filtrate was concentrated to obtain the crude hydrochloride of **WX012-2**, which was directly used in the next step. ¹HNMR (400 MHz, DMSO-d6) δ ppm 3.35 (s, 3H), 4.24 (s, 2H), 8.86 (br s, 4 H).

### Step 2: Synthesis of compound WX012-3

Ethyl 1-BOC-3-oxo-4-piperidine carboxylate (2.00 g, 7.37 mmol, 1 *eq*), **WX012-2** (2.20 g, crude hydrochloride), sodium ethoxide (1.11 g, 16.33 mmol, 2.21 *eq*) and anhydrous ethanol (30 mL) were added to a reaction flask and the reaction system was stirred at 100 °C for 4 hours. The reaction liquid was concentrated and water (20 mL) was added. The mixture was adjusted to about pH 6 with hydrochloric acid (1 M) and extracted with ethyl acetate (3 * 20 mL). The organic phases were combined, washed with saturated brine (20 mL), dried over anhydrous sodium sulfate and filtered. The filtrate was concentrated to obtain a crude. To the crude were added petroleum ether: ethyl acetate (20:1, 20 mL), and the mixture was stirred at room temperature (20 °C) for 0.2 hours and filtered. The filter cake was dried to obtain **WX012-3**. ¹H NMR (400 MHz, CDCl₃) δ ppm 1.49 (s, 9H), 2.62 (br s, 2H), 3.52 (s, 3H), 3.62 (br t, J=5.57 Hz, 2H), 4.35 (br s, 2H), 4.38 (s, 2H), 9.69 (br s, 1 H); LCMS: m/z [M+1]⁺=295.9.

### Step 3: Synthesis of compound WX012-4

**WX012-3** (1.60 g, 5.42 mmol, 1 eq), triphenylphosphine (2.85 g, 10.87 mmol, 2.01 eq) and anhydrous toluene (20 mL) were added to a reaction flask. Under nitrogen atmosphere, 2,2,2-trichloroacetonitrile (1.18 g, 8.18 mmol, 0.82 mL, 1.51 eq) was added and the reaction system was stirred at 100 °C for 1.5 hours. The reaction liquid was filtered, and the filtrate was concentrated to obtain a crude. To the crude was added a mixed solvent (petroleum ether : ethyl acetate = 10 : 1, 30 mL). At room temperature (20 °C), the mixture was stirred for 0.2 hours and filtered. The filtrate was concentrated and the crude was separated and purified by column chromatography (petroleum ether : ethyl acetate = 1 : 0-5 : 1) to obtain **WX012-4**. LCMS: m/z [M+1]⁺ = 313.9.

### Step 4: Synthesis of compound WX012-5

**WX012-5** (0.65 g, 2.07 mmol, 1 *eq*), **WX001-2** (0.45 g, 2.29 mmol, 1.11 *eq*), potassium carbonate (0.56 g, 4.05 mmol, 1.96 *eq*) and anhydrous DMF (10 mL) were added to a reaction flask and the reaction system was stirred at 100 °C for 1 hour. To the reaction liquid was added water (20 mL). The mixture was extracted with ethyl acetate (3 * 20 mL). The organic phases were combined, washed with saturated brine (2 * 20 mL), dried over anhydrous sodium sulfate and filtered and the filtrate was concentrated to obtain a crude. The crude was separated and purified by column chromatography (PE : EA = 1 : 0-2 : 1) to obtain **WX012-5**. LCMS: m/z [M+1]⁺ = 473.9.

### Step 5: Synthesis of compound WX012-6

To a reaction flask were added **WX012-5** (0.30 g, 633.60 µmοl, 1 *eq*) and anhydrous DCM (3 mL), followed by trifluoroacetic acid (20.26 mmol, 1.50 mL, 31.98 *eq*) and the resulting reaction system was stirred at room temperature (20 °C) for 2 hours. The reaction liquid was adjusted to about pH 7 by dropwise adding saturated sodium bicarbonate solution. The mixture was subjected to liquid separation and the aqueous phase was extracted with DCM (2 * 10 mL). The organic phases were combined, washed with saturated brine (10 mL), dried over anhydrous sodium sulfate and filtered and the filtrate was concentrated to obtain crude **WX012-6**, which was directly used in the next step. LCMS: m/z [M+1]⁺ = 373.9.

### Step 6: Synthesis of compound WX012-7

**WX012-6** (0.15 g, 401.75 µmοl, 1 *eq*), **A-2** (0.10 g, 401.46 µmοl, 1 *eq*), N,N-diisopropylethylamine (1.44 mmol, 0.25 mL, 3.57 *eq*) and DMA (1.5 mL) were added to a reaction flask and the reaction system was stirred at 100 °C for 3 hours. To the reaction liquid was added water (10 mL). The mixture was extracted with ethyl acetate (3 * 10 mL). The organic phases were combined, washed with saturated brine (2 * 10 mL), dried over anhydrous sodium sulfate and filtered and the filtrate was concentrated to obtain a crude. The crude was separated and purified by prep-TLC (PE : EA = 1 : 1) to obtain **WX012-7**. LCMS: m/z [M+23]⁺ = 607.8.

### Step 7: Synthesis of compound WX012

To a reaction flask were added **WX012-7** (0.12 g, 204.78 µmοl, 1 eq) and anhydrous DCM (2 mL), followed by boron tribromide (830.26 µmοl, 80 µL, 4.05 eq) and the reaction system was stirred at room temperature (20 °C) for 1 hour. Saturated sodium carbonate solution was added dropwise to the reaction liquid to quench the reaction. The reaction solution was adjusted to about pH 7 and concentrated to obtain a crude. To the crude was added a mixed solvent (DCM : MeOH = 10 : 1, 5 mL). The resulting mixture was stirred uniformly and filtered and the filtrate was concentrated. The crude was separated and purified by a preparative plate (DCM : MeOH = 20 : 1), and further separated and purified by preparative high-performance liquid chromatography (chromatographic column: Phenomenex C18 80 * 40 mm * 3 µm; mobile phase: [water (ammonia water)-ACN]; gradient (ACN)%: 35%-65%) to obtain **WX012**. ¹H NMR (400 MHz, CDCl₃) δ ppm 2.96 - 3.15 (m, 3H), 3.89 (br t, J=5.40 Hz, 2H), 4.52 (br d, J=4.02 Hz, 2H), 4.61 (s, 2H), 7.37 (br t, J=6.90 Hz, 1H), 7.59 (dd, J=8.66, 2.38 Hz, 1H), 7.69 (br dd, J=8.41, 5.40 Hz, 1H), 7.76 (s, 1H), 11.38 (br s, 1 H); LCMS: m/z [M+1]⁺ = 488.0.

### Example 13

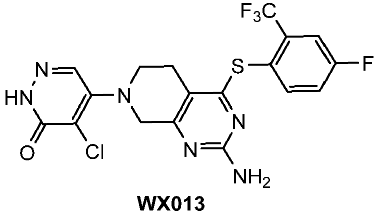

### Synthetic route:

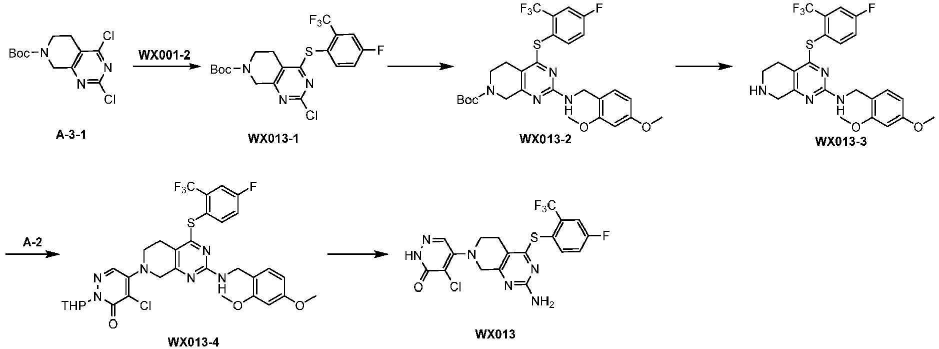

### Step 1: Synthesis of compound WX013-1

**A-3-1** (18 g, 59.18 mmol, 0.95 eq) and **WX001-2** (15.27 g, 62.29 mmol, purity: 80%, 1 eq) were dissolved in ACN (200 mL) and potassium carbonate (25.83 g, 186.88 mmol, 3 eq) was added. The reaction mixture was stirred at 50 °C for 4 hours. The mixture was cooled to room temperature and filtered and the filtrate was concentrated under reduced pressure to obtain a crude. The crude was subjected to column chromatography (petroleum ether : ethyl acetate = 20 : 1 to 10 : 1) to obtain **WX013-1.** LCMS: m/z [M+1]⁺ = 464.0.

### Step 2: Synthesis of compound WX013-2

**WX013-1** (13 g, 28.02 mmol, 1 eq), ACN (100 mL) and 2,4-dimethoxybenzylamine (23.43 g, 140.12 mmol, 21.11 mL, 5 eq) were added to a reaction flask and the resulting mixture was stirred at 80 °C under nitrogen for 12 hours. The reaction liquid was cooled to room temperature and filtered. The filter cake was separately removed and the filtrate was concentrated under reduced pressure and then separated and purified by column chromatography (PE : EA = 3 : 1) to obtain a crude. The crude and filter cake were combined and added to MeOH (15 mL). The mixture was stirred at room temperature (20 °C) for 1 hour and filtered. The filter cake was collected to obtain **WX013-2**. ¹H NMR (400 MHz, CDCl₃) δ ppm 1.49 (s, 9H), 2.64 (br s, 2H), 3.70 (br s, 2H), 3.79 (s, 6H), 4.04 (br s, 2H), 4.41 (br s, 2H), 6.29 (br d, J=8.78 Hz, 1H), 6.34 - 6.57 (m, 2H), 7.18 (br s, 1H), 7.45 (br d, J=8.03 Hz, 1H), 7.59 (dd, J=8.41, 5.65 Hz, 1H); LCMS: m/z [M+1]⁺ = 595.

### Step 3: Synthesis of compound WX013-3

**WX013-2** (10 g, 16.82 mmol, 1 eq) and a solution of hydrogen chloride in dioxane (4 M, 100 mL) were added to a reaction flask and the reaction system was stirred at room temperature (20 °C) for 3 hours. The reaction liquid was adjusted to about pH 8 by adding saturated sodium bicarbonate solution, and extracted with DCM (100 mL * 3). The organic phases were combined, washed with saturated brine (100 mL), dried over anhydrous sodium sulfate, filtered and concentrated to obtain **WX013-3**, which was directly used in the next step. ¹H NMR (400 MHz, CDCl₃) δ ppm 2.63 (br t, J=5.65 Hz, 2H), 3.21 (t, J=5.77 Hz, 2H), 3.79 (d, J=4.27 Hz, 6H), 3.83 (s, 2H), 4.04 (br s, 2H), 6.29 (br d, J=8.28 Hz, 1H), 6.41 (d, J=2.26 Hz, 1H), 6.59 (br s, 1H), 7.15 (br t, J=8.03 Hz, 1H), 7.43 (dd, J=9.16, 2.89 Hz, 1H), 7.59 (dd, J=8.53, 5.52 Hz, 1H); LCMS: m/z [M+1]⁺ = 494.9.

### Step 4: Synthesis of compound WX013-4

**WX013-3** (11 g, 22.24 mmol, 1 eq), **A-2** (4.99 g, 20.02 mmol, 0.9 eq) and N,N-diisopropylethylamine (110 mL) were mixed, warmed to 100 °C and stirred for 12 hours. The reaction liquid was concentrated under reduced pressure and the crude was separated and purified by column chromatography (PE : EA, EA% = 0-60%) to obtain **WX013-4**. ¹H NMR (400 MHz, CDCl₃) δ ppm1.72 (br s, 6H), 2.81 (br t, J=5.52 Hz, 2H), 3.79 (d, J=1.76 Hz, 8H), 3.92 - 4.20 (m, 4H), 4.37 (s, 2H), 6.04 - 6.14 (m, 1H), 6.30 (br d, J=7.78 Hz, 1H), 6.36 - 6.53 (m, 2H), 7.15 - 7.22 (m, 1H), 7.46 (dd, J=8.78, 2.76 Hz, 1H), 7.60 (dd, J=8.41, 5.40 Hz, 1H), 7.75 (s, 1H), ; LCMS: m/z [M+1]⁺ = 707.

### Step 4: Synthesis of compound WX013

**WX013-4** (5 g, 7.07 mmol, 1 eq) and DCM (50 mL) were added to a reaction flask. Trifluoroacetic acid (16.12 g, 141.42 mmol, 10.47 mL, 20 eq) was added dropwise and the reaction system was stirred at 35 °C for 12 hours. The reaction liquid was concentrated and MeOH (50 mL) was added. The resulting mixture was then concentrated to obtain a crude. To the crude was added 50 mL of DCM and the mixture was stirred at room temperature for 1 hour and filtered. To the filter cake was added ethyl acetate (15 mL) and the mixture was stirred at room temperature for 3 hours and filtered. The filter cake was dried to obtain **WX013.** ¹H NMR (400 MHz, CDCl₃) δ ppm 2.88 (t, J=5.65 Hz, 2H), 3.86 (t, J=5.77 Hz, 2H), 4.58 (s, 2H), 7.53 (td, J=8.22, 2.64 Hz, 1H), 7.70 (dd, J=9.03, 2.76 Hz, 1H), 7.81 (dd, J=8.53, 5.27 Hz, 1H), 7.97 (s, 1H); LCMS: m/z [M+1]⁺ = 472.9.

### Example 14

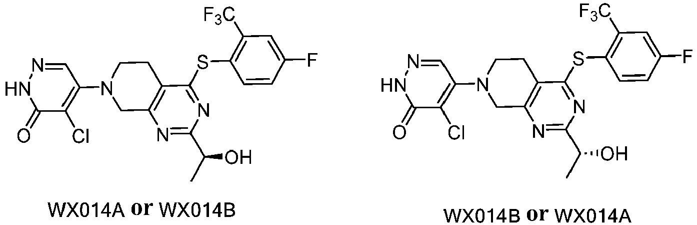

### Synthetic route:

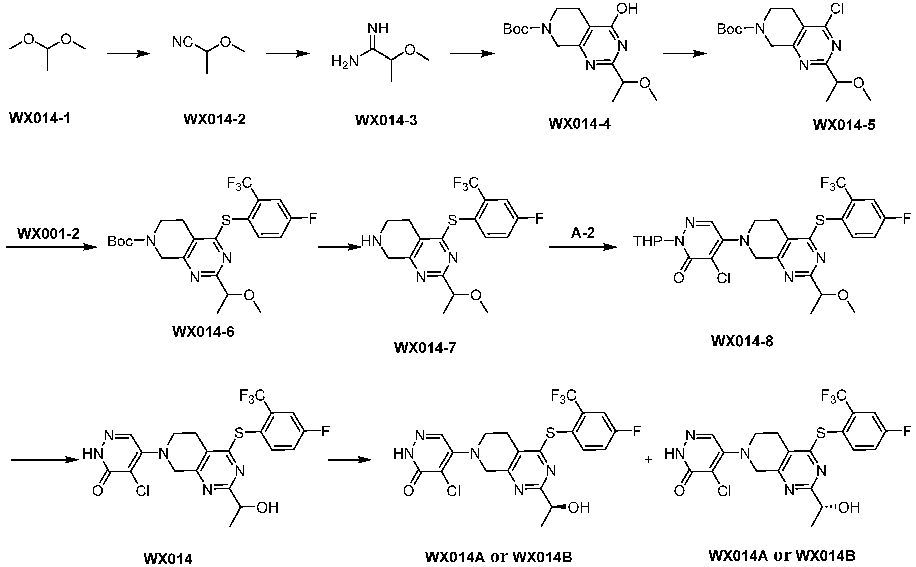

### Step 1: Synthesis of compound WX014-2

**WX014-1** (30 g, 332.89 mmol, 35.21 mL, 1 eq) and trimethylsilyl cyanide (33.02 g, 332.89 mmol, 41.64 mL, 1 eq) were added to a reaction flask. At 0 °C, boron trifluoride diethyl etherate solution (3.45 g, 24.31 mmol, 3.00 mL, 0.073 eq) was added slowly dropwise. After the addition, the mixture was stirred at 20 °C for 16 hours. Saturated sodium bicarbonate solution (20 mL) was added to the reaction liquid to quench the reaction. Then the mixture was extracted with DCM (3 × 50 mL) and the organic phase was dried over anhydrous sodium sulfate and concentrated under reduced pressure at room temperature 25 °C to obtain **WX014-2,** which was directly used in the next step. ¹H NMR (400 MHz, DMSO-d6) δ ppm 1.45 (s, 3H), 3.37 (s, 3H), 4.44 (q, J = 6.78 Hz, 1H).

### Step 2: Synthesis of compound WX014-3

**WX014-2** (20 g, 235.01 mmol, 1 eq) was added to a reaction flask containing ethanol (100 mL) and ammonia gas was introduced at -5 °C for 15 minutes. The reaction mixture was stirred at 25 °C for 16 hours. The reaction liquid was concentrated under reduced pressure and some of the ammonia gas was removed to obtain a solution of **WX014-3** in ethanol, which was directly used in the next step.

### Step 3: Synthesis of compound WX014-4

Sodium ethoxide (3.45 g, 50.68 mmol, 2.5 eq) was added to a solution of **WX014-3** in ethanol obtained in step 2. Then ethyl 1-Boc-3-oxo-4-piperidine carboxylate (5.5 g, 20.27 mmol, 1 eq) was added and the mixture was stirred and reacted at 100 °C under nitrogen protection for 6 hours. The reaction liquid was concentrated under reduced pressure and dissolved in water (100 mL). The mixture was adjusted to pH = 6 with 1 M aqueous hydrochloric acid solution and then extracted with ethyl acetate (2 × 100 mL). The organic phases were combined, washed with 100 mL of saturated brine and dried over anhydrous sodium sulfate. Then the filtrate was concentrated under reduced pressure to obtain a crude. The crude was purified by column chromatography (MeOH/DCM, MeOH% = 0%-6%) to obtain **WX014-4.** LCMS: m/z [M+1]⁺ = 309.9.

### Step 4: Synthesis of compound WX014-5

**WX014-4** (4.16 g, 13.45 mmol, 1 eq) and triphenylphosphine (10.58 g, 40.34 mmol, 3 eq) were added to anhydrous toluene (50 mL). Under nitrogen protection, 2,2,2-trichloroacetonitrile (2.91 g, 20.17 mmol, 2.02 mL, 1.5 eq) was added and the reaction mixture was reacted at 100 °C for 1 hour. The reaction liquid was cooled and 80 mL of saturated sodium bicarbonate solution was added to quench the reaction. The aqueous phase was extracted with ethyl acetate (2 × 80 mL). The organic phases were combined, washed with 100 mL of saturated brine, dried over anhydrous sodium sulfate and concentrated to obtain a crude. To the crude was added 100 mL of a mixed solvent (PE : EA = 3 : 1) and the mixture was stirred for 1 hour and then filtered. The filter cake was washed with a mixed solvent (PE : EA = 3 : 1, 50 mL). The filtrates were combined and concentrated to obtain a crude. The crude was purified by column chromatography (EA/PE, EA% = 0%-20%) to obtain **WX014-5.** LCMS: m/z [M+1]⁺ = 327.9.

### Step 5: Synthesis of compound WX014-6

Under nitrogen protection, to a reaction flask containing DMF (16 mL) were added **WX014-5** (1.62 g, 4.94 mmol, 1 eq) and **WX001-2** (1.51 g, 4.94 mmol, purity: 64%, 1 eq), followed by 1.8-diazabicyclo[5.4.0]undec-7-ene (1.50 g, 9.88 mmol, 1.49 mL, 2 eq) and the resulting mixture was stirred at 120 °C for 1 hour. The reaction liquid was cooled to room temperature and then 30 mL of ethyl acetate was added. The mixture was washed with 30 mL of saturated brine and the organic phase was dried over anhydrous sodium sulfate and concentrated to obtain a crude. The crude was purified by column chromatography (EA/PE, EA% = 0%-25%) to obtain **WX014-6.** LCMS: m/z [M+1]⁺ = 488.1.

### Step 6: Synthesis of compound WX014-7

To a reaction flask containing DCM (18 mL) was added **WX014-6** (1.79 g, 3.67 mmol, 1 eq), followed by trifluoroacetic acid (5 mL). The reaction mixture was reacted at 25 °C for 1 hour. To the reaction liquid was added 20 mL of water and then the mixture was adjusted to pH = 8 by adding sodium carbonate. The aqueous phase was extracted with DCM (2 × 20 mL). The organic phases were combined, washed with 20 mL of saturated brine, and dried over anhydrous sodium sulfate. The filtrate was concentrated under reduced pressure to obtain crude **WX014-7,** which was directly used in the next step. LCMS: m/z [M+1]⁺ = 388.0.

### Step 7: Synthesis of compound WX014-8

To a reaction flask containing N,N-diisopropylethylamine (30 mL) was added WX014-7 (1.5 g, 3.87 mmol, 1 eq), followed by **A-2** (1.06 g, 4.26 mmol, 1.1 eq) and the reaction mixture was stirred at 100 °C for 16 hours. The reaction liquid was directly concentrated under reduced pressure to obtain a crude. The crude was purified by column chromatography (MeOH/DCM, MeOH% = 0%-8%) to obtain **WX014-8.** LCMS: m/z [M+1]⁺= 600.1.

### Step 8: Synthesis of compound WX014

**WX014-8** (400 mg, 666.64 µmol, 1 eq) was added to a reaction flask containing anhydrous DCM (10 mL). The mixture was cooled to 0 °C and then a solution of boron tribromide (1.67 g, 6.67 mmol, 642.33 µL, 10 eq) in anhydrous DCM (5 mL) was added slowly dropwise. After the addition, the reaction mixture was reacted at 25 °C for 16 hours. The reaction liquid was added to aqueous sodium carbonate solution (10 mL) at 0 °C to quench the reaction. The aqueous phase was extracted with DCM (2 × 10 mL). The organic phases were combined, washed with saturated brine (10 mL), dried over anhydrous sodium sulfate and concentrated under reduced pressure to obtain a crude. The crude was purified by preparative high-performance liquid chromatography (chromatographic column: Phenomenex C18 150 * 40 mm * 5 µm; mobile phase: [water (HCl)-ACN]; gradient (ACN)%: 35%-55%) to obtain **WX014.** ¹H NMR (400 MHz, DMSO-d6) δ ppm 1.08 (d, J=6.53 Hz, 3H), 2.87 (br t, J=5.27 Hz, 2H), 3.79 (br t, J=5.52 Hz, 2H), 4.41 (q, J=6.53 Hz, 1H), 4.62 (s, 2H), 7.69 (td, J=8.47, 2.89 Hz, 1H), 7.82 - 7.91 (m, 2H), 7.98 (s, 1H), 12.96 (s, 1 H); LCMS: m/z [M+1]⁺ = 502.0.

### Step 9: Synthesis of compounds WX014A and WX014B

**WX014** (230 mg) was subjected to chiral preparative separation (chromatographic column: DAICEL CHIRALPAK AD (250 mm * 30 mm, 10 µm); mobile phase: A: CO₂, B: MeOH (0.1% ammonia water); gradient (B%): 35%-35%) to obtain **WX014A** and **WX014B.**

**WX014A:** SFC analysis method: chromatographic column: ChiralPakAD-3 150 × 4.6 mm I.D., 3 µm; mobile phase: A: CO₂, B: MeOH (0.05% DEA); gradient B: 40%, retention time: 3.356 minutes, ee = 100%. ¹H NMR (400 MHz, DMSO-d6) δ ppm 1.09 (d, J=6.53 Hz, 3H), 2.87 (br t, J=5.27 Hz, 2H), 3.80 (t, J=5.77 Hz, 2H), 4.37 - 4.46 (m, 1H), 4.63 (s, 2H), 4.88 (d, J=5.27 Hz, 1H), 7.70(td, J=8.41, 2.76 Hz, 1H), 7.83 - 7.93 (m, 2H), 7.99 (s, 1H), 12.96 (br s, 1 H); LCMS: m/z [M+1]⁺ = 502.0.

**WX014B:** SFC analysis method: chromatographic column: ChiralPakAD-3 150 × 4.6 mm I.D., 3 µm; mobile phase: A: CO₂, B: MeOH (0.05% DEA); gradient B: 40%, retention time: 3.859 minutes, ee = 97.5%. ¹ HNMR (400 MHz, DMSO-d6) δ ppm 1.09 (d, J=6.53 Hz, 3H), 2.87 (br t, J=5.52 Hz, 2H), 3.80 (br t, J=5.40 Hz, 2H), 4.42 (quin, J=6.21 Hz, 1H), 4.63 (s, 2H), 4.87 (d, J=5.27 Hz, 1H), 7.66 - 7.74 (m, 1H), 7.84 - 7.92 (m, 2H), 7.99 (s, 1H), 12.90 (s, 1 H); LCMS: m/z [M+1]⁺ = 502.0.

### Example 15

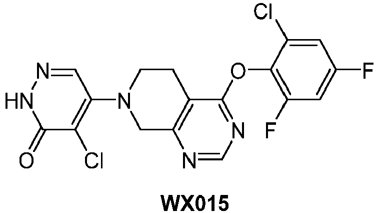

### Synthetic route:

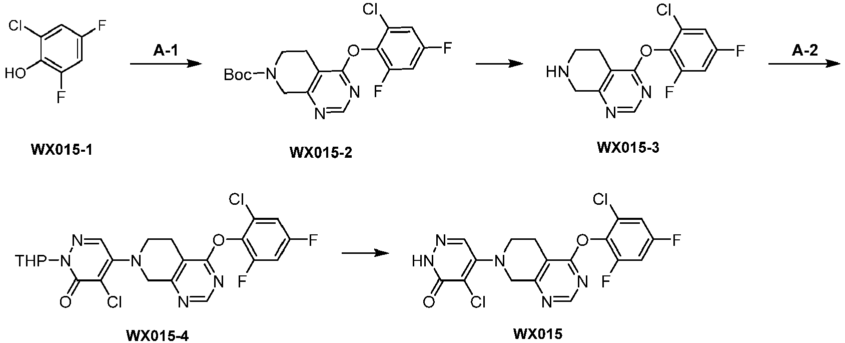

### Step 1: Synthesis of compound WX015-2

**WX015-1** (4.58 g, 27.81 mmol, 1.5 eq) and **A-1** (5 g, 18.54 mmol, 1 eq) were dissolved in DMA (50 mL) and DBU (5.64 g, 37.07 mmol, 2 eq) was added. The reaction mixture was reacted at 100 °C for 1.5 hours. The reaction was cooled to room temperature and 500 mL of half-saturated brine was added. The mixture was extracted with ethyl acetate (100 mL * 3). The organic phases were combined, washed with saturated brine (50 mL), dried over anhydrous sodium sulfate and filtered. The filtrate was concentrated under reduced pressure to obtain a crude. The crude was subjected to column chromatography (petroleum ether : ethyl acetate = 1 : 0 to 8 : 1) to obtain **WX015-2,** which was directly used in the next step. LCMS: m/z [M+1]⁺ = 398.1.

### Step 2: Synthesis of compound WX015-3

**WX015-2** (5.4 g, 13.57 mmol, 1 eq) was dissolved in anhydrous DCM (120 mL) and TFA (20 mL) was added. The reaction mixture was reacted at 20 °C for 1 hour. The reaction liquid was adjusted to pH 9 by adding saturated aqueous sodium carbonate solution and then extracted with DCM (50 mL * 3). The organic phases were combined, washed with saturated brine (50 mL), dried over anhydrous sodium sulfate and filtered. The filtrate was concentrated under reduced pressure to obtain crude **WX015-3.** LCMS: m/z [M+1]⁺ = 298.0.

### Step 3: Synthesis of compound WX015-4

To a reaction flask were added **WX015-3** (2.0 g, 5.81 mmol, 1 eq) and **A-2** (1.59 g, 6.39 mmol, 1.1 eq), followed by DIEA (229.64 mmol, 40 mL, 39.53 eq). Under nitrogen protection, the reaction mixture was stirred at 100 °C for 16 hours. The reaction mixture was cooled to room temperature and concentrated under reduced pressure to obtain a crude. The crude was purified by column chromatography (petroleum ether : ethyl acetate = 4 : 1 to 1 : 2) to obtain **WX015-4.** LCMS: m/z [M+1]⁺ = 510.0.

### Step 4: Synthesis of compound WX015

**WX015-4** (1.59 g, 3.12 mmol, 1 eq) was dissolved in anhydrous DCM (20 mL) and TFA (9 mL) was added. The reaction mixture was stirred at 20 °C for 2 hours. The reaction mixture was adjusted to about pH 9 by adding saturated aqueous sodium carbonate solution and filtered. The filter cake was dried to obtain **WX015.** ¹H NMR (400 MHz, DMSO-d6) δ ppm 3.03 (br t, J=5.27 Hz, 2 H) 3.79 (t, J=5.77 Hz, 2 H) 4.68 (s, 2 H) 7.57 - 7.65 (m, 2 H) 8.01 (s, 1 H) 8.58 (s, 1 H) 12.95 (s, 1 H); LCMS: m/z [M+1]⁺ = 425.9.

### Example 16

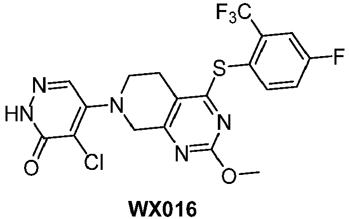

### Synthetic route:

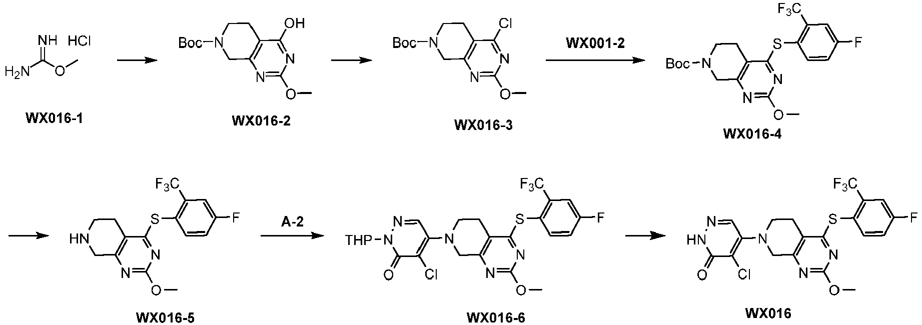

### Step 1: Synthesis of compound WX016-2

**WX016-1** (2.93 g, 26.54 mmol, 2.4 eq) and ethyl 1-Boc-3-oxo-4-piperidine carboxylate (3 g, 11.06 mmol, 1 eq) were dissolved in MeOH (30 mL). Sodium methoxide methanol solution (4.36 mL, 24.33 mmol, 2.2 eq, 30 wt%) was added and the reaction mixture was stirred at 100 °C for 4 hours. The reaction mixture was cooled to room temperature and concentrated under reduced pressure. To the residue was added 100 mL of water. The mixture was adjusted to about pH 6 with 1 M HCl and extracted with ethyl acetate (30 mL * 2). The organic phases were combined, washed with saturated brine (15 mL), dried over anhydrous sodium sulfate and filtered. The filtrate was concentrated under reduced pressure to obtain a crude. The crude was purified by column chromatography (petroleum ether : ethyl acetate = 2 : 1 to DCM : MeOH = 5 : 1). Then petroleum ether : ethyl acetate = 15 : 1 (30 mL) was added and the mixture was stirred at room temperature for 30 minutes and filtered. The filter cake was collected to obtain **WX016-2.** ¹H NMR (400 MHz, CDCl₃) δ ppm 1.50 (s, 9 H) 2.57 (br t, 2 H) 3.60 (br t, J=5.57 Hz, 2 H) 3.97 (s, 3 H) 4.29 (br s, 2 H) 11.14 (br s, 1 H).

### Step 2: Synthesis of compound WX016-3

**WX016-2** (1.5 g, 5.33 mmol, 1 eq) was mixed with anhydrous toluene (35 mL). Triphenylphosphine (2.80 g, 10.66 mmol, 2 eq) and 2,2,2-trichloroacetonitrile (8.00 mmol, 801.99 µL, 1.5 eq) were sequentially added. Under nitrogen protection, the reaction mixture was stirred at 100 °C for 1.5 hours. The reaction mixture was cooled to room temperature and concentrated under reduced pressure. The residue was purified by column chromatography (PE : EA = 1 : 0 to 5 : 1) to obtain **WX016-3.** ¹H NMR (400 MHz, CDCl₃) δ ppm 1.46 (s, 9 H) 2.74 (br t, J=5.77 Hz, 2 H) 3.68 (t, J=5.90 Hz, 2 H) 3.97 (s, 3 H) 4.52 (s, 2 H).

### Step 3: Synthesis of compound WX016-4

**WX016-3** (1.27 g, 4.24 mmol, 1 eq) and **WX001-2** (1.30 g, 4.24 mmol, 1 eq) were dissolved in ACN (13 mL) and potassium carbonate (1.76 g, 12.71 mmol, 3 eq) was added. The reaction mixture was stirred at 50 °C for 4 hours. The reaction mixture was cooled to room temperature and filtered and the filtrate was concentrated under reduced pressure. The residue was purified by column chromatography (petroleum ether : ethyl acetate = 1 : 0 to 10 : 1) to obtain **WX016-4.** ¹H NMR (400 MHz, CDCl₃) δ ppm 1.48 (s, 9 H) 2.71 (br t, J=5.63 Hz, 2 H) 3.55 (s, 3 H) 3.74 (br t, J=5.75 Hz, 2 H) 4.50 (s, 2 H) 7.31 (td, J=8.13, 2.75 Hz, 1 H) 7.53 (dd, J=8.82, 2.81 Hz, 1 H) 7.67 (dd, J=8.63, 5.38 Hz, 1 H).

### Step 4: Synthesis of compound WX016-5

**WX016-4** (1.39 g, 3.03 mmol, 1 eq) was dissolved in DCM (14 mL). TFA(2.50 mL) was added dropwise. After the addition, the reaction mixture was stirred at 25 °C for 1 hour. The reaction mixture was adjusted to pH 9 by adding saturated sodium carbonate solution, and extracted with DCM (15 mL * 3). The organic phases were combined, washed with saturated brine (15 mL), dried over anhydrous sodium sulfate and concentrated under reduced pressure to obtain crude WX016-5, which was directly used in the next step. LCMS: m/z [M+1]⁺ = 360.3.

### Step 5: Synthesis of compound WX016-6

To a 100 mL one-necked flask were added **WX016-5** (1.01 g, 2.81 mmol, 1 eq) and **A-2** (700.13 mg, 2.81 mmol, 1 eq), followed by DIEA (196.75 mmol, 34.27 mL, 70 eq) and under nitrogen protection, the reaction mixture was stirred at 100 °C for 16 hours. The reaction mixture was cooled to room temperature and concentrated under reduced pressure. The residue was subjected to column chromatography (petroleum ether : ethyl acetate = 4 : 1 to 1 : 1) to obtain **WX016-6.** ¹H NMR (400 MHz, CDCl₃) δ ppm 1.60 - 1.70 (m, 6 H) 2.92 (br t, J=5.69 Hz, 2 H) 3.60 (s, 3 H) 3.70 - 3.95 (m, 4 H) 4.49 - 4.53 (m, 2 H) 6.06 - 6.12 (m, 1 H) 7.36 (td, J=8.10, 2.69 Hz, 1 H) 7.57 (dd, J=8.82, 2.69 Hz, 1 H) 7.72 (dd, J=8.38, 5.50 Hz, 1 H) 7.77 (s, 1 H); LCMS: m/z [M+1]⁺ = 572.1.

### Step 6: Synthesis of compound WX016

**WX016-6** (400 mg, 699.33 µmol, 1 eq) was dissolved in DCM (3 mL). TFA (570 µL) was added dropwise. After the addition, the reaction mixture was reacted at 25 °C for 1 hour. The reaction mixture was adjusted to about pH 8-9 by adding saturated aqueous sodium carbonate solution and filtered. The filter cake was washed with 2 mL of dichloromethane and dried to obtain **WX016.** ¹H NMR (400 MHz, DMSO-*d*₆) δ ppm 2.75 - 2.86 (m, 2 H) 3.55 (s, 3 H) 3.77 (br t, *J*=5.57 Hz, 2 H) 4.54 (s, 2 H) 7.71 (td, *J*=8.38, 2.75 Hz, 1 H) 7.90 (dd, *J*=9.13, 3.25 Hz, 2 H) 7.97 (s, 1 H) 12.97 (br s, 1 H); LCMS: m/z [M+1]⁺ = 488.1.

### Example 17

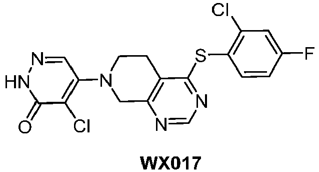

### Synthetic route:

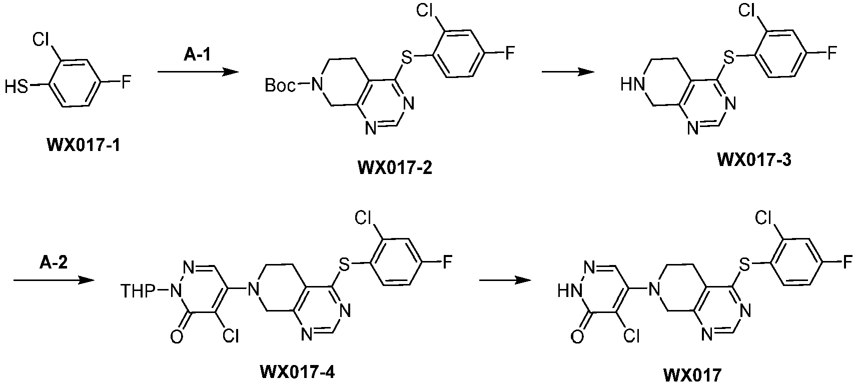

### Step 1: Synthesis of compound WX017-2

Under nitrogen protection, **A-1** (2.5 g, 9.27 mmol, 1 eq) and **WX017-1** (1.51 g, 9.27 mmol, 1 eq) were added to DMF (25 mL) and then DBU (18.54 mmol, 2.79 mL, 2 eq) was added. The reaction mixture was stirred at 120 °C for 1 hour. The reaction mixture was cooled to room temperature and then 50 mL of ethyl acetate was added. The mixture was sequentially washed with half-saturated brine (30 mL × 3) and 30 mL of saturated brine, and then dried over anhydrous sodium sulfate. The organic phase was concentrated under reduced pressure to obtain a crude, which was purified by column chromatography (EA/PE, EA%: 0%-30%) to obtain **WX017-2.** LCMS: m/z [M+1]⁺ = 396.0

### Step 2: Synthesis of compound WX017-3

**WX017-2** (2.9 g, 7.33 mmol, 1 eq) was added to DCM (29 mL) and then TFA (9.67 mL) was added. The reaction mixture was stirred at 25 °C for 1 hour. To the reaction mixture was added 30 mL of water and then the mixture was adjusted to pH = 8 with anhydrous sodium carbonate. The aqueous phase was extracted with dichloromethane (30 mL × 2) and the organic phases were combined, washed with 30 mL of saturated brine, dried over anhydrous sodium sulfate and then concentrated under reduced pressure to obtain crude **WX017-3,** which was directly used in the next step. LCMS: m/z [M+1]⁺ = 295.9.

### Step 3: Synthesis of compound WX017-4

**WX017-3** (1.5 g, 3.87 mmol, 1 eq) was added to DIEA (40 mL) and then **A-2** (1.95 g, 7.81 mmol, 1.05 eq) was added. The reaction mixture was stirred at 100 °C for 3 hours. The reaction mixture was cooled and concentrated under reduced pressure to obtain a crude. The crude was purified by column chromatography (DCM : MeOH, MeOH% : 0%-6%) to obtain **WX017-4.** LCMS: m/z [M+23]⁺ = 530.0.

### Step 4: Synthesis of compound WX017

To a reaction flask containing DCM (30 mL) was added **WX017-4** (2.8 g, 5.51 mmol, 1 eq), followed by TFA (7.34 mL) and the reaction mixture was stirred at 25 °C for 1 hour. To the reaction mixture was added 30 mL of water and then the mixture was adjusted to pH = 8 by adding anhydrous sodium carbonate and filtered. The filter cake was sequentially washed with 30 mL of DCM and 10 mL of water and dried to obtain a crude. The crude was subjected to preparative high-performance liquid chromatography (chromatographic column: Phenomenex C18 150 * 40 mm * 5 µm; mobile phase: [water (HCl)-ACN]; gradient (acetonitrile)%: 30%-60%) to obtain **WX017.** ¹H NMR (400 MHz, DMSO-d6) δ ppm 2.90 (br t, J=5.13 Hz, 2 H) 3.80 (br t, J=5.50 Hz, 2 H) 4.62 (s, 2 H) 7.37 (td, J=8.47, 2.69 Hz, 1 H) 7.72 (dd, J=8.75, 2.63 Hz, 1 H) 7.80 (dd, J=8.63, 6.13 Hz, 1 H) 8.00 (s, 1 H) 8.62 (s, 1 H) 13.00 (br s, 1 H); LCMS: m/z [M+1]⁺ = 424.1.

### Biological test data

### Experimental example 1: hTRPC5-T478C_FLIPR calcium flux IC₅₀ assay

### Experimental method:

Cell culture: hTRPC5-T478C-HEK cells were cultured in a humid environment in 5% CO₂ at 37 °C.

Cell culture medium ingredients:
DMEM, Supplier: Gibco, Catalog No. 11965-092;
FBS, Supplier: Gibco, Catalog No. 10099-141C, Concentration: 10%;
Pen/Strep, Supplier: Invitrogen, Catalog No. 15140-122, Concentration: 1%;
Zeocin, Supplier: Invitrogen, Catalog No. R25005, Concentration: 200 µg/mL;
Blasticidin S, Supplier: Invitrogen, Catalog No. R21001, Concentration: 5 µg/mL.

### Cell passaging

In general, the TRPC5-T478C cell line was passaged three times weekly. The dilution factor for each passage was 1:3. Once the cells reached about 80% confluence in the T-75 flask, the cell line was digested with trypsin for about 1 minute and the cell suspension was removed from the flask with a pipette. Depending on the dilution ratio, the removed cell suspension would be transferred to another T-75 flask containing cell culture medium. Note that in order to maintain the logarithmic growth of the cells, the confluence of the cells should be maintained at the subconfluent stage and the cells should be passaged once every 2 to 3 days depending on the proliferation rate of the cell line.

### Steps of FLIPR calcium flux assay

(1) Preparation of cell plate: the cells were separated according to the above-mentioned passaging and the cell density and viability were determined using a cell counter. The volume of the cell suspension was adjusted with a cell culture medium. After the density reached to the plating density, 1 µg/mL tetracycline was added to induce hTRPC5-T478C channel expression. The induced hTRPC5-T478C cells were then seeded onto poly-D-lysine (PDL)-coated 384-well plates at a density of 20000 cells/well (30 µL/well). Then the cells were cultured overnight in a cell culture incubator.
(2) Preparation of compound plate: the compounds were generally diluted with DMSO, prepared into a mother liquor and stored in a -20 °C refrigerator. A compound plating program was set on the ECHO processor, and the compounds were plated with ECHO according to the concentration gradient that was already set. In general, 9 concentrations were included with the highest concentration at 10 µM, 3-fold dilution, and 2 replicate wells. Englerin A was used as the channel agonist.
(3) On-machine testing: the cell plates and compound plates were prepared according to the above-mentioned methods. After the cell confluence of the cell plate reached 80%-90%, the cells were removed from the incubator and the testing started. Ca5 dye (Molecular Devices # R8185) was formulated with test buffer. After the cell plate culture medium was removed with Bravo, 25 µL of Ca5 dye was added to each well, and the plate was incubated for 1 hour. After 1 hour, the cell plate and compound plate were placed in FLIPR^{TETRA} (Molecular Devices, USA) for FLIPR detection.

### Data analysis:

Data analysis was performed using Excel 2013 (Microsoft, USA) software and GraphPad Prism 6.01 software. The maximum signal was generated by FLIPR software. Data analysis was performed using Excel (2013) and Prism 6.01. Data quality and data stability were controlled using S/B > 2.50 and Z factor> 0.50. The calculation formula was as follows: S/B = Avg100%/Avg0%; Z factor = 1 - (3 * SD100% + 3 * SD0%)/| Avg100%-Avg0% |.

**Experimental results:** The experimental results are shown in Table 1.

**Table 1 hTRPC5-T478C _FLIPR calcium flux IC₅₀ assay results of the compounds of the present disclosure**

| Test compound | FLIPR calcium flux IC₅₀ (nM) |
|---|---|
| Compound WX002 | 27 |
| Compound WX003 | 70 |
| Compound WX004 | 40 |
| Compound WX009 | 37 |
| Compound WX010 | 71 |
| Compound WX011 | 80 |
| Compound WX012 | 24 |
| Compound WX013 | 55.7 |
| Compound WX014 | 12 |
| Compound WX017 | 56 |

Conclusion: the compounds of the present disclosure had significant inhibitory effects on TRPC5.

### Experimental example 2: hTRPC5-T478C_patch clamp IC₅₀ assay

### Experimental method:

Cell culture: hTRPC5-T478C-HEK cells were cultured in a humid environment in 5% CO₂ at 37 °C.

Cell culture medium ingredients:
DMEM, Supplier: Gibco, Catalog No. 11965-092;
FBS, Supplier: Gibco, Catalog No. 10099-141C, Concentration: 10%;
Pen/Strep, Supplier: Invitrogen, Catalog No. 15140-122, Concentration: 1%;
Zeocin, Supplier: Invitrogen, Catalog No. R25005, Concentration: 200 µg/mL;
Blasticidin S, Supplier: Invitrogen, Catalog No. R21001, Concentration: 5 µg/mL.

### Cell passaging:

In general, the TRPC5-T478C cell line was passaged three times weekly. The dilution factor for each passage was 1:3. Once the cells reached about 80% confluence in the T-75 flask, the cell line was digested with trypsin for about 1 minute and the cell suspension was removed from the flask with a pipette. Depending on the dilution ratio, the removed cell suspension would be transferred to another T-75 flask containing cell culture medium. Note that in order to maintain the logarithmic growth of the cells, the confluence of the cells should be maintained at the subconfluent stage and the cells should be passaged once every 2 to 3 days depending on the proliferation rate of the cell line.

### Solutions:

(1) Extracellular fluid: sodium chloride (145 mmol/L), potassium chloride (4 mmol/L), magnesium chloride (1.5 mmol/L), glucose (10 mmol/L), hydroxyethyl piperazineethanesulfonic acid (HEPES, 10 mmol/L). The extracellular fluid was adjusted to pH 7.4 with NaOH, and the osmotic pressure was adjusted to about 305 mOsm/L with pure water or sucrose.
(2) Intracellular fluid: cesium chloride (140 mmol/L), ethylene glycol diethyl ether diamine tetraacetic acid (EGTA, 1 mmol/L), magnesium chloride (2 mmol/L), glucose (10 mmol/L), hydroxyethyl piperazineethanesulfonic acid (HEPES, 10 mmol/L). The intracellular fluid was adjusted to pH 7.2 with HCl, and the osmotic pressure was adjusted to about 295 mOsm/L with pure water or sucrose.
(3) The extracellular fluid was required to be formulated once a week, and the intracellular fluid was required to be subpackaged and stored at -20 °C after formulation.

### Preparation of compounds:

Positive reference compounds Englerin A and 2-aminoethoxydiphenyl borate (2-APB) were dissolved in 100% DMSO and stored as a 10 mM working solution after formulation. The test compounds were dissolved in 100% DMSO and stored as a 10 mM working solution after formulation. The final concentration of DMSO in the test solution should be less than 0.3%.

### Whole-cell voltage clamp recordings:

Whole-cell patch clamping was performed at room temperature. The electrical signals recorded using an EPC 10 USB amplifier (HEKAElektronik, Germany) were low-pass filtered at 3 kHz and finally recorded in PatchMaster 2 × 90.5 software (HEKA Elektronik, Germany). In this step, the quality control standards were that the cell high-resistance seal was formed at a value greater than 500 MOhms and the detection current was greater than 0.3 nA.

The recording electrode was a borosilicate glass capillary (GC150tF-10, Harvard Apparatus Co., UK), which was pulled by a vertical puller (NARISHIGE PC-10, Japan) and polished. In this step, the quality control standard was that the electrode resistance was between 2 to 5 MΩ.

During whole-cell patch clamp recordings, a continuous filling system (BT100-2J, LongerPump, China) was used to continuously perfuse extracellular fluid. The filling system was mounted on the stage of an upright microscope (FN-S2N, Nikon, Japan), and the filling tip was manually positioned under the microscope.

The voltage instructions for hTRPC5-T478C current amplitude detection were as follows: the cells were clamped as follows: the clamping potential changed in a stepped manner from -40 mV to -80 mV (duration: 50 ms); then the clamping voltage ramped up to +80 mV, and the whole process lasted for 200 ms; then clamping was performed at +80 mV for 50 ms, and finally, the voltage returned to the clamping potential of -40 mV. The detection voltage instructions were repeated every 5000 milliseconds and were executed continuously during the test for the compounds. The quality control standard for the stability of the compounds was the current amplitude under three consecutive voltage instructions with a coefficient of variation < 5%. If the compounds have no effect on the hTRPC5-T478C current amplitude, continuous monitoring is required for 5 minutes.

### Data analysis:

Data analysis was performed using PatchMaster 2 × 90.5 software (HEKAElektronik, Germany), Excel 2013 (Microsoft, USA) software and GraphPad Prism 6.01 software. For each cell's patch clamp recording, it was required to obtain the hTRPC5-T478C channel current amplitude (I_{unblock}) stimulated by the hTRPC5 agonist Englerin A, the hTRPC5-T478C channel current amplitude (I_{block}) after inhibition by the hTRPC5 inhibitor 2-APB, and the hTRPC5-T478C channel current amplitude (Iₜₑₛₜ) corresponding to the concentration of the test compounds. Iₜₑₛₜ minus cell background current (I_{block}) provides the remaining channel current after compound treatment. The remaining current percentage can be obtained by dividing same by the overall cell channel current (I_{unblock}-I_{block}). Subtracting the remaining current percentage from 100% provides the compound inhibition efficiency. That is, compound inhibition efficiency = 100%- (Iₜₑₛₜ- I_{blocked})/(I_{unblocked}-I_{blocked}).

### Experimental results: See table 1.

**Table 1 hTRPC5-T478C patch clamp IC₅₀ assay results of the compounds of the present disclosure**

| Test compound | Patch clamp IC₅₀ (nM) |
|---|---|
| Compound WX002 | 114.70 |
| Compound WX003 | 89.40 |
| Compound WX004 | 91.40 |
| Compound WX009 | 304.40 |
| Compound WX010 | 196.50 |
| Compound WX011 | 190.50 |
| Compound WX012 | 16.63 |
| Compound WX013 | 15.33 |

Experimental conclusion: the compounds of the present disclosure had significant inhibitory effects on TRPC5.

### Experimental example 3: In vivo PK studies

### Objectives:

Male SD rats were used as test animals. After intravenous injection, the blood drug concentration of the compounds was determined and the pharmacokinetic behavior was evaluated.

### Experimental operations:

Three healthy adult male SD rats were selected. The test compounds were mixed with an appropriate amount of a vehicle of the intravenous injection group and prepared into a 0.5 or 1 mg/mL clear solution, which was filtered with a 0.22 µM microporous filter membrane for later use. After intravenous administration to the rats at 0.5 or 1 mg/kg, whole blood was collected at 0.083, 0.25, 0.5, 1, 2, 4, 8, 24 hours after administration and placed in EDTA-K2 anticoagulant tubes, and the mixture was vortexed thoroughly and centrifuged at 3200 g, 4 °C for 10 minutes to obtain the plasma. The LC-MS/MS method was used to determine the drug concentration, and the Phoenix WinNonlin 6.3 pharmacokinetic software was used to calculate the relevant pharmacokinetic parameters by the non-compartmental model linear logarithmic trapezoidal method.
Vehicle 1: 10% DMSO/10% Kolliphor^{®} HS 15/80% (20% sulfobutyl-β-cyclodextrin);
Vehicle 2: 10% DMSO+20% polyoxyethylenated castor oil+70% water;
Vehicle 3: 5% DMSO+10% Kolliphor^{®} HS 15 +85% physiological saline;
Vehicle 4: 10% DMSO+60% polyethylene glycol 400+30% (10% aqueous hydroxypropyl-β-cyclodextrin solution).

The experimental results are shown in Table 2:

**Table 2 PK test results of the compounds of the present disclosure**

| Compound | Vehicle | Dose (mpk) | Vdₛₛ (L/kg) | Cl (mL/min/kg) | T_{1/2} (h) | AUC₀₋ₗₐₛₜ (nM.h) |
|---|---|---|---|---|---|---|
| **WX002** | Vehicle 1 | 1 | 2.28 | 18.2 | 1.83 | 1969 |
| **WX004** | Vehicle 2 | 1 | 0.77 | 4.6 | 2.02 | 8260 |
| **WX009** | Vehicle 3 | 0.5 | 1.37 | 6.52 | 2.59 | 2375 |
| **WX010** | Vehicle 2 | 1 | 1.13 | 7.81 | 1.73 | 4755 |
| **WX011** | | 1 | 1.06 | 8.29 | 1.46 | 4053 |
| **WX012** | Vehicle 4 | 1 | 1.62 | 10.1 | 2.31 | 3331 |
| **WX013** | | 1 | 0.88 | 3.94 | 2.65 | 9126 |

| | | | | | | |
|---|---|---|---|---|---|---|
| Note: Vdₛₛ represents volume of distribution; Cl represents clearance rate; T_{1/2} represents half-life; AUC₀₋ₗₐₛₜ represents exposure. | | | | | | |

**Conclusion:** the compounds of the present disclosure exhibited a large apparent volume of distribution, a low *in-vivo* clearance rate, a high exposure and good pharmacokinetic properties in rats.

### Experimental example 4: Rat group tissue distribution test

### Objectives:

In this study, SD male rats were selected as the test animals, and the LC/MS/MS method was used to quantitatively determine the drug concentration at different time points in the plasma, cerebrospinal fluid, brain, liver, and kidney of the test compounds administered orally to evaluate the tissue distribution of the test drugs in the rats.

### Experimental method:

Experimental materials: Sprague Dawley (SD) rats (male, 200-300 g, 7-10 weeks old, Beijing Vital River).

### Experimental operations:

A clear solution or suspension of the test compounds was administered intragastrically into SD rats (fasted) (vehicle: 5% DMSO/10% Kolliphor^{®} HS 15 /85% water). At 1, 4 and 24 h after administration, about 0.2 mL of blood was collected from the jugular vein and placed in EDTA-K2 anticoagulant tubes. The mixture was vortexed thoroughly and centrifuged at 3200 g, 4 °C for 10 minutes to obtain the plasma. The cerebrospinal fluid (about 0.02 mL) was collected. The brain, liver, and kidney were collected, rinsed with pre-cooled physiological saline, dried with paper, and then homogenated in a ratio of 1 : 4 (1 g of tissue: 4 mL of 15 mM phosphate-buffered physiological saline/MeOH (2 : 1)). The LC-MS/MS method was used to determine the drug concentration, and the Phoenix WinNonlin 6.3 pharmacokinetic software was used to calculate the relevant pharmacokinetic parameters by the non-compartmental model linear logarithmic trapezoidal method.

The experimental results are shown in Table 3.

**Table 3 rat group tissue distribution test results of the compounds of the present disclosure**

| Tissue | Time point (h) | Average drug concentration (nM) n = 3 | |
|---|---|---|---|
| | | Compound 12 | Compound 13 |
| Brain | 1 | 282 | 139 |
| | 4 | 373 | 197 |
| | 12 | BQL | 86.0 |
| Liver | 1 | 5117 | 2612 |
| | 4 | 5817 | 3358 |
| | 12 | 570 | 945 |
| Kidney | 1 | 2633 | 1258 |
| | 4 | 3437 | 1468 |
| | 12 | 276 | 590 |
| Plasma | 1 | 773 | 536 |
| | 4 | 1079 | 716 |
| | 12 | 82.7 | 272 |

| | | | |
|---|---|---|---|
| Note: BQL represents below the quantification limit. | | | |

**Conclusion:** the compounds of the present disclosure showed a high kidney and liver distribution and a low brain distribution.

### Experimental example 5: Pharmacodynamic studies

### Objectives:

PD and pharmacodynamic studies on the compounds of the present disclosure in male SD rat models of hypertension induced with deoxycorticosterone acetate-sodium chloride (DOCA-NaCl).

### Protocols:

Prior to commencing the procedure, 7-8 weeks old male SD rats were allowed one week acclimatization. On the day of surgery, the table of the surgical area was disinfected with a disinfectant, and the rats were anesthetized with 2%-5% isoflurane and fixed on the table. The abdominal hair was removed and the surgical skin was disinfected. The skin and abdominal wall were cut open to expose the right kidney. The arteries and veins near the renal hilum were ligated. After it was confirmed that there were no bleeding points, the renal blood vessels and surrounding ligaments were bluntly separated and the right kidney was removed. After it was confirmed that there were no bleeding points, the abdominal wall muscle and skin were sutured. After the rats woke up, they were returned to the cage and observed. The rats were injected subcutaneously with meloxicam injection once a day for 3 consecutive days for pain relief.

One week after unilateral kidney removal, the animals were randomly divided into vehicle group, low dose group and high dose group according to body weight/blood pressure. The drinking water of animals in all groups was changed to an aqueous solution containing 1% sodium chloride + 0.2% potassium chloride. They were free to drink for four consecutive weeks. The rats were injected subcutaneously with 20 mg/kg DOCA (deoxycorticosterone acetate) for 4 weeks, twice a week. Except for the vehicle group, animals in the remaining groups were administered intragastrically once a day according to the dose for 28 days. After 28 days of administration, the animals were placed in metabolic cages overnight and urine was collected to detect the levels of urinary albumin and biomarker Rac1.

### Experimental results:

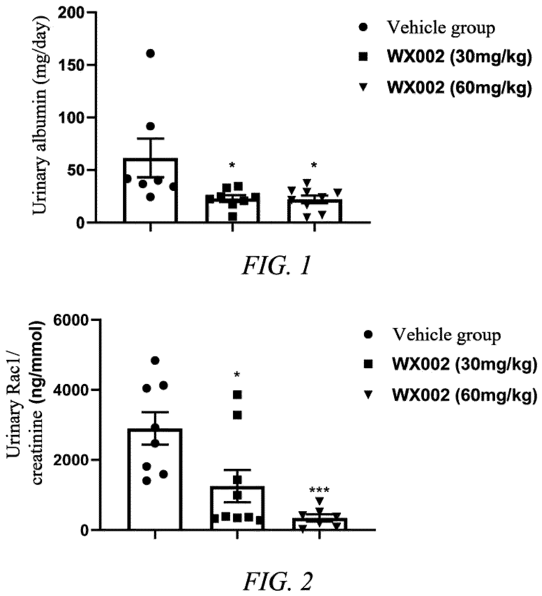

When administered at doses of 30 mg/kg and 60 mg/kg for 28 days, compound WX002 could significantly reduce the urinary albumin level in the rat models of hypertensive renal disease compared with the vehicle control group (as shown in FIG. 1). The biomarker urinary Rac1 content was further detected, and the results showed that compound WX002 could dose-dependently reduce the expression of urinary Rac1 in the rat models of hypertensive renal disease (as shown in FIG. 2). * p < 0.05; *** p < 0.001 vs vehicle group, data is shown as Mean ± SEM.

**Conclusion:** the compound of the present disclosure can significantly reduce the urinary albumin level in rat models of hypertensive renal disease, and can dose-dependently reduce urinary Rac1 expression in rat models of hypertensive renal disease.

## Claims

1. A compound represented by formula (I) or a pharmaceutically acceptable salt thereof, 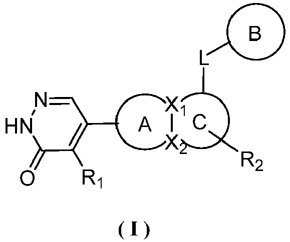 wherein
X₁ and X₂ are each independently selected from C and N;
L is selected from -O-, -S-, -S(=O)-, -C(=O)-, -CF₂-, -CH(OCH₃)-, -N(OCH₃)-, 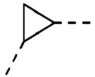 and C₂₋₃ alkenyl, and the -CH(OCH₃)-, 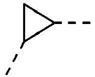 and C₂₋₃ alkenyl are optionally substituted with 1, 2 or 3 Rₐ;
R₁ is selected from F, Cl, Br and I;
R₂ is selected from H, F, Cl, Br, I, OH, NH₂, CN, COOH, CONH₂, C₁₋₃ alkyl, C₁₋₃ alkoxy and C₁₋₃ alkyl-C₁₋₃ alkoxy, and the C₁₋₃ alkyl, C₁₋₃ alkoxy and C₁₋₃ alkyl-C₁₋₃ alkoxy are each independently and optionally substituted with 1, 2 or 3 R_{b};
the structural moiety 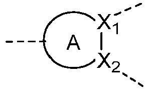 is selected from 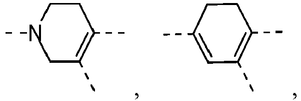 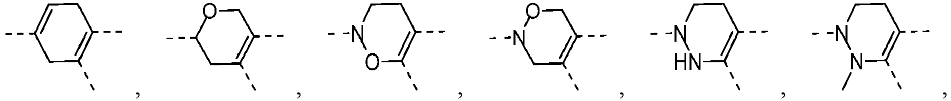 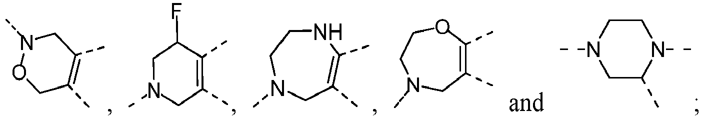
ring B is selected from phenyl and 5- to 6-membered heteroaryl, and the phenyl and 5- to 6-membered heteroaryl are each independently substituted with 1, 2 or 3 R_{c};
ring C is selected from 6-membered heteroaryl;
Rₐ is selected from F, Cl, Br, I and C₁₋₃ alkyl;
R_{b} is selected from F, Cl, Br, I, OH, NH₂, CN, COOH and CONH₂;
R_{c} is selected from F, Cl, Br, I, CONH₂, C₁₋₃ alkyl, C₁₋₃ alkoxy and C₂₋₃ alkenyl, and the C₁₋₃ alkyl, C₁₋₃ alkoxy and C₂₋₃ alkenyl are each independently and optionally substituted with 1, 2 or 3 halogen,
provided that:
1) when Ri is Cl, ring A is 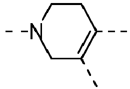 , L is O, and ring B is phenyl, then at least one substituent on ring B is Br, CONH₂, CF₂CH₃, C₁₋₃ alkoxy or C₂₋₃ alkenyl, or, ring B is substituted with 3 R_{c};
2) when R₁ is Cl, ring A is 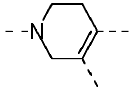 , L is O, and ring B is 2-trifluoromethyl-4-fluorophenyl, then R₂ is CN, COOH, CONH₂, CH₃, CH₂CN, CH₂COOH, CH₂CONH₂ or CH₂OCH₃.

2. The compound or the pharmaceutically acceptable salt thereof according to claim 1, wherein R_{c} is selected from F, Cl, Br, I, CONH₂, CH₃, CH₂CH₃, OCH₃ and -CH=CH₂, and the CH₃, CH₂CH₃, OCH₃ and -CH=CH₂ are each independently and optionally substituted with 1, 2 or 3 halogen.

3. The compound or the pharmaceutically acceptable salt thereof according to claim 2, wherein R_{c} is selected from F, Cl, Br, I, CONH₂, CH₃, CH₂F, CHF₂, CF₃, CH₂CH₃, CF₂CH₃, OCH₃, OCF₃ and -CF=CH₂.

4. The compound or the pharmaceutically acceptable salt thereof according to claim 1, wherein L is selected from -O-, -S-, -S(=O)-, -C(=O)-, -CF₂-, -CH(OCH₃)-, -N(OCH₃)-, 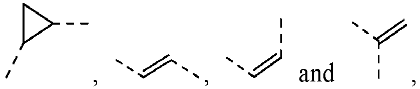 and the -CH(OCH₃)-, 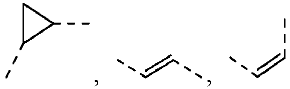 and 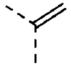 are each independently and optionally substituted with 1, 2 or 3 Rₐ.

5. The compound or the pharmaceutically acceptable salt thereof according to claim 4, wherein L is selected from -O-, -S-, -S(=O)-, -C(=O)-, -CF₂-, -CH(OCH₃)-, -N(OCH₃)-,

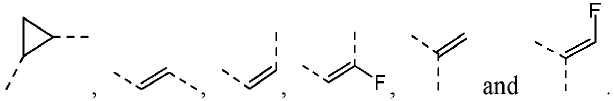

6. The compound or the pharmaceutically acceptable salt thereof according to claim 1, wherein R₂ is selected from H, F, Cl, Br, I, OH, NH₂, CN, COOH, CONH₂, CH₃, CH₂CH₃, OCH₃ and CH₂OCH₃, and the CH₃, CH₂CH₃, OCH₃ and CH₂OCH₃ are each independently and optionally substituted with 1, 2 or 3 R_{b}.

7. The compound or the pharmaceutically acceptable salt thereof according to claim 6, wherein R₂ is selected from H, F, Cl, Br, I, OH, NH₂, CN, COOH, CONH₂, CH₃, CH₂CN, CH₂COOH, CH₂CONH₂, CH₂OH, CH₂CH₃, CH(OH)CH₃, CH(OH)CH₂OH, OCH₃ and CH₂OCH₃.

8. The compound or the pharmaceutically acceptable salt thereof according to claim 1, wherein ring B is selected from phenyl, thienyl and pyridyl, and the phenyl, thienyl and pyridyl are each independently and optionally substituted with 1, 2 or 3 R_{c}.

9. The compound or the pharmaceutically acceptable salt thereof according to claim 8, wherein ring B is selected from

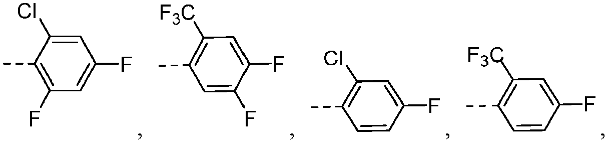

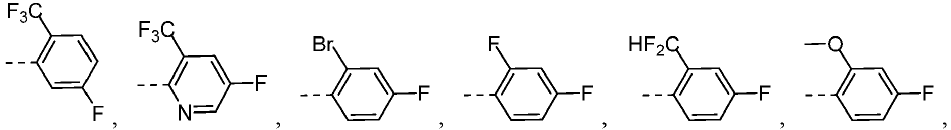

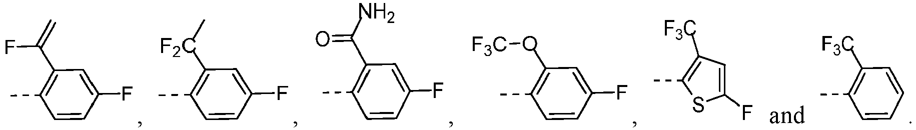

10. The compound or the pharmaceutically acceptable salt thereof according to claim 1, wherein ring C is selected from pyridyl and pyrimidyl.

11. The compound or the pharmaceutically acceptable salt thereof according to claim 1, wherein ring A is

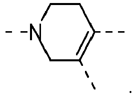

12. The compound or the pharmaceutically acceptable salt thereof according to any one of claims 1 to 11, selected from 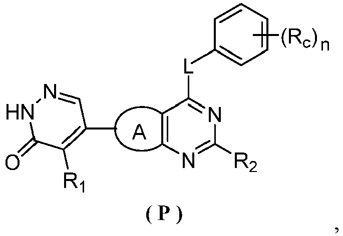 wherein
n is selected from 0, 1, 2 and 3;
R₁, R₃, R_{c}, L and ring A are as defined in any one of claims 1 to 11.

13. The compound or the pharmaceutically acceptable salt thereof according to claim 12, selected from 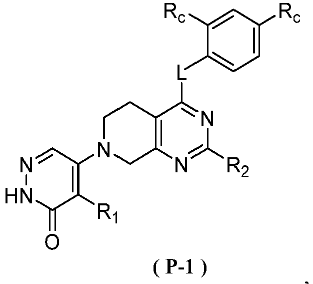 wherein R₁, R₃ and R_{c} are as defined in any one of claims 1 to 11.

14. A compound represented by the following formula or a pharmaceutically acceptable salt thereof,

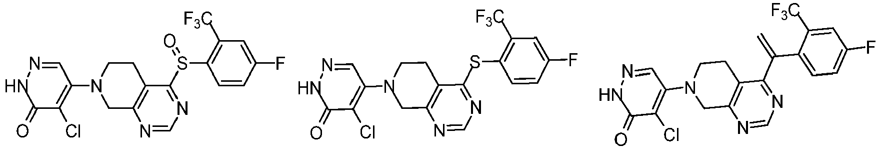

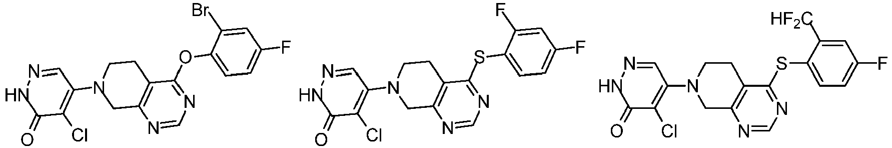

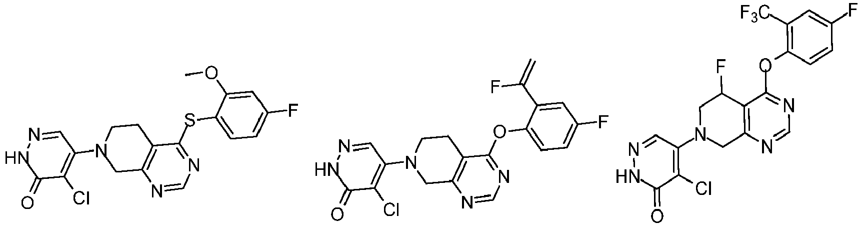

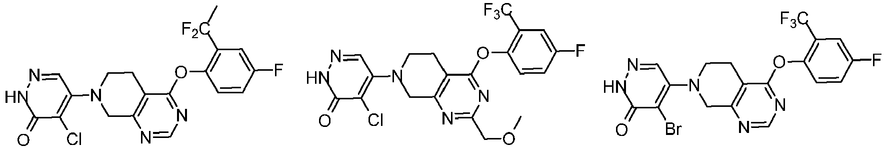

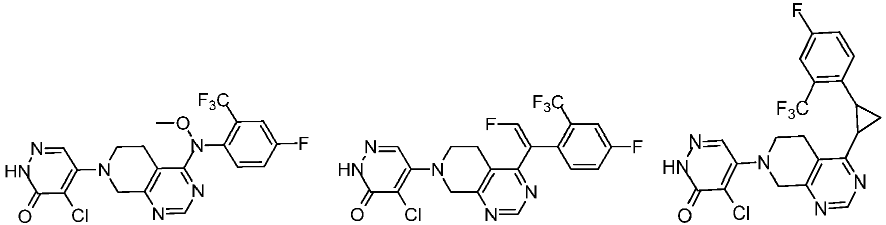

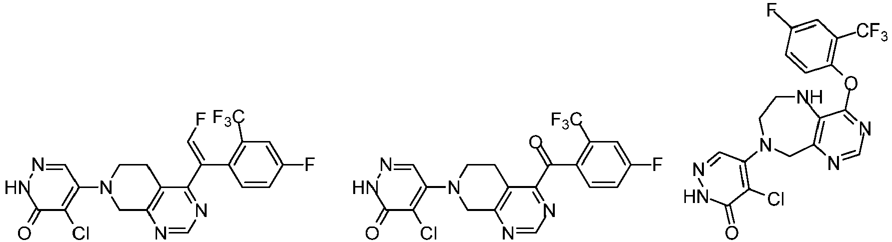

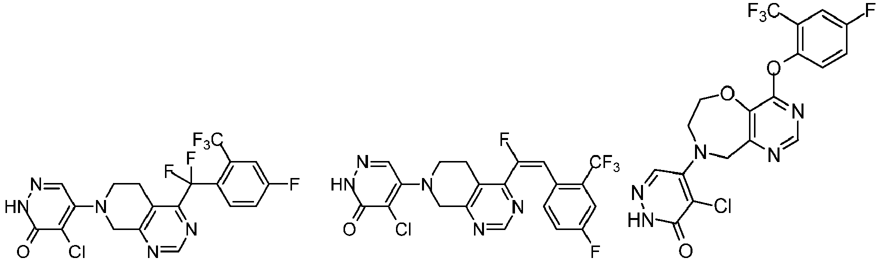

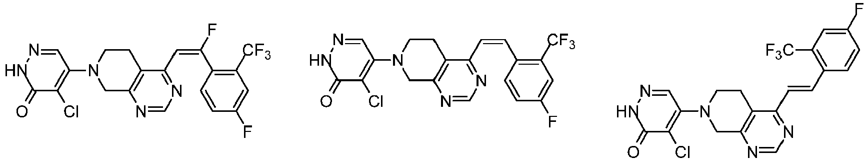

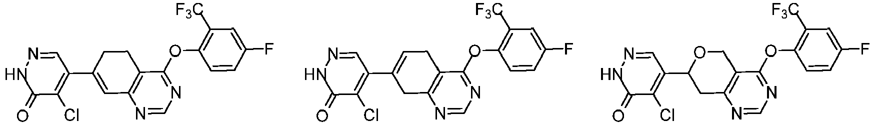

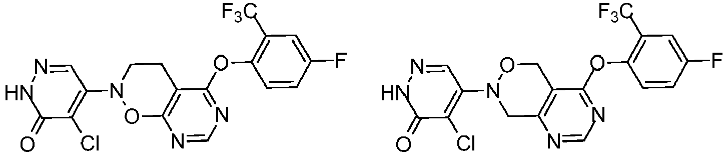

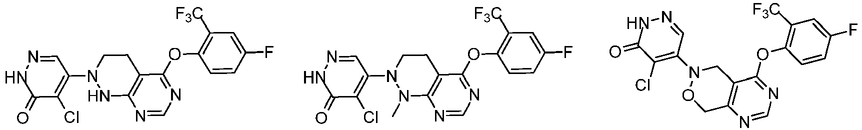

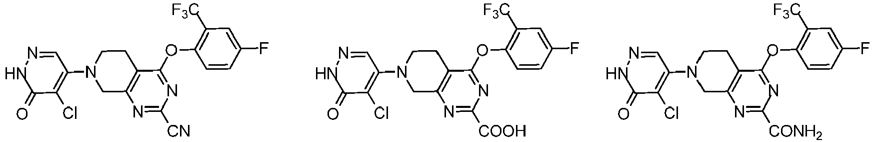

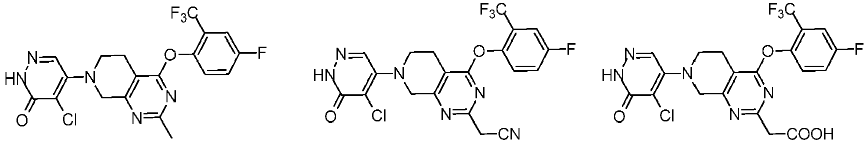

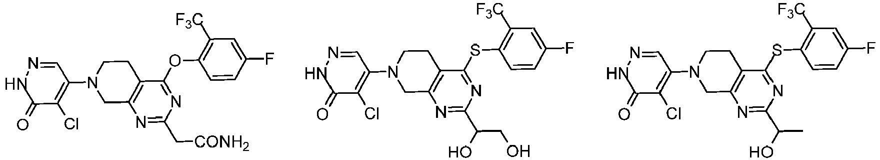

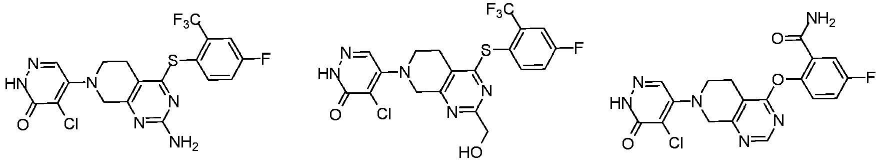

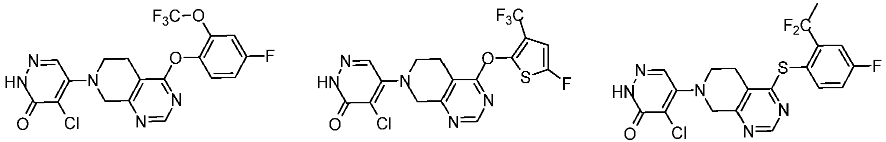

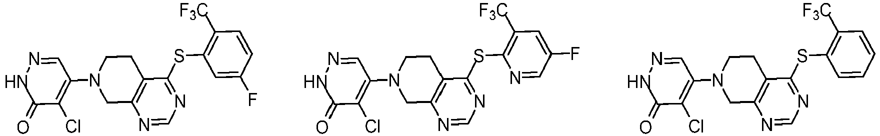

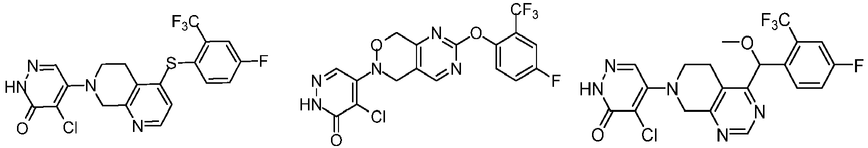

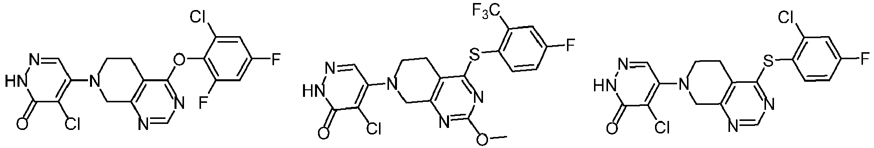

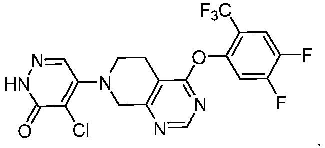

15. The compound or the pharmaceutically acceptable salt thereof according to claim 14, selected from

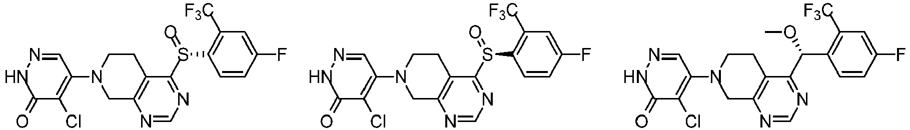

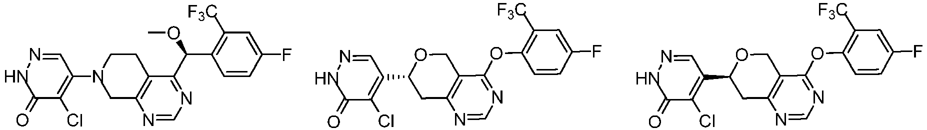

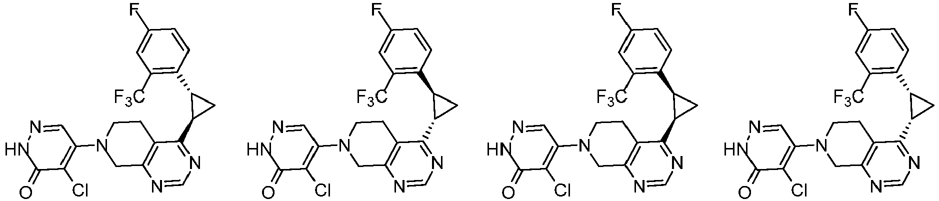

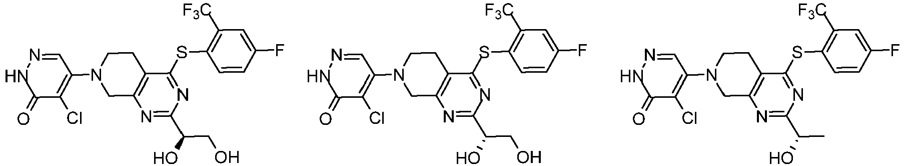

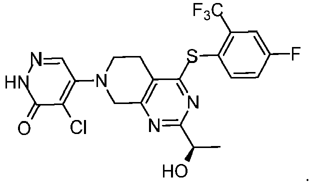

16. A use of the compound or the pharmaceutically acceptable salt thereof according to claims 1 to 15 in a medicament related to TRPC5 inhibitors.
